# EUROPEAN PATENT APPLICATION

(11) **EP 4 368 265 A1**
(43) Date of publication of application: **15.05.2024**
(21) Application number: 22855466.3
(22) Date of filing: 10.08.2022
(51) Int. Cl.: A63B 71/06, A61B 5/11, A61B 5/00, A61B 90/00

(54) **BREATHING GUIDE METHOD AND RELATED APPARATUS**

(30) Priority: 13.08.2021 CN 202110931153
(71) Applicant: Huawei Technologies Co., Ltd., Longgang Shenzhen, Guangdong 518129 (CN)
(72) Inventor: LI, Xiaolong, Shenzhen, Guangdong 518129 (CN); DONG, Xiaojie, Shenzhen, Guangdong 518129 (CN)
(74) Representative: Huawei European IPR
(86) International application number: PCT/CN2022/111414
(87) International publication number: WO 2023/016486

(57) **Abstract**

A breathing guidance method and a related apparatus are provided. The method includes: When detecting a first action, an electronic device (100) prompts, in a first prompt manner, a user to perform an inhalation action; and when detecting a second action, the electronic device (100) prompts, in a second prompt manner, the user to perform an exhalation action. The first action and the second action may be user actions detected by the electronic device (100). The first action and the second action may be actions of another electronic device (for example, exercise equipment and medical equipment) that are detected by the electronic device (100). The first action and the second action may alternatively be actions of another electronic device that are detected by the another electronic device.

## Description

This application claims priority to Chinese Patent Application No. 202110931153.8, filed with the China National Intellectual Property Administration on August 13,2021 and entitled "BREATHING GUIDANCE METHOD AND RELATED APPARATUS", which is incorporated herein by reference in its entirety.

### TECHNICAL FIELD

This application relates to the field of mobile terminal technologies, and in particular, to a breathing guidance method and a related apparatus.

### BACKGROUND

Breathing is one of the most important behaviors in human daily activities. Proper breathing is very important for both exercise and health. However, in some cases, a user may forget to breathe at a proper rhythm, for example, in the exercise field, how to achieve safe exercise and improve performance by adjusting breathing; and in the medical and health field, how to achieve good rehabilitation through breathing guidance.

Currently, there is no solution for providing breathing guidance for a user in the exercise field and the health field. How to provide scientific breathing guidance for the user in the exercise field and the health field is a problem urgently to be resolved.

### SUMMARY

This application provides a breathing guidance method and a related apparatus, to allow an electronic device to prompt, in different prompt manners, a user when to inhale and when to inhale, so that a rhythm of an action matches a breathing rhythm of the user.

According to a first aspect, this application provides a breathing guidance method, where the method includes: A first electronic device obtains sensor data;
the first electronic device determines an action type of a user based on the sensor data; and the first electronic device outputs a first prompt when the first electronic device determines that the action type of the user is a first action type, where the first prompt is used to prompt the user to perform an inhalation action; or the first electronic device outputs a second prompt when the first electronic device determines that the action type of the user is a second action type, where the second prompt is used to prompt the user to perform an exhalation action, and the first prompt is different from the second prompt.

The first electronic device may be a mobile phone, a wearable device, a headset, smart glasses, an augmented reality (augmented reality, AR) device, a virtual reality (virtual reality, VR) device, or the like. The wearable device may be a wrist-supported device, for example, a smartwatch, a smart band, or a smart wrist strap. Alternatively, the wearable device may be an ankle-supported device, for example, a smart anklet, smart shoes, smart socks, or another device that can be worn on a leg. Alternatively, the wearable device may be a head-supported device, for example, a smart helmet or a smart head strap (which may also be referred to as a smart headband).

Alternatively, the first electronic device may be a medical equipment device, a fitness equipment device, or the like. The medical equipment device may include but is not limited to a ventilator, a nebulizer, a gastroscope device, a chest X-ray detection device, and the like. The fitness equipment device may include but is not limited to a hull, a rowing machine, an elliptical machine, a barbell, and the like.

In a possible implementation, a third electronic device collects the sensor data, and determines the action type of the user based on the sensor data. When the third electronic device determines that the action type of the user is the first action type, the third electronic device sends an instruction 1 to the first electronic device. After receiving the instruction 1, the first electronic device outputs the first prompt, where the first prompt is used to prompt the user to perform an inhalation action. When the third electronic device determines that the action type of the user is the second action type, the third electronic device sends an instruction 2 to the first electronic device. After receiving the instruction 2, the first electronic device outputs the second prompt, where the second prompt is used to prompt the user to perform an exhalation action, and the first prompt is different from the second prompt.

According to the method provided in the first aspect, the electronic device may prompt, in different prompt manners, the user when to inhale and when to inhale, so that a rhythm of an action matches a breathing rhythm of the user, thereby saving physical energy and improving an exercise capability of the user.

With reference to the first aspect, in a possible implementation, a type of the first prompt is any one or more of the following: vibration, voice, text, and picture; and a type of the second prompt is any one or more of the following: vibration, voice, text, and picture.

With reference to the first aspect, in a possible implementation, that the first prompt is different from the second prompt specifically includes:
a vibration frequency of the first prompt is different from a vibration frequency of the second prompt.

With reference to the first aspect, in a possible implementation, that the first prompt is different from the second prompt specifically includes:
voice content of the first prompt is different from voice content of the second prompt.

With reference to the first aspect, in a possible implementation, that the first prompt is different from the second prompt specifically includes: a type of the first prompt is any one or more of the following: vibration, voice, text, and picture; and the second prompt does not output any content; or the first prompt does not output any content; and a type of the second prompt is any one or more of the following: vibration, voice, text, and picture.

With reference to the first aspect, in a possible implementation, before the first electronic device starts to guide breathing of the user, the first electronic device may display a guiding action on a display screen. The guiding action is used to indicate breathing actions corresponding to different action types to the user.

With reference to the first aspect, in a possible implementation, before the first electronic device starts to guide breathing of the user, the first electronic device may display an animation on a display screen. The animation is used to indicate breathing actions corresponding to different action types to the user.

With reference to the first aspect, in a possible implementation, before the first electronic device starts to guide breathing of the user, the first electronic device may play voice. The voice is used to indicate breathing actions corresponding to different action types to the user.

With reference to the first aspect, in a possible implementation, the method further includes: When the first electronic device outputs the first prompt, the first electronic device sends a first instruction to a second electronic device, where the first instruction is used to instruct the second electronic device to output a third prompt, the third prompt is used to prompt the user to perform an inhalation action, and a type of the third prompt is any one or more of the following: vibration, voice, text, and picture. In this way, when outputting the first prompt, the first electronic device may output the third prompt by using another electronic device (the second electronic device is a headset or a mobile phone) or the like to which the first electronic device establishes a connection.

In another possible implementation, the first electronic device may not output any content, but output the third prompt by using another electronic device (the second electronic device is a headset or a mobile phone) or the like to which the first electronic device establishes a connection.

With reference to the first aspect, in a possible implementation, that the electronic device outputs a first prompt when the first electronic device determines that the action type of the user is a first action type specifically includes: The electronic device outputs the first prompt when the first electronic device determines, for n consecutive times, that the action type of the user is the first action type, where n is a positive integer greater than or equal to 1.

That the electronic device outputs a second prompt when the first electronic device determines that the action type of the user is a second action type specifically includes: The electronic device outputs the second prompt when the first electronic device determines, for m consecutive times, that the action type of the user is the second action type, where m is a positive integer greater than or equal to 1. The first electronic device may enable the user to perform one inhalation action or one exhalation action when the first electronic device detects a plurality of first actions or second actions. For example, in running, "one exhalation action is performed per step", and in swimming, "two stroke actions may correspond to one breathing action". In this way, the first electronic device provides different breathing rhythms based on different exercise types, thereby reflecting breathing guidance flexibility.

With reference to the first aspect, in a possible implementation, after the first electronic device outputs the first prompt, and before the first electronic device outputs the second prompt, the method further includes: The first electronic device outputs a fourth prompt when the first electronic device determines that the action type of the user is a third action type, where the fourth prompt is used to prompt the user to perform a breath holding action, and a type of the fourth prompt is any one or more of the following: vibration, voice, text, and picture. For example, in weight lifting exercise, after the user completes a first action, the user holds a barbell at a highest point for a period of time. In this period of time, the action of the user may be referred to as a third action. For example, in swimming exercise, after the user completes a first action, the user keeps an arm action stationary for a period of time. The user action herein may also be referred to as a third action. When the user performs the third action, in a possible implementation, the first electronic device prompts, in a fourth prompt manner, the user to hold breath. In another possible implementation, the first electronic device prompts, in a fourth prompt manner, the user to perform one or more sets of brief exhalation and inhalation actions.

With reference to the first aspect, in a possible implementation, that the first electronic device outputs a first prompt when the first electronic device determines that the action type of the user is a first action type, where the first prompt is used to prompt the user to perform an inhalation action specifically includes: The first electronic device outputs the first prompt when the first electronic device determines that the action type of the user is the first action type and the first electronic device detects that the user has completed a first action, where the first prompt is used to prompt the user to perform an inhalation action. For example, in a swimming application scenario, only when the user completes the first action, the head of the user can extend out of the water to perform an inhalation action.

In another possible implementation, the first electronic device outputs the second prompt when the first electronic device determines that the action type of the user is the second action type and the first electronic device detects that the user has completed a second action, where the second prompt is used to prompt the user to perform an exhalation action. For example, in a swimming application scenario, when the user completes the second action, the first electronic device prompts the user to perform an exhalation action.

With reference to the first aspect, in a possible implementation, before the first electronic device obtains the sensor data, the method further includes: The first electronic device receives a first input operation, and responds to the first input operation to determine a first exercise mode, where the first exercise mode is any one of the following: a running mode, a swimming mode, a weight lifting mode, an elliptical machine exercise mode, a rowing machine exercise mode, and a rowing mode; and that the first electronic device determines an action type of a user based on the sensor data specifically includes: The first electronic device determines the action type of the user in the first exercise mode based on the sensor data. In this way, before exercise, the first electronic device may enable different exercise modes, and provide different breathing guidance solutions in different exercise modes.

In another possible implementation, the first electronic device may adaptively enable the first exercise mode based on collected motion sensor data.

After the first electronic device adaptively enables the first exercise mode, the first electronic device displays a first interface, and a first control is displayed in the first interface. The first electronic device receives and responds to an input operation of the user for the first control, and the first electronic device cancels enabling of the first exercise mode. In this way, the first electronic device is prevented from mistakenly enabling the first exercise mode, thereby increasing consumption of the first electronic device.

With reference to the first aspect, in a possible implementation, before the first electronic device obtains the sensor data, the method further includes: The first electronic device receives the sensor data sent by a third electronic device.

The third electronic device may be a fitness equipment device, or may be a medical equipment device. That is, the first electronic device establishes a communication connection to the first electronic device. The third electronic device collects motion sensor data on the third electronic device in real time, and sends the motion sensor data to the first electronic device in real time.

With reference to the first aspect, in a possible implementation, the sensor data includes one or more of acceleration data, gyroscope data, image data, gravity data, and pressure data.

With reference to the first aspect, in a possible implementation, within first time after the first electronic device determines the first exercise mode, the first electronic device suspends obtaining of the sensor data. In this way, after providing breathing guidance for the user for a period of time, the first electronic device may suspend a breathing guidance function, thereby reducing power consumption of the first electronic device.

With reference to the first aspect, in a possible implementation, after the first electronic device suspends obtaining of the sensor data, if the first electronic device detects that a heart rate of the user is greater than a preset heart rate value and/or a respiratory rate of the user is greater than a preset frequency, the first electronic device continues obtaining the sensor data. In this way, breathing guidance flexibility of the first electronic device is reflected.

According to a second aspect, this application provides an electronic device, where the electronic device includes one or more processors and one or more memories, the one or more memories are coupled to the one or more processors, the one or more memories are configured to store computer program code, the computer program code includes computer instructions, and the one or more processors invoke the computer instructions to enable the electronic device to perform the method according to any one of the implementations of the first aspect.

According to a third aspect, this application provides a computer-readable storage medium, including instructions, where when the instructions are run on an electronic device, the electronic device is enabled to perform the method according to any one of the implementations of the first aspect.

According to a fourth aspect, this application provides a computer program product, where when the computer program product runs on an electronic device, the electronic device is enabled to perform the method according to any one of the implementations of the first aspect.

### BRIEF DESCRIPTION OF DRAWINGS

FIG. 1 is a schematic diagram of a hardware structure of an electronic device 100 according to an embodiment of this application;
FIG. 2 is a block diagram of a software structure of an electronic device 100 according to an embodiment of this application;
FIG. 3A to FIG. 3E are a group of UI diagrams when an electronic device 100 enables a running mode according to an embodiment of this application;
FIG. 4 is a UI diagram of a motion sensor when an electronic device 100 enables a running mode according to an embodiment of this application;
FIG. 5A to FIG. 5O are a group of UI diagrams in a breathing guidance solution in a running process according to an embodiment of this application;
FIG. 6A and FIG. 6B are a group of schematic diagrams of a first action and a second action in breaststroke according to an embodiment of this application;
FIG. 7A and FIG. 7B are a group of schematic diagrams of a first action and a second action in freestyle swimming according to an embodiment of this application;
FIG. 8A and FIG. 8B are a group of schematic diagrams of a first action and a second action in backstroke according to an embodiment of this application;
FIG. 9A and FIG. 9B are a group of schematic diagrams of a first action and a second action in butterfly stroke according to an embodiment of this application;
FIG. 10A and FIG. 10B are a group of schematic diagrams of a first action and a second action in weight lifting exercise according to an embodiment of this application;
FIG. 11A and FIG. 11B are a group of schematic diagrams of a first action and a second action in rowing exercise according to an embodiment of this application;
FIG. 12A and FIG. 12B are a group of schematic diagrams of a first action and a second action when exercise is performed by using a rowing machine according to an embodiment of this application;
FIG. 13A and FIG. 13B are a group of schematic diagrams of a first action and a second action when exercise is performed by using an elliptical machine according to an embodiment of this application;
FIG. 14A to FIG. 14C are a group of schematic diagrams of determining an action type in weight lifting exercise based on acceleration data according to an embodiment of this application;
FIG. 15A to FIG. 15C are a group of schematic diagrams of determining an action type in rowing exercise based on acceleration data according to an embodiment of this application;
FIG. 16A to FIG. 16C are a group of schematic diagrams of determining, based on acceleration data, an action type when exercise is performed by using a rowing machine according to an embodiment of this application;
FIG. 17A to FIG. 17C are a group of schematic diagrams of determining, based on acceleration data, an action type when exercise is performed by using an elliptical machine according to an embodiment of this application;
FIG. 18 is a schematic diagram of a scenario in gastroscope examination according to an embodiment of this application;
FIG. 19 is a schematic diagram of a scenario in nebulized inhalation therapy according to an embodiment of this application;
FIG. 20 is a schematic method flowchart of a breathing guidance method according to an embodiment of this application; and
FIG. 21 is a schematic diagram of obtaining, by an electronic device 100, an exercise capability of a user based on personal information of the user according to an embodiment of this application.

### DESCRIPTION OF EMBODIMENTS

The following clearly and comprehensively describes technical solutions in embodiments of this application with reference to the accompanying drawings. In descriptions of the embodiments of this application, unless otherwise specified, "/" indicates "or". For example, A/B may indicate A or B. The term "and/or" in this specification describes only an association relationship between associated objects, and represents that three relationships may exist. For example, A and/or B may represent the following three cases: Only A exists, both A and B exist, and only B exists. In addition, in the descriptions of the embodiments of this application, "a plurality of" means "two or more".

The following terms "first" and "second" are merely intended for a purpose of description, and shall not be understood as an indication or implication of relative importance or implicit indication of a quantity of indicated technical features. Therefore, a feature limited by "first" or "second" may explicitly or implicitly include one or more features. In the descriptions of the embodiments of this application, unless otherwise specified, "a plurality of" means "two or more".

A term "user interface (user interface, UI)" in the following embodiments of this application is a medium interface for interaction and information exchange between an application or an operating system and a user, and implements conversion between an internal form of information and a form acceptable to the user. The user interface is source code written in a specific computer language such as Java or an extensible markup language (extensible markup language, XML). Interface source code is parsed and rendered on an electronic device, and is finally presented as content that can be identified by the user. The user interface is generally represented in a form of a graphical user interface (graphic user interface, GUI), and is a user interface that is related to a computer operation and that is displayed in a graphic manner. The user interface may be a visual interface element displayed on a display screen of the electronic device, for example, a text, an icon, a button, a menu, a tab, a text box, a dialog box, a status bar, a navigation bar, or a Widget.

Currently, in the exercise field, as people pay more attention to physical health, increasingly more people participate in exercise activities, and a large quantity of exercise-type applications also emerge. A current exercise-type application can only record motion data of a user in a simple manner, or provide some offline articles or tutorials for reference of the user before exercise. Alternatively, some electronic devices (for example, a wearable device) have a daily breathing training function. For example, when the user is nervous, the user may adjust a breathing rhythm of the user based on a breathing tutorial on the wearable device, so as to relax emotions. However, the breathing training is only used to adjust the emotion of the user, and cannot guide the breathing rhythm of the user in an exercise process to improve an exercise effect and an exercise result.

In the health field, in some cases, because a cardiopulmonary function of the user is not good, breathing training needs to be combined to improve the cardiopulmonary function. Currently, a doctor generally notifies, in a manner of oral guidance, the user to perform breathing rehabilitation training in specific time according to a specific rule. Oral medical advice can be easily forgotten by patients with poor memory. In addition, breathing rehabilitation training performed by the user based on memory may be different from breathing rehabilitation training guided by the doctor, resulting in a poor training effect.

Alternatively, in some other cases, in some health examination processes, the user needs to cooperate with the doctor in examination with reference to a breathing rhythm. Currently, the user is generally notified, based on experience and judgment of the doctor, when to inhale and when to exhale. Experience and judgment of the doctor may have an error. In addition, the doctor performs examination for many patients every day. If the doctor needs to prompt the user to breathe each time, workload of the doctor is greatly increased.

Therefore, embodiments of this application provide a breathing guidance method. The method includes: When detecting a first action, an electronic device prompts, in a first prompt manner, a user to inhale; and when detecting a second action, the electronic device prompts, in a second prompt manner, the user to exhale.

The first action and the second action may be user actions detected by the electronic device. Alternatively, the first action and the second action may be actions of another electronic device (for example, exercise equipment and medical equipment) that are detected by the electronic device.

A type of the first prompt manner may be any one or more of vibration, voice, text, and picture. A type of the second prompt manner may also be any one or more of vibration, voice, text, and picture.

When both the type of the first prompt manner and the type of the second prompt manner are vibration, a vibration frequency of the first prompt manner may be different from a vibration frequency of the second prompt manner. For example, the first prompt manner may be long vibration, and the second prompt manner may be intermittent vibration. The vibration frequency of the first prompt manner may also be the same as the vibration frequency of the second prompt manner. For example, the first prompt manner may be vibration only once, and the second prompt manner may also be vibration only once.

When both the type of the first prompt manner and the type of the second prompt manner are voice, voice content of the first prompt manner is different from voice content of the second prompt manner. For example, the voice content of the first prompt manner may be "please inhale", and the voice content of the second prompt manner may be "please exhale".

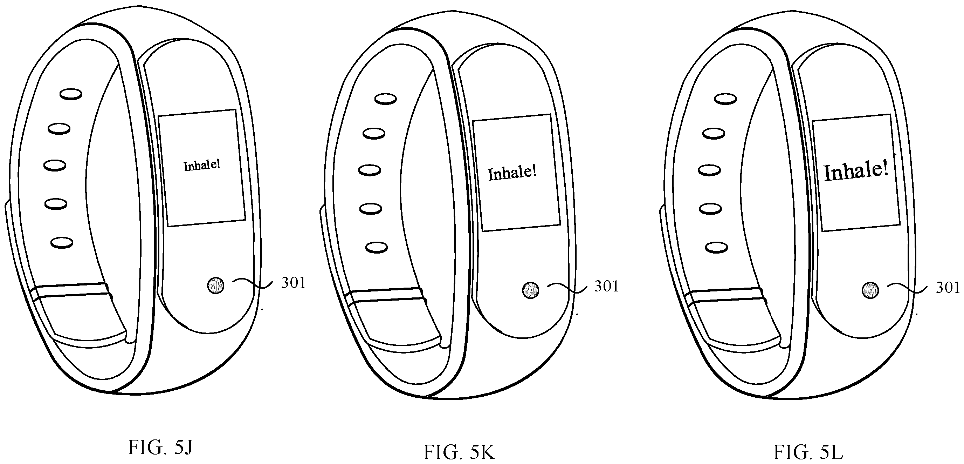

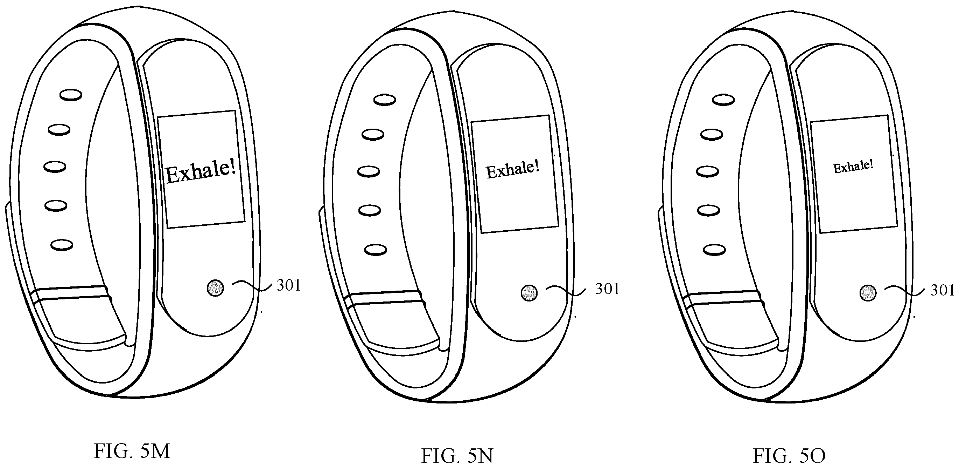

When both the type of the first prompt manner and the type of the second prompt manner are text, and the electronic device outputs a first prompt, the electronic device may display a text "inhale" on a display screen. In addition, a size, a color, and a shape of the text displayed on the display screen may gradually change (for example, a font gradually becomes larger) as a user performs an inhalation action. For details, refer to subsequent embodiments described in FIG. 5J to FIG. 5L. When the electronic device outputs a second prompt, the electronic device may display a text "exhale" on the display screen, and a size, a color, and a shape of the text displayed on the display screen may gradually change (for example, the font gradually becomes smaller) as the user performs an exhalation action. For details, refer to subsequent embodiments described in FIG. 5M to FIG. 5O.

When both the type of the first prompt manner and the type of the second prompt manner are pictures, and the electronic device outputs the first prompt, the electronic device may display an image on a display screen. In addition, a size, a color, and a shape of the image displayed on the display screen may gradually change (for example, the image gradually becomes larger) as a user performs an inhalation action. When the electronic device outputs the second prompt, the electronic device may display an image on the display screen, and a size, a color, and a shape of the image displayed on the display screen may gradually change (for example, the image gradually becomes smaller) as the user performs an exhalation action.

Alternatively, the type of the first prompt manner and the type of the second prompt manner may be a combination of two or more of the foregoing vibration, voice, text, and picture.

The type of the first prompt manner may be any one or more of vibration, voice, text, and picture, and the second prompt manner does not output any prompt. For example, when the electronic device detects that the user performs the first action, the electronic device prompts, through vibration, voice, text, picture, and the like, the user to perform an inhalation action. When the electronic device detects that the user performs the second action, the electronic device does not output any prompt.

The type of the first prompt manner may not output any prompt, and the second prompt manner may be any one or more of vibration, voice, text, and picture. For example, when the electronic device detects that the user performs the first action, the electronic device does not output any prompt. When the electronic device detects that the user performs the second action, the electronic device prompts, through vibration, voice, text, picture, and the like, the user to perform an inhalation action.

Alternatively, the first prompt manner and the second prompt manner may have other implementations. Specific implementations of the first prompt manner and the second prompt manner are not limited in embodiments of this application.

In this way, the electronic device may prompt, in different prompt manners, the user when to inhale and when to inhale, so that a rhythm of an action matches a breathing rhythm of the user.

The method can be applied to the exercise field. In the exercise field, rhythmic breathing matching rhythmic exercise can achieve twice a training effect of the user with half the effort. That is, when the electronic device detects that the user performs the first action, the electronic device prompts, in the first prompt manner, the user to inhale. When the electronic device detects that the user performs the second action, the electronic device prompts, in the second prompt manner, the user to exhale. In this way, the method can be used to guide the breathing rhythm and the exercise rhythm in the exercise process of the user, so that the breathing rhythm of the user matches the exercise rhythm. Therefore, sufficient oxygen and energy can be provided for muscles in the exercise process, to reduce physical energy consumption of the user and enhance an exercise effect.

The method may be applied to the health field. In some health examination processes, when the user needs to cooperate with the doctor in examination with reference to a breathing rhythm, and the electronic device detects that another electronic device (for example, medical equipment) performs the first action, the electronic device prompts, in the first prompt manner, the user to inhale. When the electronic device detects that another electronic device (for example, medical equipment) performs the second action, the electronic device prompts, in the second prompt manner, the user to exhale. In this way, the breathing rhythm of the user may cooperate with an examination action of another electronic device (for example, medical equipment), so that user experience in the examination process can be improved, discomfort can be reduced, and health examination efficiency can also be improved. In addition, when a patient needs to perform breathing rehabilitation training, the patient may store, in the electronic device, an electronic version of a breathing guidance solution provided by the doctor, and the electronic device regularly prompts, based on the electronic version of the breathing guidance solution, the user to perform breathing rehabilitation training. In this way, the user can regularly perform breathing rehabilitation training based on the breathing guidance solution, thereby improving a rehabilitation effect.

**The following describes the electronic device provided in embodiments of this application**.

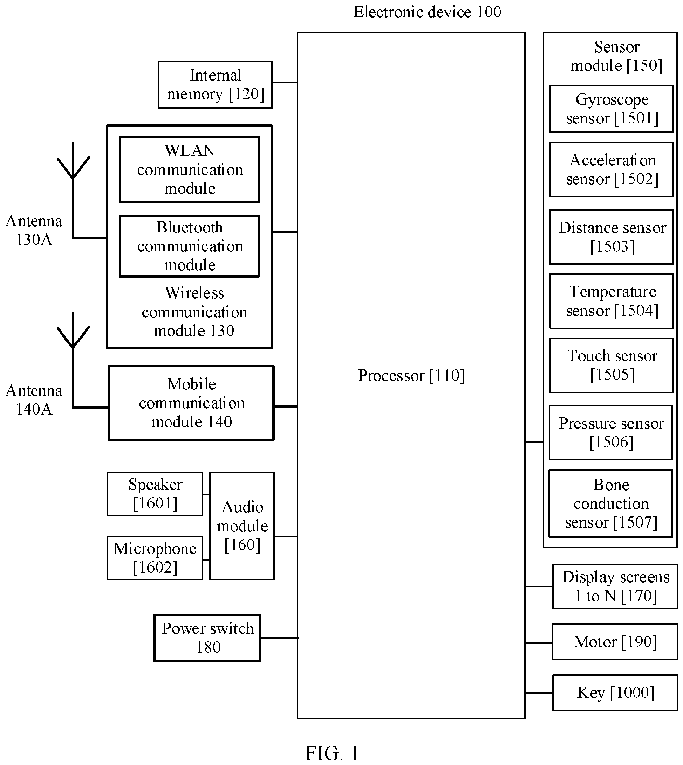

FIG. 1 is a schematic diagram of a hardware structure of an electronic device 100 according to an embodiment of this application.

The electronic device 100 may be a mobile phone, a tablet computer, a desktop computer, a laptop computer, a handheld computer, a notebook computer, an ultra-mobile personal computer (ultra-mobile personal computer, UMPC), a netbook, a cellular phone, a personal digital assistant (personal digital assistant, PDA), an augmented reality (augmented reality, AR) device, a virtual reality (virtual reality, VR) device, an artificial intelligence (artificial intelligence, AI) device, a wearable device (a smartwatch and a smart band), a vehicle-mounted device, a smart home device, and/or a smart city device. A specific type of the electronic device is not specifically limited in this embodiment of this application.

The electronic device 100 may include a processor 110, an internal memory 120, a wireless communication module 130, a mobile communication module 140, a sensor module 150, an audio module 160, a display screen 170, a power switch 180, a motor 190, and a key 1000.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural processing unit (neural-processing unit, NPU), and/or the like. Different processing units may be independent devices, or may be integrated into one or more processors.

The internal memory 120 may include one or more random access memories (random access memory, RAM) and one or more non-volatile memories (non-volatile memory, NVM).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, for example, a fifth generation DDR SDRAM is generally referred to as a DDR5 SDRAM), and the like. The non-volatile memory may include a magnetic disk storage device and a flash memory (flash memory).

According to operation principles, the flash memory may include NOR FLASH, NAND FLASH, 3D NAND FLASH, and the like. According to quantities of potential levels of storage units, the flash memory may include a single-level cell (single-level cell, SLC), a multi-level cell (multi-level cell, MLC), a triple-level cell (triple-level cell, TLC), a quad-level cell (quad-level cell, QLC), and the like. According to storage specifications, the flash memory may include a universal flash storage (English: universal flash storage, UFS), an embedded multimedia card (embedded multimedia Card, eMMC), and the like.

The random access memory may be directly read and written by the processor 110, may be configured to store executable programs (for example, machine instructions) of an operating system or other running programs, or may be configured to store data of users and applications.

The nonvolatile memory may also store an executable program, data of a user, data of an application, and the like, which may be loaded into the random access memory in advance for directly reading and writing by the processor 110.

A wireless communication function of the electronic device 100 may be implemented by using an antenna 130A, an antenna 140A, the wireless communication module 130, the mobile communication module 140, the modem processor, the baseband processor, and the like.

The antenna 130A and the antenna 140A may be configured to transmit and receive electromagnetic wave signals. Each antenna in the electronic device 100 may be configured to cover a single communication frequency band or a plurality of communication frequency bands. Different antennas may be further multiplexed, to improve antenna utilization.

The wireless communication module 130 can provide a solution for wireless communication including a wireless local area network (wireless local area networks, WLAN), Bluetooth (Bluetooth, BT), a global navigation satellite system (global navigation satellite system, GNSS), frequency modulation (frequency modulation, FM), a near field communication technology (near field communication, NFC), an infrared technology (infrared, IR) and the like to be applied to the electronic device 100. The wireless communication module 130 may be one or more devices that integrate at least one communication processing module. The wireless communication module 130 receives an electromagnetic wave by using the antenna 130A, performs frequency modulation on and filters the electromagnetic wave signal, and sends a processed signal to the processor 110. The wireless communication module 130 may further receive a to-be-sent signal from the processor 110, perform frequency modulation and amplification on the signal, and convert the signal into an electromagnetic wave for radiation through the antenna 130A.

The mobile communication module 140 can provide a solution for wireless communication including 2G/3G/4G/5G and the like to be applied to the electronic device 100. The mobile communication module 140 may include at least one filter, a switch, a power amplifier, a low noise amplifier (low noise amplifier, LNA), and the like. The mobile communication module 140 may receive an electromagnetic wave through the antenna 140A, perform processing such as filtering or amplification on the received electromagnetic wave, and transmit the electromagnetic wave to the modem processor for demodulation. The mobile communication module 140 may further amplify a signal modulated by the modem processor, and convert the signal into an electromagnetic wave for radiation through the antenna 140A.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then the demodulator transfers the low-frequency baseband signal obtained through demodulation to the baseband processor for processing. The low-frequency baseband signal is processed by the baseband processor and then transferred to an application processor. The application processor outputs a sound signal by using an audio device, or displays an image or a video by using the display screen 170.

In some embodiments, the mobile communication module 140 and the wireless communication module 130 may be configured to: establish a connection to another device, and obtain motion data collected by the another device, where the motion data includes a heart rate, calories, a respiratory rate, and the like; or control the another device to prompt a user to breathe, adjust an action, suspend or continue exercise, and the like, where the another device may refer to a mobile phone, a wristband, a body fat scale, a computer, a notebook, and the like.

The sensor module 150 includes a gyroscope sensor 1501, an acceleration sensor 1502, a distance sensor 1503, a temperature sensor 1504, a touch sensor 1505, a pressure sensor 1506, and a bone conduction sensor 1507.

The gyroscope sensor 1501 may be configured to determine a motion posture of the electronic device 100. In some embodiments, an angular velocity of the electronic device 100 around three axes (namely, x, y, and z axes) may be determined by using the gyroscope sensor 1501. The gyroscope sensor 1501 may be used for image stabilization during photographing. For example, when a shutter is pressed, the gyroscope sensor 1501 detects an angle at which the electronic device 100 jitters, calculates, based on the angle, a distance for which a lens module needs to compensate, and allows the lens to cancel the jitter of the electronic device 100 through reverse motion, to implement image stabilization. The gyroscope sensor 1501 may be further used in a navigation scenario and a motion sensing game scenario.

The acceleration sensor 1502 may be configured to detect magnitudes of accelerations in various directions (generally on three axes) of the electronic device 100. When the electronic device 100 is still, a magnitude and a direction of gravity may be detected. The acceleration sensor 1502 may be further configured to recognize a posture of the electronic device, and is applied to applications such as landscape and portrait orientation switching and a pedometer.

The distance sensor 1503 may be configured to measure a distance. The electronic device 100 may measure the distance by using infrared or laser. In some embodiments, in a photographing scene, the electronic device 100 may use the distance sensor 1503 to measure a distance to implement fast focusing.

The temperature sensor 1504 is configured to detect a temperature. In some embodiments, the electronic device 100 performs a temperature handling policy based on the temperature detected by the temperature sensor 1504. For example, when the temperature reported by the temperature sensor 1504 exceeds a threshold, the electronic device 100 lowers performance of a processor located near the temperature sensor 1504, to reduce power consumption to implement thermal protection. In some other embodiments, when the temperature is less than another threshold, the electronic device 100 heats the battery, to prevent the electronic device 100 from being abnormally powered off due to the low temperature. In some other embodiments, when the temperature is less than still another threshold, the electronic device 100 boosts an output voltage of the battery, to avoid abnormal power-off caused by the low temperature.

The touch sensor 1505 is also referred to as a "touch device". The touch sensor 1505 may be arranged on the display screen 170. The touch sensor 1505 and the display screen 170 form a touchscreen, which is also referred to as a "touch screen". The touch sensor 1505 is configured to detect a touch operation performed on or near the touch sensor 1505. The touch sensor 1505 may transmit the detected touch operation to the application processor, to determine a touch event type. A visual output related to the touch operation may be provided through the display screen 170. In some other embodiments, the touch sensor 1505 may alternatively be disposed on a surface of the electronic device 100, and is at a position different from that of the display screen 170.

The pressure sensor 1506 is configured to sense a pressure signal, and is capable of converting the pressure signal to an electrical signal. In some embodiments, the pressure sensor 1506 may be disposed on the display screen 170. There are many types of pressure sensors 1506, for example, a resistive pressure sensor, an inductive pressure sensor, and a capacitive pressure sensor. The capacitive pressure sensor may include at least two parallel plates made of conductive materials. When force is applied to the pressure sensor 1506, a capacitance between electrodes changes. The electronic device 100 determines intensity of pressure based on a change of the capacitance. When a touch operation is performed on the display screen 170, the electronic device 100 detects strength of the touch operation by using the pressure sensor 1506. The electronic device 100 may also calculate a touch position based on a detected signal of the pressure sensor 1506. In some embodiments, touch operations that are applied to a same touch location but have different touch operation intensity may correspond to different operation instructions. For example, when a touch operation with touch operation intensity less than a first pressure threshold is applied to an SMS message application icon, an instruction for viewing an SMS message is executed. When a touch operation with touch operation intensity greater than or equal to the first pressure threshold is applied to the SMS message application icon, an instruction for creating a new SMS message is executed.

The bone conduction sensor 1507 may collect a vibration signal. In some embodiments, the bone conduction sensor 1507 may obtain a vibration signal of a vibration bone in a human vocal-cord part. The bone conduction sensor 1507 may also be in contact with a human pulse to receive a blood pressure pulse signal. In some embodiments, the bone conduction sensor 1507 may alternatively be disposed in a headset, to form a bone conduction headset in combination with the headset. The audio module 160 may obtain a voice signal through parsing based on the vibration signal, of the vibration bone of the vocal-cord part, that is obtained by the bone conduction sensor 1507, to implement a voice function. The application processor may parse heart rate information based on the blood pressure beating signal obtained by the bone conduction sensor 1507, to implement a heart rate detection function.

The audio module 160 is configured to convert digital audio information into analog audio signal output, and is also configured to convert analog audio input into a digital audio signal. The audio module 160 may be further configured to encode and decode an audio signal. In some embodiments, the audio module 160 may be disposed in the processor 110, or some functional modules in the audio module 160 are disposed in the processor 110.

The speaker 1601, also referred to as a "horn", is configured to convert an audio electrical signal into a sound signal. The electronic device 100 may be configured to listen to music or answer a call in a hands-free mode by using the speaker 1601.

The microphone 1602, also referred to as a "mic" or "mike", is configured to convert a sound signal into an electrical signal. When making a call or sending a voice message, a user can make a sound near the microphone 1602 through the mouth of the user to input a sound signal to the microphone 1602. At least one microphone 1602 may be disposed in the electronic device 100. In some other embodiments, the electronic device 100 may be provided with two microphones 1602, to implement a noise reduction function in addition to collecting a sound signal. In some other embodiments, three, four, or more microphones 1602 may be disposed in the electronic device 100, to collect a sound signal, reduce noise, and identify a sound source, so as to implement a directional recording function and the like.

The electronic device 100 may implement a display function through the GPU, the display screen 170, the application processor, and the like. The GPU is a microprocessor for image processing, and is connected to the display screen 170 and the application processor. The GPU is configured to perform mathematical and geometric calculation, and is configured to render an image. The processor 110 may include one or more GPUs, and the GPU executes a program instruction to generate or change display information.

The display screen 170 is configured to display an image, a video, and the like. The display screen 170 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flex light-emitting diode (flex light-emitting diode, FLED), a Miniled, a MicroLed, a Micro-oLed, a quantum dot light emitting diode (quantum dot light emitting diodes, QLED), or the like. In some embodiments, the electronic device 100 may include one or N display screens 170, where N is a positive integer greater than 1.

The power switch 180 may be configured to control power supply to supply power to the electronic device 100.

The motor 190 may generate a vibration prompt. The motor 190 may be configured to provide a vibration prompt for an incoming call, and may be further configured to provide a touch vibration feedback. For example, touch operations applied to different applications (for example, photographing and audio playback) may correspond to different vibration feedback effects. For touch operations performed on different areas of the display screen 170, the motor 190 may also correspond to different vibration feedback effects. Different application scenarios (for example, a time reminder, information receiving, an alarm clock, and a game) may also correspond to different vibration feedback effects. A touch vibration feedback effect may be further customized.

The key 1000 includes a power button, a volume button, and the like. The key 1000 may be a mechanical key, or may be a touch key. The electronic device 1000 may receive a key input and generate a key signal input related to user settings and function control of the electronic device 100.

It may be understood that the structure illustrated in this embodiment of the present invention does not constitute a specific limitation on the electronic device 100. In some other embodiments of this application, the electronic device 100 may include more or fewer components than those shown in the figure, or combine some components, or split some components, or have a different component arrangement. The components shown in the figure may be implemented by hardware, software, or a combination of software and hardware.

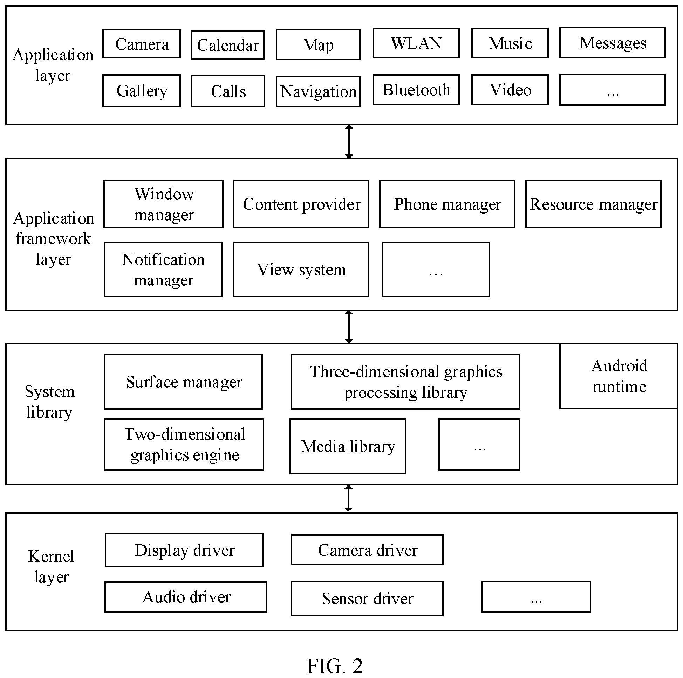

**FIG. 2** **is a block diagram of a software structure of an electronic device 100 according to an** e**mbodiment of this application.**

A software system of the electronic device 100 may use a layered architecture, an event-driven architecture, a microkernel architecture, a micro service architecture, or a cloud architecture. In this embodiment of the present invention, an Android system of a layered architecture is used as an example to illustrate the software structure of the electronic device 100.

In a layered architecture, software is divided into several layers, and each layer has a clear role and task. The layers communicate with each other through software interfaces. In some embodiments, the Android system is divided into four layers: an application layer, an application framework layer, an Android runtime (Android runtime) and system library, and a kernel layer from top to bottom.

The application layer may include a series of application packages.

As shown in FIG. 2, the application packages may include applications such as Camera, Gallery, Calendar, Calls, Map, Navigation, WLAN, Bluetooth, Music, Video, and Messages.

The application framework layer provides an application programming interface (application programming interface, API) and a programming framework for an application at the application layer. The application framework layer includes some predefined functions.

As shown in FIG. 2, the application framework layer may include a window manager, a content provider, a view system, a phone manager, a resource manager, a notification manager, and the like.

The window manager is configured to manage a window program. The window manager may obtain a size of the display screen, determine whether there is a status bar, lock the screen, take a screenshot, and the like.

The content provider is configured to store and obtain data, and enable the data to be accessed by an application. The data may include a video, an image, an audio, calls that are made and received, a browsing history and bookmarks, an address book, and the like.

The view system includes visual controls, such as a control for displaying a text and a control for displaying an image. The view system may be configured to construct an application. A display interface may include one or more views. For example, a display interface including an SMS message notification icon may include a text display view and an image display view.

The phone manager is configured to provide a communication function for the electronic device 100, for example, management of a call status (including answering, declining, or the like).

The resource manager provides various resources such as a localized character string, an icon, an image, a layout file, and a video file for an application.

The notification manager enables an application to display notification information in a status bar, and may be configured to convey a notification type message. The displayed notification information may automatically disappear after a short pause without user interaction. For example, the notification manager is configured to notify download completion, provide a message reminder, and the like. The notification manager may alternatively display a notification in a form of a chart or a scroll bar text in a status bar at the top of the system, for example, a notification of an application run in the background, or may display a notification in a form of a dialog window on the screen. For example, text information is displayed in the status bar, an announcement is given, the electronic device vibrates, or an indicator light blinks.

The Android runtime includes a kernel library and a virtual machine. The Android runtime is responsible for scheduling and management of the Android system.

The core library includes two parts: One is a function that needs to be called by a java language, and the other is a core library of Android.

The application layer and the application framework layer are run in the virtual machine. The virtual machine executes java files of the application layer and the application framework layer as binary files. The virtual machine is used to perform functions such as object lifecycle management, stack management, thread management, security and exception management, and garbage collection.

The system library may include a plurality of functional modules, for example, a surface manager (surface manager), media libraries (Media Libraries), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media library supports playback and recording in a plurality of commonly used audio and video formats, and static image files. The media library may support a plurality of audio and video encoding formats, for example, MPEG4, H.264, MP3, AAC, AMR, JPG, and PNG.

The three-dimensional graphics processing library is configured to implement three-dimensional graphics drawing, image rendering, composition, layer processing, and the like.

The 2D graphics engine is a drawing engine for 2D drawing.

The kernel layer is a layer between hardware and software. The kernel layer includes at least a display driver, a camera driver, an audio driver, and a sensor driver.

The following describes in detail a breathing guidance method provided in an embodiment of this application with reference to Embodiment 1 and Embodiment 2.

### Embodiment 1

Embodiment 1 is a breathing guidance solution for a field of exercise.

An electronic device 100 may be a mobile phone, a wearable device, a headset, smart glasses, an augmented reality (augmented reality, AR) device, a virtual reality (virtual reality, VR) device, or the like. The wearable device may be a wrist-supported device, for example, a smartwatch, a smart band, or a smart wrist strap. Alternatively, the wearable device may be an ankle-supported device, for example, a smart anklet, smart shoes, smart socks, or another device that can be worn on a leg. Alternatively, the wearable device may be a head-supported device, for example, a smart helmet or a smart head strap (which may also be referred to as a smart headband).

In the following embodiment, an example in which the electronic device 100 is a smart band is used for description. The electronic device 100 may monitor an action of a user in real time. When the electronic device 100 detects that the user performs a first action, the electronic device 100 prompts, in a first prompt manner, the user to inhale. When the electronic device 100 detects that the user performs a second action, the electronic device 100 prompts, in a second prompt manner, the user to exhale. In this way, the user may control a breathing rhythm and an exercise rhythm, and match the breathing rhythm with the exercise rhythm, so as to improve an exercise effect.

Different exercise types correspond to different user actions. Exercise types may be divided into exercise combined with fitness equipment and exercise not combined with fitness equipment. Exercise combined with fitness equipment may include, but is not limited to, running, swimming, and the like. Exercise combined with fitness equipment may include, but is not limited to, rowing, rowing machine exercise, elliptical machine exercise, weight lifting exercise, and the like. The exercise type may further include another exercise. This is not limited in this embodiment of this application.

For exercise not combined with fitness equipment, the electronic device 100 collects motion sensor data, and determines whether an action of the user is the first action or the second action based on the motion sensor data. After determining the action of the user, the electronic device 100 prompts, in the first prompt manner, the user to inhale and prompts, in the second prompt manner, the user to exhale.

For exercise combined with fitness equipment, in a possible implementation, the fitness equipment (for example, a barbell, an elliptical machine, or a rowing machine) may replace a function of the electronic device 100. That is, the fitness equipment collects the motion sensor data, and determines whether the action of the user is the first action or the second action based on the motion sensor data. After determining the action of the user, the fitness equipment prompts, in the first prompt manner, the user to inhale and prompts, in the second prompt manner, the user to exhale.

In another possible implementation, the fitness equipment needs to establish a communication connection to the electronic device 100. The fitness equipment collects the motion sensor data, and determines whether the action of the user is the first action or the second action based on the motion sensor data. After determining that the action of the user is the first action, the fitness equipment sends an instruction 1 to the electronic device 100. After receiving the instruction 1, the electronic device 100 prompts, in the first prompt manner, the user to inhale. After determining that the action of the user is the second action, the fitness equipment sends an instruction 2 to the electronic device 100. After receiving the instruction 2, the electronic device 100 prompts, in the second prompt manner, the user to inhale.

In another possible implementation, the fitness equipment needs to establish a communication connection to the electronic device 100. The fitness equipment collects motion sensor data, and the fitness equipment sends the motion sensor data to the electronic device 100 in real time. After obtaining the motion sensor data, the electronic device 100 determines, based on the motion sensor data, whether the action of the user is the first action or the second action. After determining the action of the user, the electronic device 100 prompts, in the first prompt manner, the user to inhale and prompts, in the second prompt manner, the user to exhale.

The communication connection between the fitness equipment and the electronic device 100 may be a wired connection or a wireless connection. The wireless connection may be a close-range connection such as a wireless local area network (wireless local area network, WLAN) connection, a wireless fidelity (wireless fidelity, Wi-Fi) connection, a Bluetooth connection, an infrared connection, a near field communication (near field communication, NFC) connection, ZigBee, or another wireless communication technology that appears in subsequent development. Alternatively, the fitness equipment may establish a long-distance connection to the electronic device 100. The long-distance connection includes but is not limited to a long-distance connection based on a mobile network of 2G, 3G, 4G, 5G, and a subsequent standard protocol. Alternatively, the fitness equipment and the electronic device 100 may log in to a same user account (for example, a Huawei account), and then establish a remote connection by using a server.

Because of different exercise types, corresponding user actions are also different. For example, for running, the first action may be an action in which an arm of the user swings forward, and the second action may be an action in which the arm of the user swings backward. For swimming, the first action may be an arm action of the user when the head of the user comes out of the water surface. The second action may be an arm action of the user when the head of the user goes beneath the water surface. In some embodiments, the first action and the second action may alternatively be leg actions. In the following embodiments of this application, an example in which the first action and the second action are hand actions is used for description. User actions corresponding to different exercise types are described in detail in subsequent embodiments, and are not described one by one in this embodiment of this application.

**Before the user starts to exercise, the electronic device 100 needs to enable an exercise mode of a corresponding exercise type.**

**The electronic device 100 may enable the exercise type in the following two manners. In this embodiment of this application, an example in which the electronic device 100 enables a running mode is used for description.**

### Manner 1:

**In some embodiments, the electronic device 100 receives input of the user and enables the running mode in response to the input of the user.**

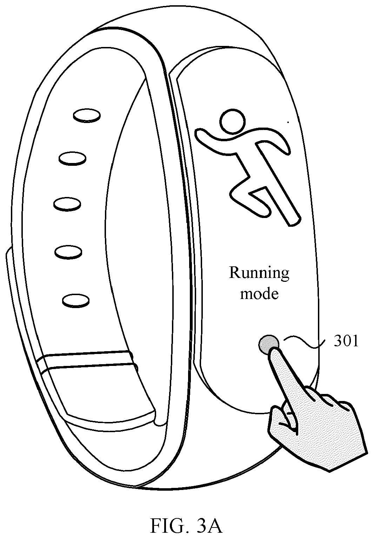

**FIG. 3A** **shows an example of a UI diagram in which the electronic device 100 enables the running mode.**

As shown in FIG. 3A, when the electronic device 100 detects a touch and hold operation performed on a touch control 301, the electronic device 100 may enable or end the running mode selected by the user. When the electronic device 100 does not enable the running mode, and detects a touch and hold operation acting on the touch control 301, the electronic device 100 may enable the running mode by counting down a specific period of time (for example, three seconds) after vibration. After the running mode is enabled, the electronic device 100 may display a running icon and a text "running mode" on a display screen. In this way, the user may be prompted that the electronic device 100 has enabled the running mode.

When the electronic device 100 has enabled the running mode, and detects a touch and hold operation acting on the touch control 301, the electronic device 100 may end the running mode by counting down a specific period of time (for example, three seconds) after vibration.

In a possible implementation, after the running mode is enabled for a period of time (for example, 1 minute or 2 minutes), the electronic device 100 may automatically turn off the display screen. This can effectively reduce power consumption of the electronic device 100. When a short press operation performed on the touch control 301 is detected, the electronic device 100 may light up the display screen. After being lit, the display screen may display a running icon and a text "running mode" shown in FIG. 3A.

**In some other embodiments, after the electronic device 100 establishes a communication connection to an electronic device 200, the electronic device 100 may further enable the running mode by receiving a running mode enabling request sent by the electronic device 200.**

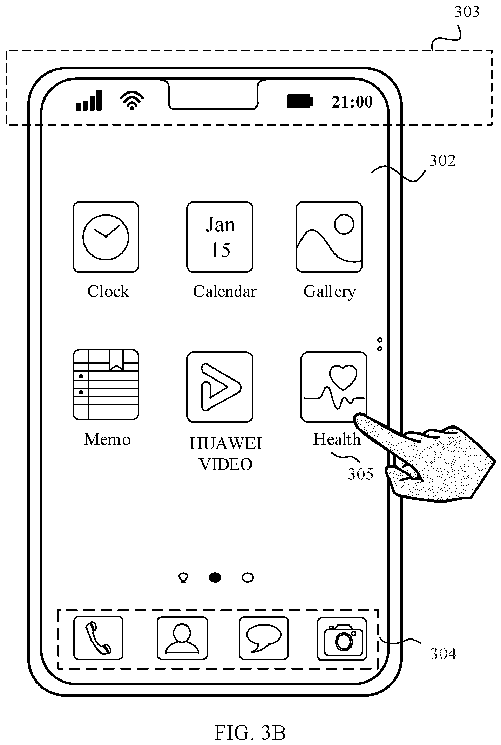

**FIG. 3B to FIG. 3D** **show examples of UI diagrams of enabling the running mode by the electronic device 100 based on the running mode enabling request sent by the electronic device 200.**

The electronic device 100 may establish a communication connection relationship with the electronic device 200 (for example, a mobile phone or a tablet computer). When detecting a user operation used to enable or end the running mode, the electronic device 200 may send, to the electronic device 100, an instruction for enabling the running mode. When the electronic device 100 receives the instruction used to enable the running mode, the electronic device 100 may enable the running mode.

As shown in FIG. 3B, the electronic device 200 displays a home screen user interface 302. The user interface 302 may include a status bar 303, a tray 304 with frequently used application icons, and other application icons. The status bar 303 may include a time indicator, a battery status indicator, one or more signal strength indicators of a wireless fidelity (wireless fidelity, Wi-Fi) signal, and one or more signal strength indicators of a mobile communication signal (which may also be referred to as a cellular signal). The tray 304 with frequently used application icons may display: a Camera icon, a Phone icon, a Contact icon, and a Messages icon. The other application icons may be, for example, a Clock icon, a Calendar icon, a Gallery icon, a Memo icon, a Huawei Video icon, and a Health icon 305. An icon of any application may be used to respond to an operation (for example, a tap operation) of the user, so that the electronic device 200 starts an application corresponding to the icon. The Health icon 305 may be used to start an application Health. The application Health may be used by the electronic device 200 to establish a communication connection relationship with the electronic device 100. The electronic device 200 may display motion data of the user to the user by using the application Health.

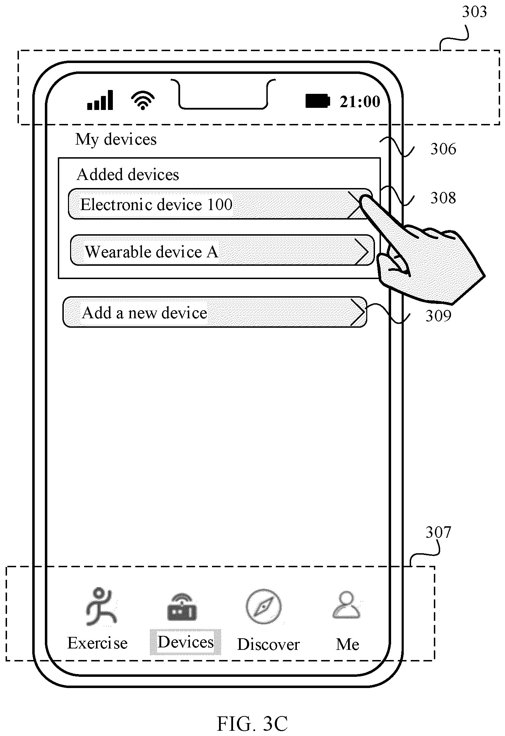

The electronic device 200 receives and responds to a user operation (for example, a tap operation) performed on the Health icon 305, and the electronic device 200 may display an application interface 306 of Health shown in FIG. 3C.

As shown in FIG. 3C, the application interface 306 may include a status bar 303 and an interface viewing option 307. The interface viewing option 307 may include an exercise option, a device option, a discover option, and a me option. Any option may be used to respond to an operation (for example, a tap operation) of the user, so that the electronic device 200 displays, on the application interface 306, content corresponding to the option. For example, content corresponding to the device option may include device information added to the electronic device and a control for adding a new device. When the electronic device 200 detects a user operation (for example, a tap operation) performed on the device option, the electronic device 200 may display an added device option 308 and a device addition option 309.

The device addition option 309 may be used to trigger the electronic device 200 to add a new device. The new device is a device that establishes a communication connection relationship with the electronic device 200 for the first time. When the electronic device 200 detects a user operation (for example, a tap operation) performed on the device addition option 309, the electronic device 200 may display a device addition setting interface, so that the electronic device 200 establishes a communication connection relationship with the new device. The device addition setting interface may be used by the user to search for a new device and a manner of establishing a communication connection, for example, a Bluetooth connection. A process in which the electronic device 200 establishes the communication connection relationship with the new device is not limited in this embodiment of this application.

The added device option 308 may include identifiers of a plurality of electronic devices. Each of the plurality of electronic devices has established a communication connection relationship with the electronic device 200. For example, the electronic device 200 has established a communication connection relationship with the electronic device 100. When a user operation (for example, a tap operation) performed on any device option in the added device option 308 is detected, the electronic device may display related information corresponding to the device.

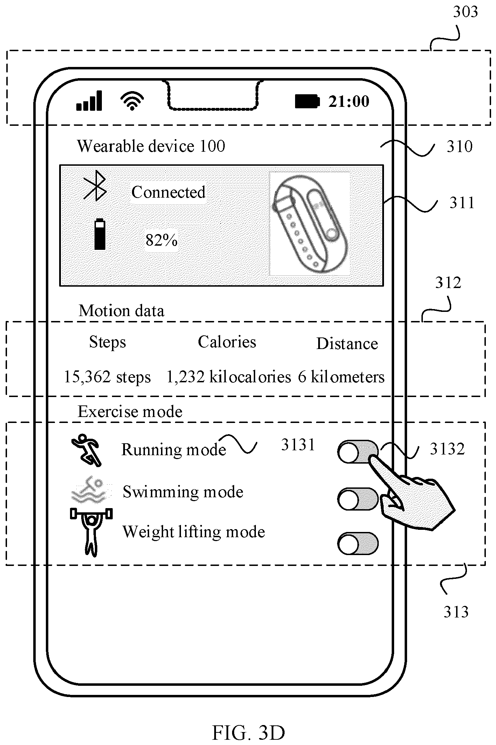

When the electronic device 200 detects a user operation (for example, a tap operation) performed on an identifier of the electronic device 100 in the added device option 308, the electronic device 200 may display an application interface 310 shown in FIG. 3D.

As shown in FIG. 3D, the application interface 310 may include a status bar 303, a device status bar 311, motion data 312, and an exercise mode option 313. The device status bar 311 may be used to display a connection status between the electronic device 100 and the electronic device 200 and a battery level of the electronic device 100. For example, when it is detected that the electronic device 200 establishes a communication connection relationship with the electronic device 100 in a Bluetooth connection manner, the device status bar 311 may prompt that the connection manner is the Bluetooth connection and the connection status is "connected". Further, the electronic device 200 may obtain battery level information of the electronic device 100. The device status bar 311 may indicate a current battery level (for example, 77%) of the electronic device 100. Content prompted by the device status bar 311 may further include more content. This is not limited in this embodiment of this application. The motion data 312 may include a quantity of steps that the user moves, calories consumed, and a movement distance that are recorded by the electronic device 100. Data in the motion data 312 is data of the user within one day that is recorded by the electronic device 100 in a working state (for example, a total quantity of steps moved, calories consumed, and the distance moved by the user during activities such as daily walking and running are included).

The exercise mode option 313 may be used to enable or end a running mode, a swimming mode, and a weight lifting mode, and the exercise mode option 313 may also include other more modes, such as an elliptical machine exercise mode, a rowing machine exercise mode, a rowing mode, and the like, which are not reflected in the exercise mode option 313. The exercise mode option 313 may include a running mode identifier 3131 and a control 3132 for enabling the running mode, a swimming mode identifier and a control for enabling the swimming mode, a weight lifting mode identifier, and a control for enabling the weight lifting mode. In response to a user operation (for example, tap) performed on the control 3132 for enabling the running mode, the electronic device 200 may send, to the electronic device 100, an instruction for enabling the running mode. When receiving the instruction that is sent by the electronic device 200 and that is for enabling the running mode, the electronic device 100 may enable the running mode a specific period of time (for example, a countdown of 3 seconds) later after vibration. When the running mode is enabled, the electronic device 100 may display a running icon and a text "running mode" on the display screen. In this way, the user may be prompted that the electronic device 100 has enabled the running mode.

When receiving an instruction that is sent by the electronic device 200 and that is for ending the running mode, the electronic device 100 may end the running mode a specific period of time (for example, a countdown of 3 seconds) later after vibration.

In addition, when the running mode is enabled, the electronic device 100 may automatically enable a do-not-disturb mode. For example, when the do-not-disturb mode is enabled, and when the electronic device 200 receives an incoming call or a message notification, the electronic device 100 may block a reminder instruction of the incoming call or the message notification sent by the electronic device. That is, the electronic device 100 does not remind the user of the incoming call or the message notification in a manner such as vibration or ringing. In this way, when the electronic device 100 has enabled the running mode, and there is an incoming call or a message notification, the electronic device 100 does not cause interference to running of the user.

**Manner 2: The electronic device 100 automatically enables the running mode based on data collected by a sensor.**

When the electronic device 100 does not enable the running mode, and detects that the user is in a running state, the electronic device 100 may automatically enable the running mode. A manner in which the electronic device 100 determines that the user is in the running state may be that a motion sensor collects motion data, for example, an acceleration sensor collects acceleration data, and the electronic device 100 obtains an acceleration waveform feature map based on the acceleration data. The electronic device 100 inputs the acceleration waveform feature map into a classification model. The classification model is trained in advance, and the classification model may obtain through analysis an exercise type (for example, running or swimming) of the user based on input motion data. If the classification model determines, through analysis based on the acceleration waveform feature map, that the user is running, the electronic device 100 may determine that the user is in the running state.

In some other embodiments, alternatively, the electronic device 100 may directly input the acceleration data collected by the acceleration sensor into the classification model, and the classification model directly outputs that the user is running or that the user is performing other exercise.

Alternatively, the electronic device 100 may determine, in another manner, that the user is in the running state. This is not limited in this embodiment of this application.

In some embodiments, when the electronic device 100 enables another exercise mode, for example, a weight lifting exercise mode, but detects that the user is in the running state, the electronic device 100 may automatically switch the another exercise mode to the running mode.

In some embodiments, when the electronic device 100 enables the running mode and detects that the user is not in the running state, the electronic device 100 may automatically end the running mode.

In this way, when the user is running and forgets to enable the running mode, or an enabled exercise mode is incorrectly selected, the electronic device 100 may adaptively enable the running mode. In addition, when the user forgets to end the running mode after enabling the running mode, the electronic device 100 may adaptively end the running mode, thereby reducing power consumption of the electronic device 100.

In some cases, after the electronic device 100 adaptively enables the running mode, the electronic device 100 may also receive a user operation to disable the running mode. The electronic device 100 is prevented from mistakenly enabling the running mode, which increases consumption of the electronic device 100.

For example, the user wears the electronic device 100 on a wrist. To cross a road as soon as possible within a specific period of time, the user trots several steps. The electronic device 100 may mistakenly consider that the user is running, and switch the exercise mode of the user from a walking mode to the running mode. To prevent the electronic device 100 from mistakenly switching the exercise mode, a cancel control displayed on the display screen of the electronic device 100 may receive an input operation of the user, so that the electronic device 100 switches the exercise mode to a previous exercise mode.

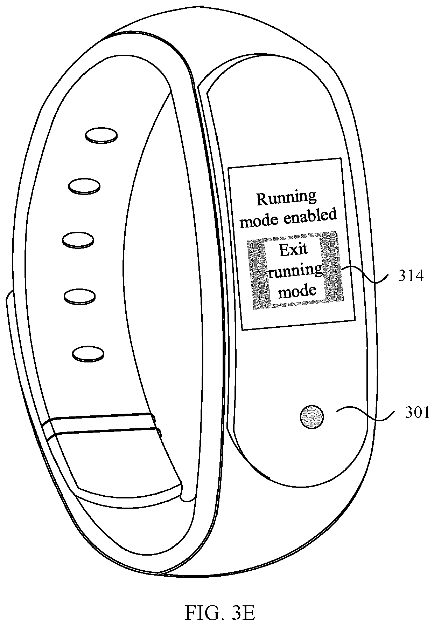

As shown in FIG. 3E, the electronic device 100 has previously enabled the walking mode, and the electronic device 100 switches the walking mode to the running mode due to mistaken identification. The electronic device 100 displays a text "running mode enabled" and a control 314 on the display screen. The electronic device 100 may receive an input operation of the user for the control 314, and in response to the input operation of the user, the electronic device 100 switches the running mode to the walking mode.

This is not limited to receiving, by the electronic device 100, an input operation of the user to switch the exercise mode to a previous exercise mode. Alternatively, the electronic device 100 may identify a voice result of the user, and switch the exercise mode to a previous exercise mode.

In addition to enabling the running mode by monitoring a user operation acting on the touch control 301 and receiving an instruction sent by the electronic device 200 in the foregoing, and adaptively enabling the running mode, the electronic device 100 may further enable the running mode in another manner, for example, enabling the running mode based on a somatosensory gesture. This is not limited in this embodiment of this application.

**In some embodiments, before enabling the exercise mode, the electronic device 100 may detect whether the motion sensor is in the working state.**

For example, before enabling the running mode, the electronic device 100 may detect whether the acceleration sensor is in the working state.

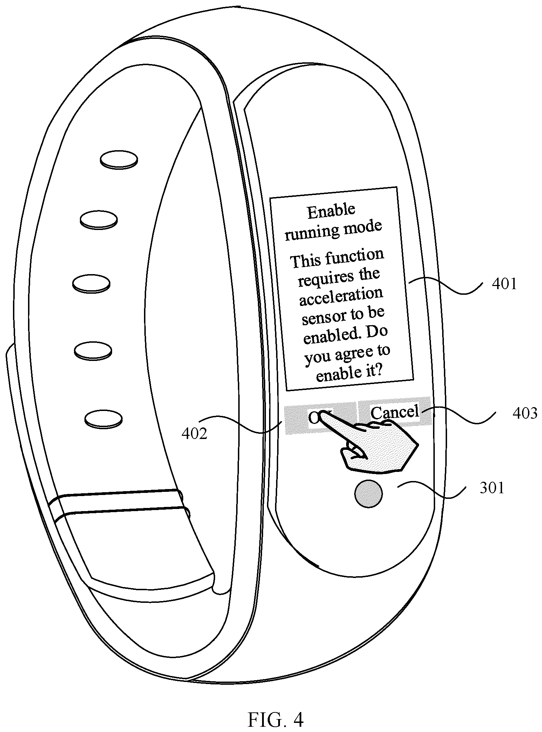

FIG. 4 is a UI diagram of the motion sensor when the electronic device 100 enables the running mode.

When a user operation for enabling the running mode is detected (for example, a touch and hold operation acting on the touch control 301 in FIG. 3A), the electronic device 100 may display, on the display screen, a user interface shown in FIG. 4. The user interface may include a prompt box 401, an OK control 402, and a cancel control 403.

The prompt box 401 includes prompt content. The prompt content is used to prompt that the motion sensor (such as the acceleration sensor) in the electronic device 100 is in the working state when the running mode is enabled, to determine whether the user needs to enable the running mode. The prompt content may include "This function requires the acceleration sensor to be enabled. Do you agree to enable it?".

The OK control 402 may be used to enable the running mode. In response to a user operation (for example, a tap operation) performed on the OK control 402, the electronic device 100 may detect whether the motion sensor (for example, the acceleration sensor) is in the working state. If the motion sensor (such as the acceleration sensor) is not in the working state, the electronic device 100 may automatically enable the motion sensor (such as the acceleration sensor) to be in the working state. In this way, the electronic device 100 may enable the running mode, and vibrate for a period of time (for example, 3 seconds) after the running mode is enabled, to remind the user that the running mode is enabled.

The cancel control 403 may be used to not enable the motion sensor in the running mode. In response to a user operation (for example, a tap operation) performed on the cancel control 403, the electronic device 100 does not enable the motion sensor in the running mode.

It should be noted that an operation of enabling a motion sensor in another exercise mode by the electronic device 100 is similar to the operation of enabling the motion sensor in the running mode by the electronic device 100 described above. A difference lies in that different exercise types relate to different types of motion sensors. Types of motion sensors related to different exercise types are described one by one in subsequent embodiments. Details are not described in this embodiment of this application.

After the electronic device 100 enables the exercise mode selected by the user, the electronic device 100 monitors an action of the user in real time, and provides breathing guidance in combination with the motion action of the user in an exercise process of the user, so that the motion action of the user matches a breathing rhythm.

Currently, the exercise mode may be divided into exercise combined with fitness equipment and exercise not combined with fitness equipment. Exercise combined with fitness equipment may include, but is not limited to, running, swimming, and the like. Exercise combined with fitness equipment may include, but is not limited to, rowing, rowing machine exercise, elliptical machine exercise, weight lifting exercise, and the like.

Breathing guidance solutions involved in exercise combined with fitness equipment and exercise not combined with fitness equipment are separately explained in detail below.

### I. Breathing guidance solution for exercise not combined with fitness equipment

**It can be learned from the foregoing analysis that exercise not combined with fitness equipment may include but is not limited to running, swimming, and the like.**

### 1. Breathing guidance solution in a running process

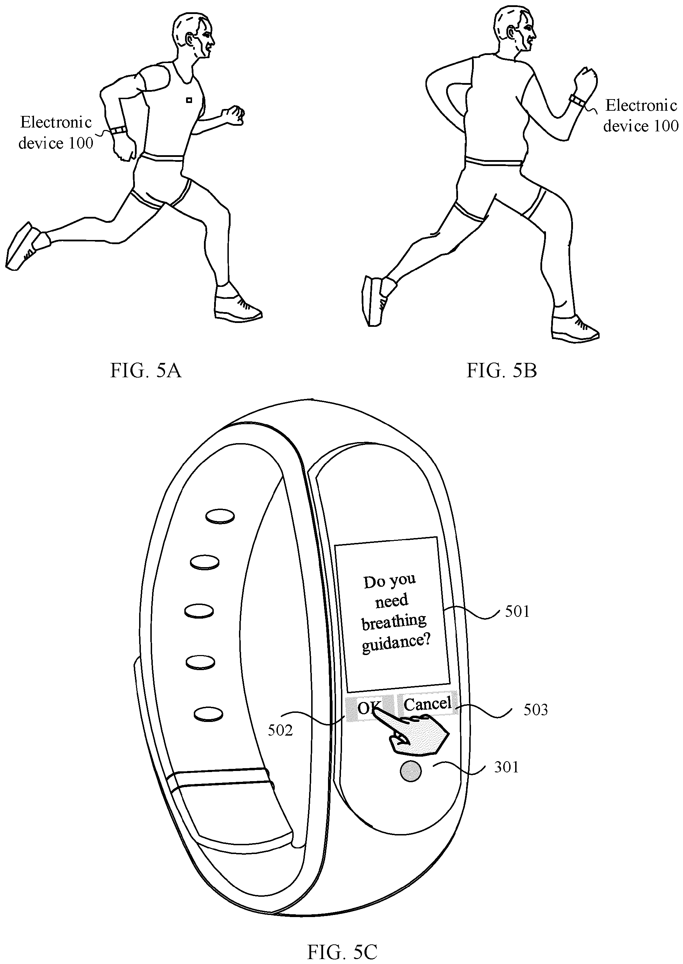

FIG. 5A to FIG. 5O show examples of UI diagrams involved in the breathing guidance solution in the running process.

FIG. 5A and FIG. 5B show examples of schematic diagrams of motion postures corresponding to a first action and a second action in the running process.

The first action may be a motion posture of an arm of the user in a process in which an action of the arm shown in FIG. 5A changes to an action of the arm shown in FIG. 5B. In other words, the first action is an arm action when the arm of the user wearing the electronic device 100 swings forward.

The second action may be an arm motion posture of the user in a process in which the action of the arm shown in FIG. 5B changes to the action of the arm shown in FIG. 5A. In other words, the first action is an arm action when the arm of the user wearing the electronic device 100 swings backward.

Optionally, the first action may alternatively be an arm action when the arm of the user wearing the electronic device 100 swings backward, and the second action may alternatively be an arm action when the arm of the user wearing the electronic device 100 swings forward.

**Optionally, before the electronic device 100 starts to detect the action of the user, the electronic device 100 may prompt, through voice and/or text information, the user whether breathing guidance is needed. In this way, the electronic device 100 may start to monitor the action of the user after soliciting an opinion of the user, thereby respecting an intention of the user. In some embodiments, the user selects the running mode. After the user starts to exercise, the electronic device 100 directly starts to monitor the action of the user without asking the user for an opinion.**

In a possible implementation, the user selects the running mode, and before the electronic device 100 starts to detect the action of the user, the electronic device 100 plays voice "Exercise is about to start. Do you need breathing guidance?". When the user replies with "yes", the electronic device 100 receives and responds to the voice reply of the user, monitors the action of the user after the user starts to exercise, and provides breathing suggestion guidance with reference to the action of the user. When the user replies with "no", the electronic device 100 receives and responds to the voice reply of the user, and the electronic device 100 does not provide breathing guidance in an exercise process of the user.

In some embodiments, the user usually wears a headset in the running process. After the electronic device 100 is paired with the headset, the user may play music in the electronic device 100 by using the headset, to increase fun in the running process. The electronic device 100 may play, by using the headset, voice "Exercise is about to start. Do you need breathing guidance?".

In another possible implementation, the user selects the running mode. Before the electronic device 100 starts to detect the action of the user, the electronic device 100 may display text information on the display screen of the electronic device 100. The text information is used to prompt the user whether breathing guidance is needed.

FIG. 5C shows an example of a UI diagram in which the electronic device 100 displays a text prompt.

The electronic device 100 may display, on the display screen, a user interface shown in FIG. 5C. The user interface may include a prompt box 501, an OK control 502, and a cancel control 503.

The prompt box 501 includes prompt content, and the prompt content is used to prompt that the electronic device 100 enables a breathing guidance function when the running mode is enabled. The text information may include "Do you need breathing guidance?".

The OK control 502 may be used to enable the breathing guidance function. In response to a user operation (for example, a tap operation) performed on the OK control 502, the electronic device 100 may enable the breathing guidance function.

The cancel control 503 may be used to not enable the breathing guidance function. In response to a user operation (for example, a tap operation) performed on the cancel control 503, the electronic device 100 does not enable the breathing guidance function.

In another possible implementation, the electronic device 100 prompts, in combination with the foregoing voice and text information manners, the user whether breathing guidance is needed. Alternatively, the electronic device 100 may notify the user in another manner, for example, the electronic device 100 vibrates, whether breathing guidance is needed. A manner in which the electronic device 100 enables the breathing guidance function is not limited in this embodiment of this application.

**Optionally, after the electronic device 100 enables the breathing guidance function, the electronic device 100 may display a picture and text information on the display screen. The picture and the text information are used to remind the user of breathing actions corresponding to different running actions.**

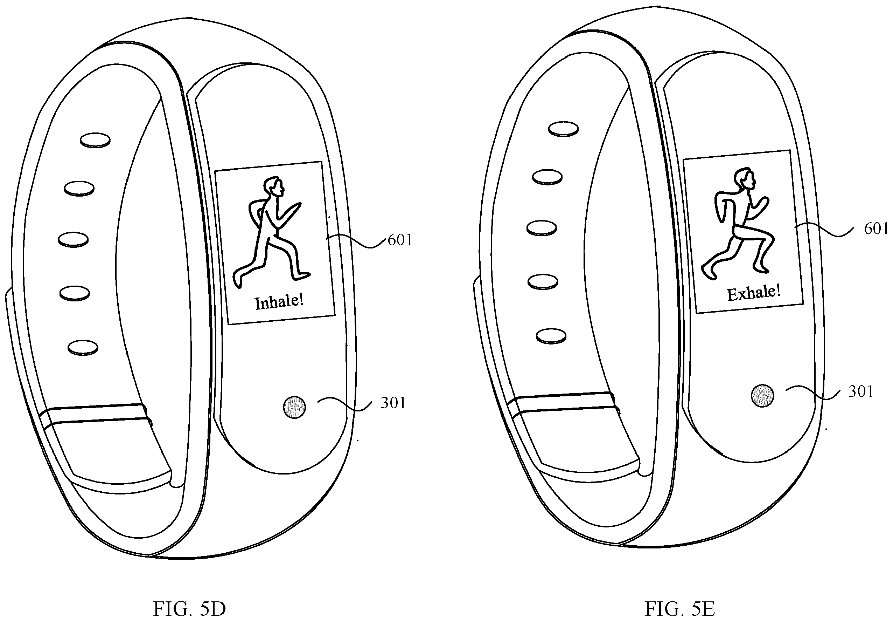

FIG. 5D to FIG. 5F show examples of UI diagrams in which the electronic device 100 displays a picture and text information.

Before the user starts to run, the electronic device 100 may display a picture on the display screen. The picture is used to indicate, to the user, breathing actions corresponding to different arm swinging actions in the running process.

FIG. 5D shows an example of a user interface in which the electronic device 100 displays an arm swing action picture corresponding to an inhale action. In FIG. 5D, an arm (for example, the right arm) of the user carrying the electronic device 100 swings forward, and a breathing action of the user is to inhale. In this way, it may be understood that, when the arm (for example, the right arm) of the user carrying the electronic device 100 swings forward, the user is in a force exerting stage, and muscles need to be tightened during force exerting, and inhalation may provide some support for the body. In FIG. 5E, when the arm (for example, the right arm) of the user carrying the electronic device 100 swings backward, the user is in a force unloading stage, and muscles relax during force unloading, so that a burden of the user can be reduced. In this way, guiding the user to breathe correctly can provide sufficient oxygen and energy for muscles, save physical energy of the user, help the user correctly and scientifically complete exercise, and enhance an exercise effect.

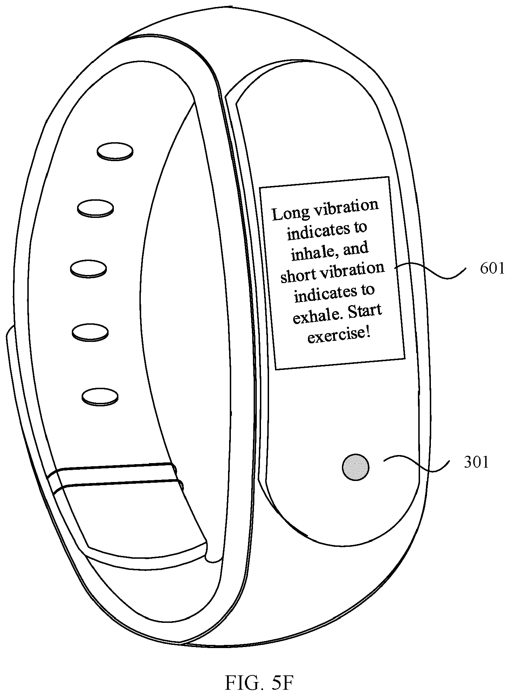

The electronic device 100 may further display, on the display screen, a user interface shown in FIG. 5F. The user interface may include a prompt box 601.

The prompt box 601 includes text information, and the text information is used to prompt breathing actions corresponding to different prompt manners. The text information may include "Long vibration indicates to inhale, and short vibration indicates to exhale". In this way, the user may perform corresponding breathing actions according to different vibration frequencies in the running process.

Optionally, after the electronic device 100 enables the breathing guidance function, the electronic device 100 may broadcast the text information in the prompt box 601 in a voice manner.

Optionally, after the electronic device 100 enables the breathing guidance function, the electronic device 100 may broadcast the text information in the prompt box 601 in the voice manner and display, on the display screen, the text information in the prompt box 601.

Optionally, in addition to displaying a picture and text information on the display screen to remind the user of breathing actions corresponding to different arm swing actions, the electronic device 100 may further display a picture and text information on another device (for example, the electronic device 200) that establishes a connection relationship with the electronic device 100, to remind the user of breathing actions corresponding to different arm swing actions.

**After the electronic device 100 enables the breathing guidance function, the electronic device 100 needs to monitor actions** (a **first action and** a **second action) of the user in the running process, and provide a specific breathing guidance solution with reference to the action of the user.**

**The following describes how the electronic device 100 determines, based on the motion data collected by the motion sensor, whether an arm motion posture of the user is the first action or the second action.**

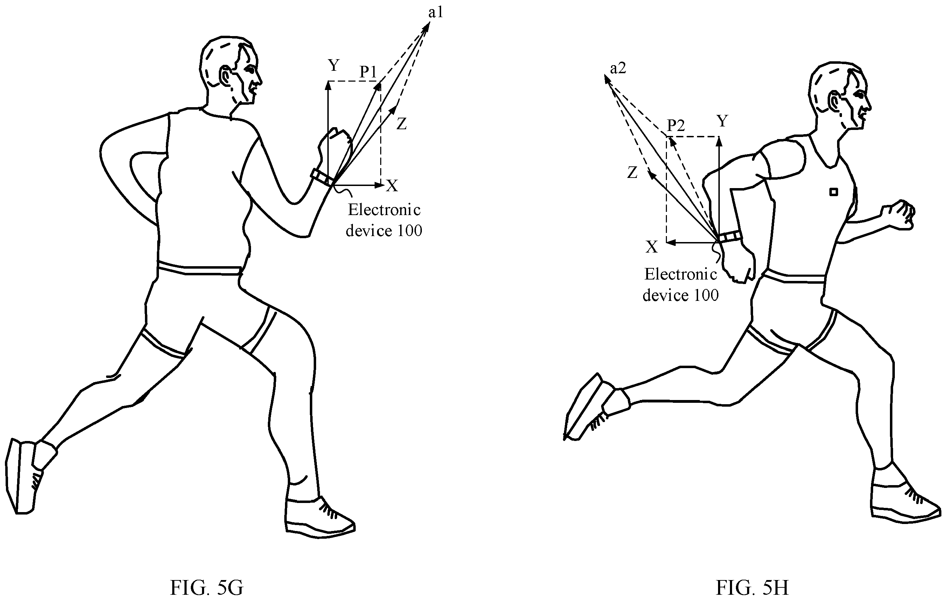

FIG. 5G to FIG. 5I show examples of schematic diagrams in which the electronic device 100 determines, based on acceleration data, whether an arm motion posture of the user is the first action or the second action.

In a possible implementation, the electronic device 100 may determine, based on acceleration data collected by an acceleration sensor, whether the user is performing the first action or the second action.

The acceleration sensor in the electronic device 100 may collect acceleration data of a wrist part of the user, and determine an arm action of the user based on the acceleration data of the wrist part of the user. The electronic device 100 collects component acceleration data in three directions of an X-axis, a Y-axis, and a Z-axis, and the electronic device 100 obtains sum acceleration data according to the component acceleration data in the three directions of the X-axis, the Y-axis, and the Z-axis, and determines, based on a magnitude of a sum acceleration, whether the user is performing the first action or the second action.

According to the foregoing, the first action is an action of swinging the arm of the user forward, and the second action is an action of swinging the arm of the user backward.

FIG. 5G shows an example of a schematic diagram of a magnitude and a direction of the sum acceleration collected by the electronic device 100 when the arm of the user swings forward.

FIG. 5G shows an example of magnitudes and directions of the component acceleration data in the three directions of the X-axis, the Y-axis, and the Z-axis. Acceleration data P1 of X-axis component acceleration data and Y-axis component acceleration data may be obtained through geometric calculation. Sum acceleration data a1 of the acceleration data P1 and Z-axis component acceleration data may be obtained through geometric calculation. It may be understood that the sum acceleration data a1 is sum acceleration data in the three directions of the X-axis, the Y-axis, and the Z-axis.

FIG. 5H shows an example of a schematic diagram of a magnitude and a direction of the sum acceleration collected by the electronic device 100 when the arm of the user swings backward.

FIG. 5H shows an example of magnitudes and directions of the component acceleration data in the three directions of the X-axis, the Y-axis, and the Z-axis. Acceleration data P2 of X-axis component acceleration data and Y-axis component acceleration data may be obtained through geometric calculation. Sum acceleration data a2 of the acceleration data P2 and Z-axis component acceleration data may be obtained through geometric calculation. It may be understood that the sum acceleration data a2 is sum acceleration data in the three directions of the X-axis, the Y-axis, and the Z-axis.

In the following embodiments of this application, when the arm of the user swings forward from a static state, a value of the sum acceleration gradually increases from zero to a maximum value, and in a process in which the arm of the user swings forward to a highest point, the value of the sum acceleration gradually decreases from the maximum value to a positive local minimum (for example, 0.3). After the arm of the user swings forward to the highest point, when the arm of the user swings backward from the highest point, the value of the sum acceleration gradually decreases from the positive local minimum (for example, 0.3) to a minimum value, and in a process in which the arm of the user swings backward to the highest point, the value of the sum acceleration increases gradually from the minimum value to a negative local maximum (for example, -0.2). The sum acceleration here is in a vector unit. That is, the sum acceleration represents not only the magnitude of the sum acceleration, but also the direction of the sum acceleration. It can be learned from the foregoing analysis that when the sum acceleration is greater than 0, it indicates that the arm of the user swings forward. When the sum acceleration is less than 0, it indicates that the arm of the user swings backward. In this way, the electronic device 100 may determine whether the arm action of the user is the first action or the second action based on that the sum acceleration data is positive or negative.

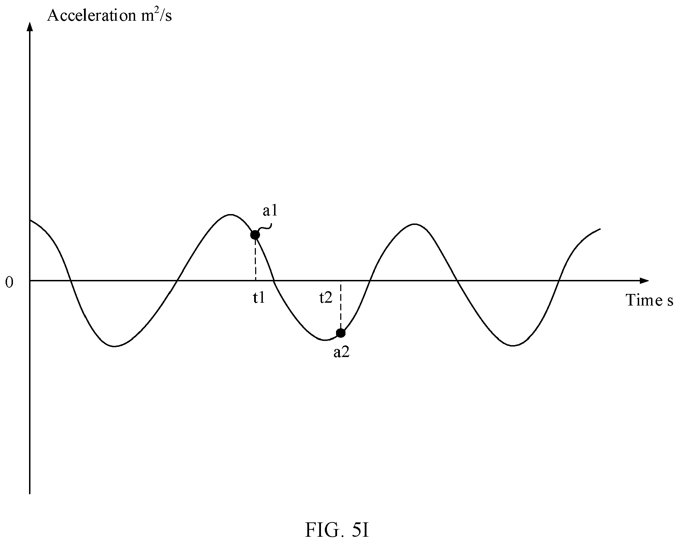

FIG. 5I shows an example of a schematic diagram of the sum acceleration collected by the electronic device 100 in the running process of the user.

In FIG. 5I, a horizontal axis represents time, and a vertical axis represents the magnitude of the sum acceleration. In FIG. 5F, when the sum acceleration is greater than 0, it indicates that the arm of the user is swinging forward; and when the sum acceleration is less than 0, it indicates that the arm of the user is swinging backward. In the running process, because the arm of the user periodically swings back and forth, the magnitude of the sum acceleration collected by the electronic device 100 also periodically changes, and the magnitude of the sum acceleration collected by the electronic device 100 periodically changes between a positive value and a negative value. For example, the sum acceleration data a1 shown in FIG. 5G may be acceleration data a1 shown at a moment t1 in FIG. 5I. The sum acceleration data a2 shown in FIG. 5H may be acceleration data a2 shown at a moment t2 in FIG. 5I.

In another possible implementation, the electronic device 100 may obtain a motion track map of the arm of the user based on the acceleration data, compare the motion track map of the arm of the user with a template, and determine whether the arm action of the user is the first action or the second action. Alternatively, the electronic device 100 may determine, in another manner, whether the arm action of the user is the first action or the second action. This is not limited in this embodiment of this application.

**In the running process of the user, when the electronic device 100 determines that the arm of the user swings forward, the electronic device 100 prompts, in a first prompt manner, the user to inhale. When the electronic device 100 determines that the arm of the user swings backward, the electronic device 100 prompts, in a second prompt manner, the user to exhale. In this way, the electronic device 100 may use different prompt manners, so that an arm swing action of the user in the running process matches a breathing rhythm, thereby saving physical strength of the user and improving an exercise capability of the user.**

Specific implementations of the first prompt manner and the second prompt manner are described in detail in the foregoing embodiment, and details are not described herein again in this embodiment of this application.

Optionally, when both the type of the first prompt manner and the type of the second prompt manner are text, and the electronic device outputs a first prompt, the electronic device may display a text "inhale" on a display screen. In addition, a size, a color, and a shape of the text displayed on the display screen may gradually change (for example, a font gradually becomes larger) as a user performs an inhalation action. When the electronic device outputs a second prompt, the electronic device may display a text "exhale" on the display screen, and a size, a color, and a shape of the text displayed on the display screen may gradually change (for example, the font gradually becomes smaller) as the user performs an exhalation action. As shown in FIG. 5J to FIG. 5L, when the electronic device 100 detects that the user performs the first action, and the electronic device 100 prompts the user to inhale, a font of a text "inhale" displayed on the display screen of the electronic device 100 gradually becomes larger. As shown in FIG. 5M to FIG. 5O, the electronic device 100 detects that the user performs the second action, and the electronic device 100 prompts the user to exhale, a font of a text "exhale" displayed on the display screen of the electronic device 100 gradually becomes smaller.

When the electronic device 100 guides, based on the arm swing action of the user in the running process, the user to perform a breathing action, after consecutively detecting the first action for n times, the electronic device 100 may prompt, in the first prompt manner, the user to perform an inhalation action once. After consecutively detecting the second action for m times, the electronic device 100 prompts, in the second prompt manner, the user to perform an exhalation action, n is a positive integer greater than or equal to 1, and m is a positive integer greater than or equal to 1. For example, when n is 1 and n=2, it may be understood as "one step, one inhale; and two steps, one exhale". That is, each time the electronic device 100 detects that the user performs the first action once, the electronic device 100 prompts, in the first prompt manner, the user to perform an inhalation action once. Each time the electronic device 100 detects that the user consecutively performs the second action twice, the electronic device 100 prompts, in the second prompt manner, the user to perform an exhalation action once. It may be understood that, in different running modes, breathing guidance provided by the electronic device 100 based on detected arm swing actions of the user is also different. A respiratory rate of jogging is lower than that of fast running. The electronic device 100 may provide different breathing guidance to the user based on different running modes, which reflects flexibility of breathing guidance of the electronic device 100.

### 2. Breathing guidance solution in a swimming process

Swimming types may be divided into breaststroke, freestyle swimming, backstroke, and butterfly stroke. Users of different swimming types also have different actions. Therefore, the electronic device 100 may further provide different breathing guidance for the user based on different swimming types and with reference to an arm action of the user during swimming.

The following separately describes correspondences between arm actions of the user in breaststroke, freestyle swimming, backstroke, and butterfly stroke and the first action and the second action.

### (1) Breaststroke

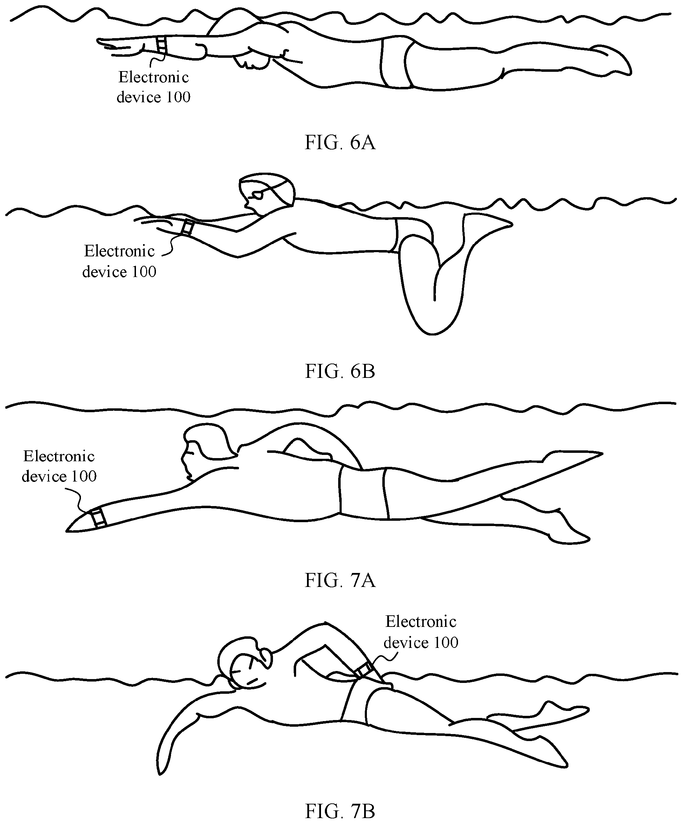

FIG. 6A and FIG. 6B show examples of schematic diagrams of the first action and the second action in breaststroke.

The first action may be a stroke posture of the arm of the user in a process in which an action of the arm shown in FIG. 6A changes to an action of the arm shown in FIG. 6B. In other words, the first action is a corresponding arm action when the arm of the user wearing the electronic device 100 strokes backward. When the arm of the user strokes backward, correspondingly, the head of the user can extend out of the water surface, and in this case, the user can inhale.

It should be noted that, in breaststroke, after completing the first action, the user remains still for a period of time (for example, 2s), and stops stroking hands and feet, that is, holds a posture action shown in FIG. 6B for 2s. In this way, physical energy can be saved. That is, after the user completes the first action, the head of the user extends out of the water surface, and the user can inhale.

The second action may be a stroke posture of the arm of the user in a process in which the action of the arm shown in FIG. 6B changes to the action of the arm shown in FIG. 6A. In other words, the second action is a corresponding arm action when the arm of the user wearing the electronic device 100 strokes forward. When the arm of the user strokes forward, correspondingly, the head of the user can extend into the water surface, and in this case, the user can exhale.

It should be noted that, in the swimming process, after completing the second action, the user remains still for a period of time (for example, 3s), and stops stroking hands and feet, that is, holds a posture action shown in FIG. 6A for 3s. In this way, physical energy can be saved. In a possible implementation, after completing the second action, the user may perform an exhalation action. In another possible implementation, the user may alternatively perform an exhalation action when just starting to perform the second action.

An action in which the arm of the user is still after the first action is completed and before the second action starts to be performed may be referred to as a third action. An arm action corresponding to the third action is static.

When the user is still, that is, the electronic device 100 detects that the user is performing the third action, the electronic device 100 may prompt, in a third prompt manner, the user to perform a breath holding action, or prompt the user to perform one or more groups of rapid exhalation and inhalation actions. A type of the third prompt manner may be any one or more of vibration, voice, text, and picture.

### (2) Freestyle swimming

FIG. 7A and FIG. 7B show examples of schematic diagrams of the first action and the second action in freestyle swimming.

The first action may be a stroke posture of the arm of the user in a process in which an action of the arm shown in FIG. 7A changes to an action of the arm shown in FIG. 6B. In other words, the first action is a corresponding arm action when the arm of the user wearing the electronic device 100 strokes upward. When the arm of the user strokes upward, correspondingly, the head of the user can extend out of the water surface, and in this case, the user can inhale.

It should be noted that, in freestyle swimming, after completing the first action, the user remains still for a period of time (for example, 2s), and stops stroking hands and feet, that is, holds a posture action shown in FIG. 7B for 2s. In this way, physical energy can be saved. That is, after the user completes the first action, the head of the user extends out of the water surface, and the user can inhale.

The second action may be a stroke posture of the arm of the user in a process in which the action of the arm shown in FIG. 7B changes to the action of the arm shown in FIG. 7A In other words, the second action is a corresponding arm action when the arm of the user wearing the electronic device 100 strokes downward. When the arm of the user strokes downward, correspondingly, the head of the user can extend into the water surface, and in this case, the user can inhale.

It should be noted that, in freestyle swimming, after completing the second action, the user remains still for a period of time (for example, 3s), and stops stroking hands and feet, that is, holds a posture action shown in FIG. 7A for 3s. In this way, physical energy can be saved. In a possible implementation, after completing the second action, the user may perform an exhalation action. In another possible implementation, the user may alternatively perform an exhalation action when just starting to perform the second action.

An action in which the arm of the user is still after the first action is completed and before the second action starts to be performed may be referred to as a third action. An arm action corresponding to the third action is static.

When the user is still, that is, the electronic device 100 detects that the user is performing the third action, the electronic device 100 may prompt, in a third prompt manner, the user to perform a breath holding action, or prompt the user to perform one or more groups of rapid exhalation and inhalation actions. A type of the third prompt manner may be any one or more of vibration, voice, text, and picture.

### (3) Backstroke

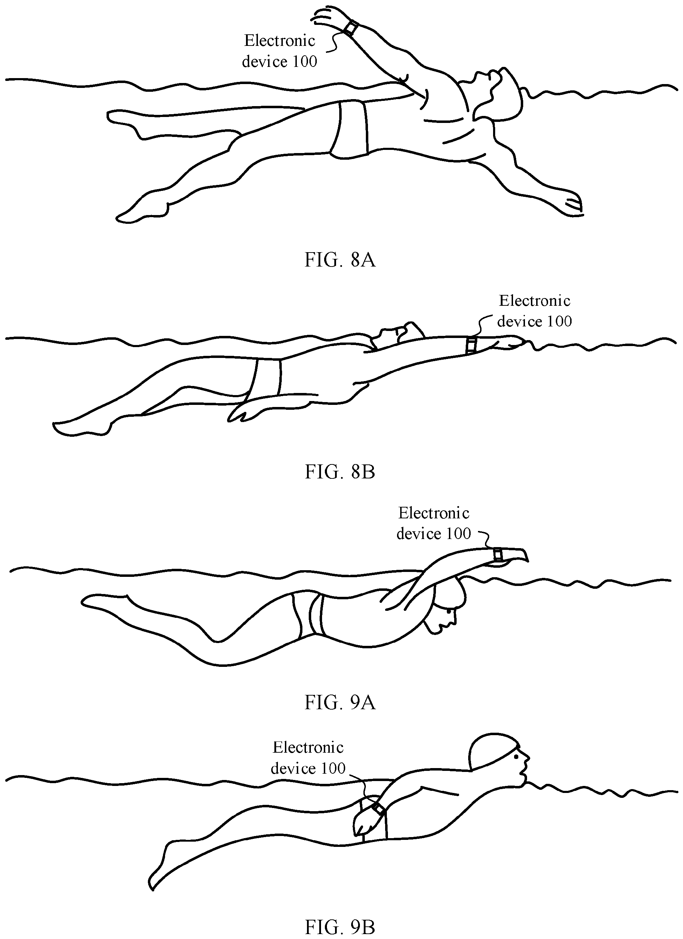

FIG. 8A and FIG. 8B show examples of schematic diagrams of the first action and the second action in backstroke.

The first action may be a stroke posture of the arm of the user in a process in which an action of the arm shown in FIG. 8A changes to an action of the arm shown in FIG. 8B. In other words, the first action is a corresponding arm action when the arm of the user wearing the electronic device 100 strokes upward. When the arm of the user strokes upward, the user can inhale.

It should be noted that, in backstroke, after completing the first action, the user remains still for a period of time (for example, 2s), and stops stroking hands and feet, that is, holds a posture action shown in FIG. 8B for 2s. In this way, physical energy can be saved. In backstroke, the head of the user is always out of the water surface. In a possible implementation, after completing the first action, the user performs an inhalation action. In another possible implementation, the user may alternatively perform an inhalation action when just starting to perform the first action. The second action may be a stroke posture of the arm of the user in a process in which the action of the arm shown in FIG. 8B changes to the action of the arm shown in FIG. 8A. In other words, the second action is a corresponding arm action when the arm of the user wearing the electronic device 100 strokes downward. When the arm of the user strokes downward, the user can inhale.

Optionally, the first action may alternatively be a stroke posture of the arm of the user in a process in which the action of the arm shown in FIG. 8B changes to the action of the arm shown in FIG. 8A, and the second action may alternatively be a stroke posture of the arm of the user in a process in which the action of the arm shown in FIG. 8A changes to the action of the arm shown in FIG. 8B. This is not limited in this embodiment of this application.

It should be noted that, in backstroke, after completing the second action, the user remains still for a period of time (for example, 3s), and stops stroking hands and feet, that is, holds a posture action shown in FIG. 8A for 3s. In this way, physical energy can be saved. In backstroke, the head of the user is always out of the water surface. In a possible implementation, after completing the second action, the user performs an inhalation action. In another possible implementation, the user may alternatively perform an inhalation action when just starting to perform the second action.

An action in which the arm of the user is still after the first action is completed and before the second action starts to be performed may be referred to as a third action. An arm action corresponding to the third action is static.

When the user is still, that is, the electronic device 100 detects that the user is performing the third action, the electronic device 100 may prompt, in a third prompt manner, the user to perform a breath holding action, or prompt the user to perform one or more groups of rapid exhalation and inhalation actions. A type of the third prompt manner may be any one or more of vibration, voice, text, and picture.

### (4) Butterfly stroke

FIG. 9A and FIG. 9B show examples of schematic diagrams of the first action and the second action in butterfly stroke.

The first action may be a stroke posture of the arm of the user in a process in which an action of the arm shown in FIG. 9A changes to an action of the arm shown in FIG. 9B. In other words, the first action is a corresponding arm action when the arm of the user wearing the electronic device 100 strokes downward. When the arm of the user strokes downward, the user can inhale.

It should be noted that, in butterfly stroke, after completing the first action, the user remains still for a period of time (for example, 2s), and stops stroking hands and feet, that is, holds a posture action shown in FIG. 9B for 2s. In this way, physical energy can be saved. That is, after the user completes the first action, the head of the user extends out of the water surface, and the user can inhale.

The second action may be a stroke posture of the arm of the user in a process in which the action of the arm shown in FIG. 9B changes to the action of the arm shown in FIG. 9A In other words, the second action is a corresponding arm action when the arm of the user wearing the electronic device 100 strokes upward. When the arm of the user strokes upward, the user can inhale. It should be noted that, in butterfly stroke, after completing the second action, the user remains still for a period of time (for example, 3s), and stops stroking hands and feet, that is, holds a posture action shown in FIG. 9A for 3s. In this way, physical energy can be saved. In a possible implementation, after completing the second action, the user may perform an exhalation action. In another possible implementation, the user may alternatively perform an exhalation action when just starting to perform the second action.

An action in which the arm of the user is still after the first action is completed and before the second action starts to be performed may be referred to as a third action. An arm action corresponding to the third action is static.

When the user is still, that is, the electronic device 100 detects that the user is performing the third action, the electronic device 100 may prompt, in a third prompt manner, the user to perform a breath holding action, or prompt the user to perform one or more groups of rapid exhalation and inhalation actions. A type of the third prompt manner may be any one or more of vibration, voice, text, and picture.

Optionally, before the electronic device 100 starts to detect the action of the user, the electronic device 100 may prompt, through voice and/or text information, the user whether breathing guidance is needed. In this way, the electronic device 100 may start to monitor the action of the user after soliciting an opinion of the user, thereby respecting an intention of the user. In some embodiments, the user selects the swimming mode. After the user starts to exercise, the electronic device 100 directly starts to monitor the action of the user without asking the user for an opinion. Herein, for how to enable breathing guidance in the swimming mode, refer to the description of how to enable breathing guidance in the running mode. Principles are similar, but exercise modes are different. Details are not described herein again in this embodiment of this application.

Optionally, after the electronic device 100 enables the breathing guidance function, the electronic device 100 may display a picture and text information on the display screen. The picture and the text information are used to remind the user of breathing actions corresponding to different swimming actions. Herein, for how the electronic device 100 reminds the user of breathing actions corresponding to different swimming actions, refer to how the electronic device 100 reminds the user of breathing actions corresponding to different running actions in the running mode. Principles are similar, but exercise modes are different. Details are not described herein again in this embodiment of this application.

In a possible implementation, the electronic device 100 may determine, based on acceleration data collected by a motion sensor (for example, an acceleration sensor), whether the arm action of the user is the first action or the second action.

In another possible implementation, the electronic device 100 may obtain a motion track map of the arm of the user based on the acceleration data collected by the motion sensor (for example, the acceleration sensor), compare the motion track map of the arm of the user with a template, and determine whether the arm action of the user is the first action or the second action. This is not limited in this embodiment of this application.

After the electronic device 100 enables the breathing guidance function, the electronic device 100 needs to monitor actions (a first action and a second action) of the user in the swimming process, and provide a specific breathing guidance solution with reference to the action of the user. A difference between swimming and running is that, in swimming, after the user strokes the arm to make the head extend out of the water surface, the user inhales. Generally, the head remains above the water surface for a period of time (for example, 2 seconds). Then, after the user strokes the arm to make the head enter the water surface, the user exhales. Generally, the head remains beneath the water surface for a period of time (for example, 2 seconds). After that, the above actions are repeated. In this case, it may be understood that, after the electronic device 100 detects that the user performs the first action, and completes the first action, the head of the user extends out of the water surface, the electronic device 100 prompts, in the first prompt manner, the user to inhale. When the electronic device 100 detects that the user just starts to perform the second action, the electronic device 100 prompts, in the second prompt manner, the user to exhale; or after the electronic device 100 detects that the user performs the second action, and completes the second action, the electronic device 100 prompts, in the second prompt manner, the user to exhale.

FIG. 6A, FIG. 6B, FIG. 7A, FIG. 7B, FIG. 8A, FIG. 8B, FIG. 9A, and FIG. 9B are all described by using an example in which the electronic device 100 is a smart band, and the electronic device 100 collects motion data of an arm. In another embodiment, when the user is swimming, the smart band is generally not worn. In this case, the electronic device 100 may be any one of smart swimming goggles, smart earplugs, a headset, a smart swimming cap, or the like. A device such as smart swimming goggles, smart earplugs, a headset, or a smart swimming cap may collect motion data of the head of the user, and determine an action type of the user based on the motion data of the head.

Optionally, when the electronic device 100 is any one of smart swimming goggles, smart earplugs, a headset, or a smart swimming cap, and the head of the user extends out of the water surface, the electronic device 100 determines that the user is performing the first action. When the head of the user enters the water surface, the electronic device 100 determines that the user is performing the second action. Because pressure inside the water surface is greater than pressure outside the water surface, the electronic device 100 may collect pressure sensor data, and determine, based on the pressure sensor data, whether the user performs the first action or the second action. Specifically, when the electronic device 100 determines that the pressure sensor data suddenly increases, the electronic device 100 determines that the user is performing the first action, that is, the head of the user extends out of the water surface. When the electronic device 100 determines that the pressure sensor data suddenly decreases, the electronic device 100 determines that the user is performing the second action, that is, the head of the user enters the water surface.

Optionally, in the exercise process of the user, the electronic device 100 may monitor both a respiratory rate and a stride frequency of the user, to determine whether the respiratory rate and the stride frequency of the user are consistent. If they are inconsistent, the electronic device 100 provides an adjustment suggestion. Specifically, the electronic device 100 may collect the respiratory rate of the user by using a microphone built in the electronic device 100, or the headset is connected to the electronic device 100, and the user listens to music while exercising with the headset; or the electronic device 100 may collect the respiratory rate of the user by using a microphone on the headset connected to the electronic device 100. The electronic device 100 may record an exercise status by using exercise software (for example, a health application). The exercise status includes but is not limited to a motion track, a stride length and a stride frequency in the exercise process, and the like. When the electronic device 100 detects that the respiratory rate of the user does not match the stride frequency, the electronic device 100 may prompt, through voice, text, vibration, or the like, the user to adjust the respiratory rate or the stride frequency.

Optionally, when the electronic device 100 guides, based on the arm action or the head action of the user in the swimming process, the user to perform a breathing action, after consecutively detecting the first action for n times, the electronic device 100 may prompt, in the first prompt manner, the user to perform an inhalation action once. After consecutively detecting the second action for m times, the electronic device 100 prompts, in the second prompt manner, the user to perform an exhalation action, where n is a positive integer greater than or equal to 1, and m is a positive integer greater than or equal to 1. For example, m may be 1, and n may be 1, that is, "stroke once and inhale once". Each time the electronic device 100 detects that the user performs the first action once, the electronic device 100 prompts, in the first prompt manner, the user to perform an inhalation action once. Each time the electronic device 100 detects that the user performs the second action once, the electronic device 100 prompts, in the second prompt manner, the user to perform an exhalation action once. It may be understood that, in different swimming modes, breathing guidance provided by the electronic device 100 based on detected arm swing action or head action of the user is also different. That is, breaststroke, backstroke, freestyle swimming, and butterfly stroke have different respiratory rates. The electronic device 100 may provide different breathing guidance to the user based on different swimming modes, which reflects flexibility of breathing guidance of the electronic device 100.

In this way, the electronic device 100 may use different prompt manners, so that an arm swing action of the user in the swimming process matches a breathing rhythm, thereby saving physical strength of the user and improving a swimming capability of the user.

### II. Breathing guidance solution for exercise combined with fitness equipment

As can be learned from the foregoing, exercise combined with fitness equipment may include, but is not limited to, weight lifting exercise, rowing, rowing machine exercise, elliptical machine exercise, and the like.

The user inhales when the user is performing the first action in a process of performing exercise combined with fitness equipment. When the user is performing the second action, the user exhales. In this case, the electronic device 100 may prompt the user to inhale when detecting that the user is performing the first action; and prompt the user to exhale when detecting that the user is performing the second action. In this way, the exercise rhythm of the user can match the breathing rhythm, and the exercise capability of the user can be improved.

Next, motion postures corresponding to action types of the user in weight lifting exercise, rowing, rowing machine exercise, and elliptical machine exercise are separately described.

### 1. Motion postures corresponding to the first action and the second action of the user in a weight lifting process.

Weight lifting may be classified into standing weight lifting, lying weight lifting, and the like. In the following embodiments of this application, lying weight lifting is used as an example for description.

In some embodiments, the first action and the second action may be arm actions of the user collected by the electronic device 100.

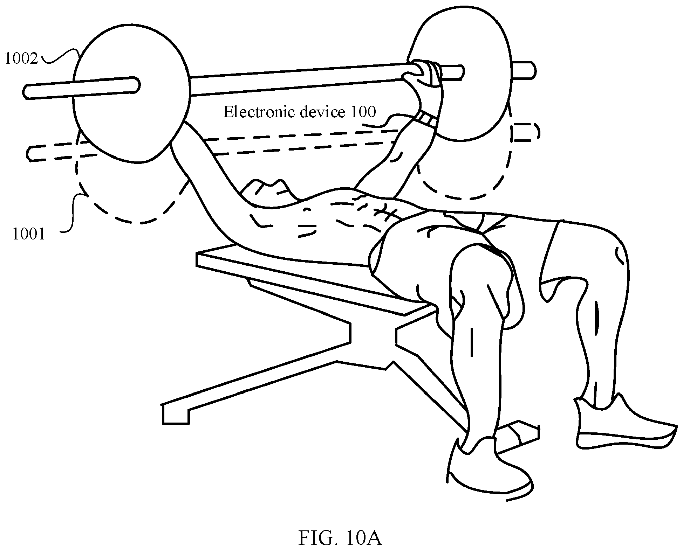

FIG. 10A shows an example of a schematic diagram of the first action in the weight lifting process.

In a process in which the user completes pushing up a barbell, a wrist of the user moves upward from a lowest point to a highest point perpendicular to the ground, and correspondingly a posture of the barbell changes from a posture 1001 to a posture 1002. A barbell posture formed by a solid line is the posture 1002, and a barbell posture formed by a dashed line is the posture 1001. The first action may be a push-up action of the arm in a process of changing the barbell posture 1001 to the posture 1002 shown in FIG. 10A.

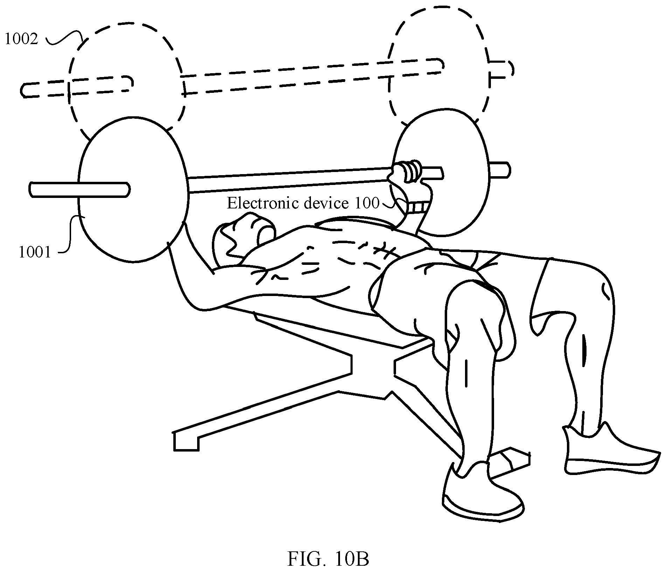

FIG. 10B shows an example of a schematic diagram of the second action in the weight lifting process.

In a process in which the user completes pulling down the barbell, the wrist of the user moves downward from the highest point to the lowest point perpendicular to the ground, and correspondingly the posture of the barbell changes from the posture 1002 to the posture 1001. A barbell posture formed by a dashed line is the posture 1002, and a barbell posture formed by a solid line is the posture 1001. The second action may be a pull-down action of the arm in a process of changing the barbell posture 1002 to the posture 1001 shown in FIG. 10B.

Optionally, the first action may alternatively be a pull-down action of the arm in the process of changing the barbell posture 1002 to the posture 1001. The second action may alternatively be a push-up action of the arm in the process of changing the barbell posture 1001 to the posture 1002.

It should be noted that, in the weight lifting process, after the user completes the first action, the barbell is located at the highest point, and the barbell posture is the posture 1002. Generally, the user keeps the barbell posture 1002 at the highest point for a period of time, and then the user performs the second action, so that the barbell posture changes from the posture 1002 to the posture 1001. Therefore, the arm action when the barbell is kept at the posture 1002 may be referred to as a third action. An arm action corresponding to the third action is static, and the wrist of the user is located at the highest point. When the user is still, that is, the electronic device 100 detects that the user is performing the third action, the electronic device 100 may prompt, in a third prompt manner, the user to perform a breath holding action, or prompt the user to perform one or more groups of rapid exhalation and inhalation actions. A type of the third prompt manner may be any one or more of vibration, voice, text, and picture.

### 2. Motion postures corresponding to the first action and the second action of the user in a rowing process.

In some embodiments, the first action and the second action may be arm actions of the user collected by the electronic device 100.

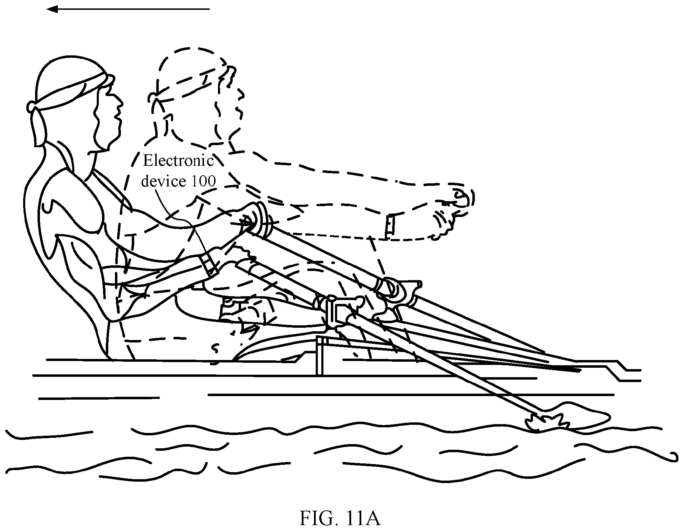

FIG. 11A shows an example of a schematic diagram of the first action in the rowing process.

FIG. 11A shows an example of a schematic diagram of the first action of the user in the rowing process.

In a process in which the user pulls a grip rod of an oar backward, the wrist of the user moves backward from a static state, and correspondingly the body of the user also moves as the grip rod of the oar moves backward. In this case, the first action may be an arm action in a process in which the user moves from a dashed-line image to a solid-line image shown in FIG. 11A, that is, the first action is an action of moving backward and retracting the arm.

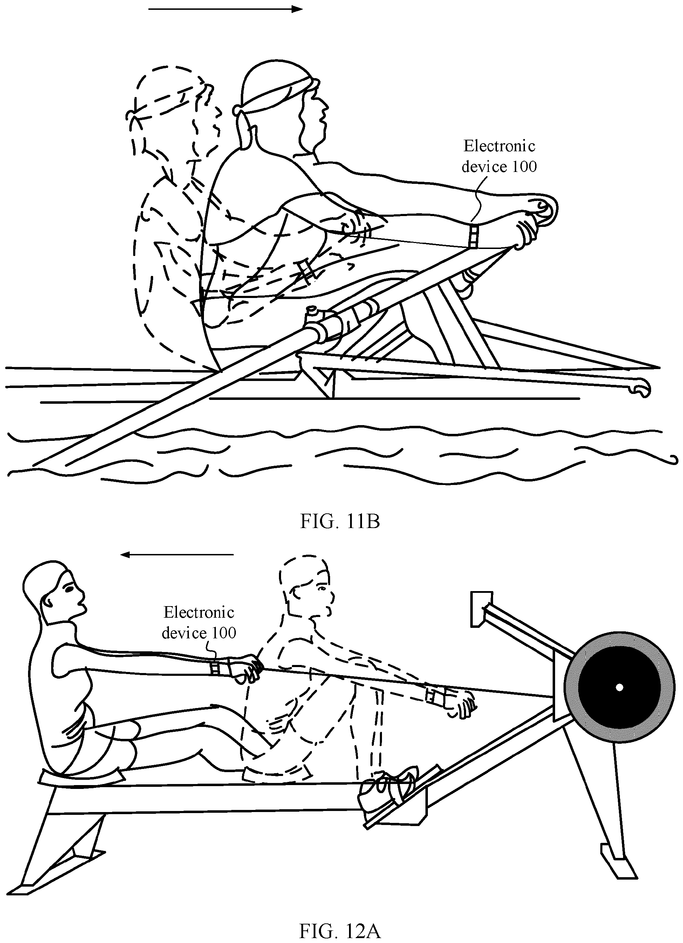

FIG. 11B shows an example of a schematic diagram of the second action of the user in the rowing process.

In a process in which the user pushes the grip rod of the oar forward, the wrist of the user moves forward from the static state, and correspondingly the body of the user also moves as the grip rod of the oar moves forward. In this case, the second action may be an arm action in a process in which the user moves from a dashed-line image to a solid-line image shown in FIG. 11B, that is, the second action is an action of moving forward and extending the arm.

Optionally, the first action may alternatively be an action of moving forward and extending the arm. The second action may alternatively be an action of moving backward and retracting the arm.

### 3. Motion postures corresponding to the first action and the second action of the user in a process of performing exercise by using a rowing machine.

In some embodiments, the first action and the second action may be actions of a wrist portion of the user collected by the electronic device 100.

FIG. 12A shows an example of a schematic diagram of the first action of the user in the process of using the rowing machine.

In a process in which the user pulls a handle of the rowing machine backward, the wrist of the user moves backward from an initial position away from the rowing machine, and correspondingly the body of the user also moves as the handle of the rowing machine moves backward. In this case, the first action may be an arm action in a process in which the user moves from a dashed-line image to a solid-line image shown in FIG. 11A, that is, the first action is an arm action of moving backward and away from the rowing machine.

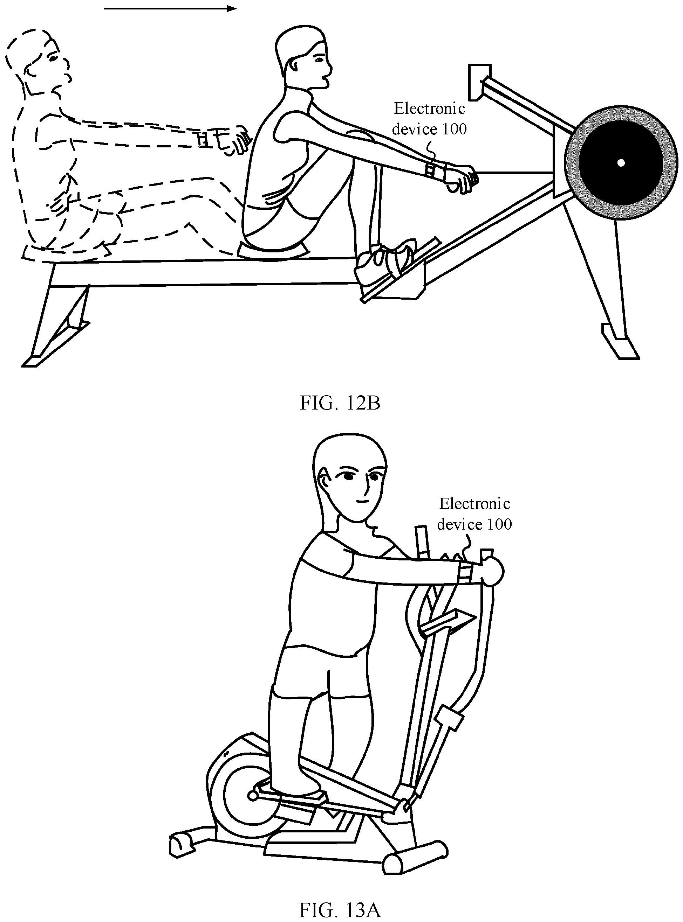

FIG. 12B shows an example of a schematic diagram of the second action of the user in the process of using the rowing machine.

In a process in which the user puts the handle of the rowing machine back to the initial position, the wrist of the user moves forward close to the rowing machine, and correspondingly the body of the user moves as the pull rod of the rowing machine moves forward. In this case, the second action may be an arm action in a process in which the user moves from a dashed-line image to a solid-line image shown in FIG. 11B, that is, the second action is an arm action of moving forward and close to the rowing machine.

Optionally, the first action may alternatively be an arm action of moving forward and close to the rowing machine. The second action may alternatively be an arm action of moving backward and away from the rowing machine.

### 4. Motion postures corresponding to the first action and the second action of the user in a process of performing exercise by using an elliptical machine.

In some embodiments, the first action and the second action may be actions of a wrist portion of the user collected by the electronic device 100.

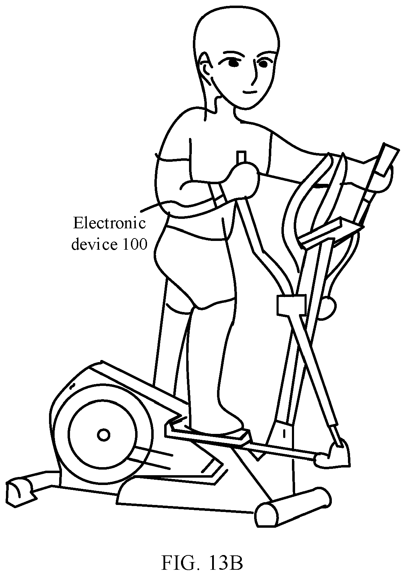

FIG. 13A and FIG. 13B show examples of schematic diagrams of the first action and the second action of the user in the process of using the elliptical machine.

In a process in which the user pulls an armrest of the elliptical machine backward, the wrist of the user moves backward from an initial position away from the elliptical machine, and correspondingly the body of the user also moves as the armrest of the elliptical machine moves backward. In this case, the first action may be a motion posture of the arm of the user in a process in which an action of the arm shown in FIG. 13A changes to an action of the arm shown in FIG. 13B. When the arm moves backward, the user can inhale.

In a process in which the user pushes the armrest of the elliptical machine forward, the wrist of the user moves forward and close to the elliptical machine, and correspondingly the body of the user also moves as the armrest of the elliptical machine moves forward. In this case, the second action may be a motion posture of the arm of the user in a process in which an action of the arm shown in FIG. 13B changes to an action of the arm shown in FIG. 13A. When the arm moves forward, the user can exhale.

Optionally, the first action may alternatively be an arm action of moving forward and close to the elliptical machine. The second action may alternatively be an arm action of moving backward and away from the elliptical machine.

**Optionally, before the electronic device 100 starts to detect the action of the user, the electronic device 100 may prompt, through voice and/or text information, the user whether breathing guidance is needed. In this way, the electronic device 100 may start to monitor the action of the user after soliciting an opinion of the user, thereby respecting an intention of the user. In some embodiments, the user selects a corresponding exercise mode. After the user starts the exercise, the electronic device 100 directly starts to monitor the action of the user without asking the user for an opinion.**

Herein, for how to enable breathing guidance in the foregoing several types of exercise combined with fitness equipment, refer to the description of how to enable breathing guidance in the running mode. Principles are similar, but exercise modes are different. Details are not described herein again in this embodiment of this application.

Optionally, after the electronic device 100 enables the breathing guidance function, the electronic device 100 may display a picture and text information on the display screen. The picture and the text information are used to remind the user of breathing actions corresponding to arm actions in the foregoing several types of exercise combined with fitness equipment. Herein, for how the electronic device 100 reminds the user of breathing actions corresponding to arm actions in the foregoing several types of exercise combined with fitness equipment, refer to how the electronic device 100 reminds the user of breathing actions corresponding to different running actions in the running mode. Principles are similar, but exercise modes are different. Details are not described herein again in this embodiment of this application.

**After the electronic device 100 enables the breathing guidance function, the electronic device 100 needs to monitor actions (a first action and a second action) of the user in the exercise process, and provide a specific breathing guidance solution with reference to the action of the user.**

**The following separately describes how the electronic device 100 determines, based on the motion data collected by the motion sensor, whether the user is performing the first action or the second action in weight lifting exercise, rowing, rowing machine exercise, and elliptical machine exercise.**

**I. In weight lifting exercise, the electronic device 100 determines specific implementations of the first action, the second action, and the third action of the user based on the motion data collected by the motion sensor.**

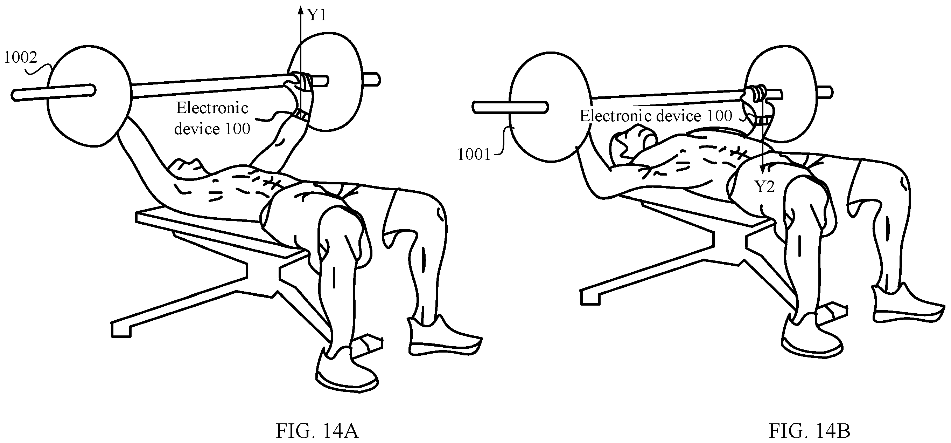

FIG. 14A to FIG. 14C show examples of schematic diagrams in which the electronic device 100 determines, based on acceleration data, that arm motion postures of the user are the first action, the second action, and the third action.

In a possible implementation, the electronic device 100 may determine, based on the acceleration data collected by the acceleration sensor, that the user is performing the first action, the second action, and the third action.

The acceleration sensor in the electronic device 100 may collect acceleration data of a wrist part of the user, and determine an arm action of the user based on the acceleration data of the wrist part of the user. The electronic device 100 collects component acceleration data in three directions of an X-axis, a Y-axis, and a Z-axis, and the electronic device 100 obtains sum acceleration data according to the component acceleration data in the three directions of the X-axis, the Y-axis, and the Z-axis, and determines, based on a magnitude of a sum acceleration, whether the user is performing the first action or the second action.

As can be learned from the foregoing, the first action is a push-up action of the arm in the process of changing the barbell posture 1001 to the posture 1002, and the second action is a pull-down action of the arm in the process of changing the barbell posture 1002 to the posture 1001.

In weight lifting exercise, because the arm of the user moves up and down, in an ideal case, it may be understood that component acceleration data collected by the electronic device 100 in three directions of an X-axis and a Z-axis is 0. In this case, the electronic device 100 collects only component acceleration data in a direction of a Y-axis.

FIG. 14A shows an example of a schematic diagram of a magnitude and a direction of a sum acceleration collected by the electronic device 100 in a push-up process of the arm of the user.

In FIG. 14A, component acceleration data that is in the direction of the Y-axis and that is collected by the electronic device 100 is acceleration data Y1. A direction of the acceleration data Y1 is upward and perpendicular to a horizontal plane. It may be understood that the acceleration data Y1 is sum acceleration data in the three directions of the X-axis, the Y-axis, and the Z-axis.

FIG. 14B shows an example of a schematic diagram of a magnitude and a direction of a sum acceleration collected by the electronic device 100 in a pull-down process of the arm of the user.

In FIG. 14B, component acceleration data that is in the direction of the Y-axis and that is collected by the electronic device 100 is acceleration data Y2. A direction of the acceleration data Y2 is downward and perpendicular to the horizontal plane. It may be understood that the acceleration data Y2 is sum acceleration data in the three directions of the X-axis, the Y-axis, and the Z-axis.

In the following embodiments of this application, a value of the sum acceleration data gradually increases from zero to a maximum value in the push-up process of the arm of the user, and the value of the sum acceleration data gradually decreases from the maximum value to close to 0 in the process of pushing up the arm of the user to a highest point. After the user pushes the arm up to the highest point, the barbell is held at the highest point for a period of time, during which the value of the acceleration is close to 0. Then, when the arm of the user is pulled down from the highest point, the value of the sum acceleration data gradually decreases from around 0 to a minimum value. In a process of pulling down the arm of the user to a lowest point, the value of the sum acceleration data gradually increases from the minimum value to around 0. The sum acceleration data here is in a vector unit. That is, the sum acceleration data represents not only the magnitude of the acceleration, but also the direction of the acceleration. It can be learned from the foregoing analysis that when the sum acceleration data is greater than 0, it indicates that the arm of the user is performing a push-up action. When the sum acceleration data is less than 0, it indicates that the arm of the user is performing a pull-down action. When the sum acceleration data is kept around 0 for a period of time (for example, 2 seconds), it indicates that the arm of the user is performing an action of keeping the barbell at the highest point. In this way, the electronic device 100 may determine, based on the magnitude of the sum acceleration data, whether the arm action of the user is the first action, the second action, or the third action.

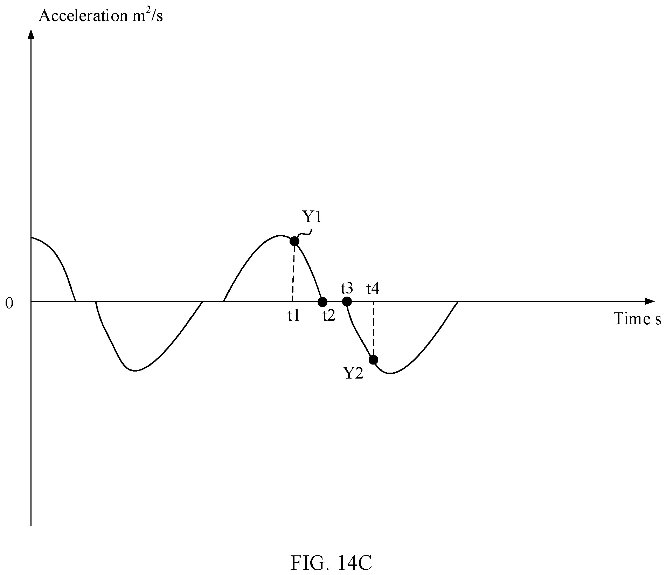

FIG. 14C shows an example of a schematic diagram of the sum acceleration data collected by the electronic device 100 in the weight lifting process of the user.

In FIG. 14C, a horizontal axis represents time, and a vertical axis represents the magnitude of the sum acceleration data. When the sum acceleration data is greater than 0, it indicates that the arm of the user is performing a push-up action. When the sum acceleration data is less than 0, it indicates that the arm of the user is performing a pull-down action. When the sum acceleration data is kept around 0 for a period of time (for example, 2 seconds), it indicates that the arm of the user is performing an action of keeping the barbell at the highest point. In the weight lifting process, the arm of the user is periodically pushed up and pulled down. Therefore, the magnitude of the sum acceleration data collected by the electronic device 100 also periodically changes, and the magnitude of the sum acceleration data collected by the electronic device 100 periodically changes between a positive value and a negative value. For example, the acceleration data Y1 shown in FIG. 14A may be acceleration data Y1 shown at a moment t1 in FIG. 14C. The sum acceleration data Y2 shown in FIG. 14B may be acceleration data Y2 shown at a moment t4 in FIG. 14C. In FIG. 14C, the acceleration gradually decreases from a positive value to around 0 (an acceleration value corresponding to a moment t2), a value of the acceleration remains 0 from the moment t2 to a moment t3, and a difference between the moment t2 and the moment t3 is greater than a preset value. It indicates that an action type between the moment t2 and the moment t3 is that the arm of the user is performing an action of keeping the barbell at the highest point.

In another possible implementation, the electronic device 100 may obtain a motion track map of the arm of the user based on the acceleration data, compare the motion track map of the arm of the user with a template, and determine whether the arm actions of the user are the first action, the second action, and the third action. Alternatively, the electronic device 100 may determine, in another manner, whether the arm actions of the user are the first action, the second action, and the third action. This is not limited in this embodiment of this application.

### II. In a rowing process, the electronic device 100 determines specific implementations of the first action and the second action of the user based on the motion data collected by the motion sensor.

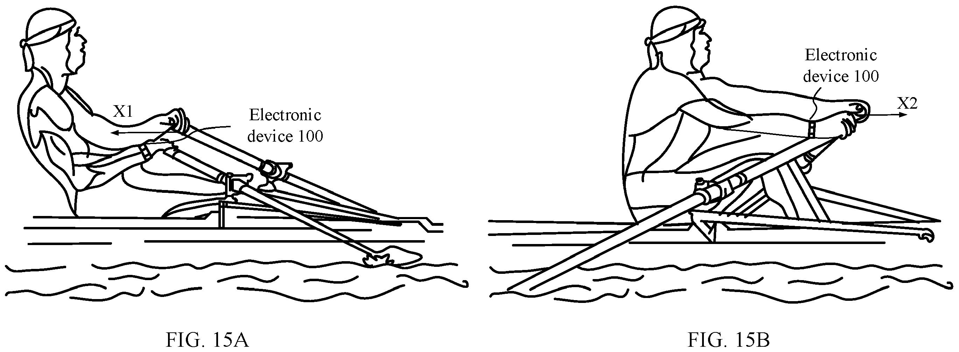

FIG. 15A to FIG. 15C show examples of schematic diagrams in which the electronic device 100 determines, based on acceleration data, whether a wrist motion posture of the user is the first action or the second action.

In a possible implementation, the electronic device 100 may determine, based on acceleration data collected by an acceleration sensor, whether the user is performing the first action or the second action.

The acceleration sensor in the electronic device 100 may collect acceleration data of a wrist part of the user, and determine an arm action of the user based on the acceleration data of the wrist part of the user. The electronic device 100 collects component acceleration data in three directions of an X-axis, a Y-axis, and a Z-axis, and the electronic device 100 obtains sum acceleration data according to the component acceleration data in the three directions of the X-axis, the Y-axis, and the Z-axis, and determines, based on a magnitude of a sum acceleration, whether the user is performing the first action or the second action.

As can be learned from the foregoing, the first action is an action of moving backward and retracting the arm, and the second action is an action of moving forward and extending the arm.

In the rowing process, because the wrist part of the user moves back and forth, in an ideal case, it may be understood that component acceleration data collected by the electronic device 100 in three directions of a Y-axis and a Z-axis is 0. In this case, the electronic device 100 collects only component acceleration data in a direction of an X-axis.

FIG. 15A shows an example of a schematic diagram of a magnitude and a direction of a sum acceleration collected by the electronic device 100 in a process in which the user holds a grip rod of an oar and pulls the grip rod backward.

In FIG. 15A, component acceleration data that is in the direction of the X-axis and that is collected by the electronic device 100 is acceleration data X1. A direction of the acceleration data X1 is leftward and parallel to a horizontal plane. It may be understood that the acceleration data X1 is sum acceleration data in the three directions of the X-axis, the Y-axis, and the Z-axis.

FIG. 15B shows an example of a schematic diagram of a magnitude and a direction of a sum acceleration collected by the electronic device 100 in a process in which the user holds the grip rod of the oar and pushes the grip rod forward.

In FIG. 15B, component acceleration data that is in the direction of the X-axis and that is collected by the electronic device 100 is acceleration data X2. A direction of the acceleration data X2 is rightward and parallel to the horizontal plane. It may be understood that the acceleration data X2 is sum acceleration data in the three directions of the X-axis, the Y-axis, and the Z-axis.

In the following embodiments of this application, in the process in which the user holds the grip rod of the oar and pulls the grip rod backward, the value of the sum acceleration data gradually increases from zero to a maximum value, and then the value of the sum acceleration data gradually decreases from the maximum value to a positive local minimum (for example, 0.3). In the process in which the user holds the grip rod of the oar and pushes the grip rod forward, the value of the sum acceleration data gradually decreases from the positive local minimum (for example, 0.3) to a minimum value, and then the value of the sum acceleration data gradually increases from the minimum value to a negative local maximum (for example, -0.2). The sum acceleration data here is in a vector unit. That is, the sum acceleration data represents not only the magnitude of the acceleration, but also the direction of the acceleration. It can be learned from the foregoing analysis that when the sum acceleration data is greater than 0, it indicates that the user is performing an action of pulling the grip rod backward. When the sum acceleration data is less than 0, it indicates that the user is performing an action of pushing the grip rod forward. In this way, the electronic device 100 may determine whether the arm action of the user is the first action or the second action based on that the sum acceleration data is positive or negative.

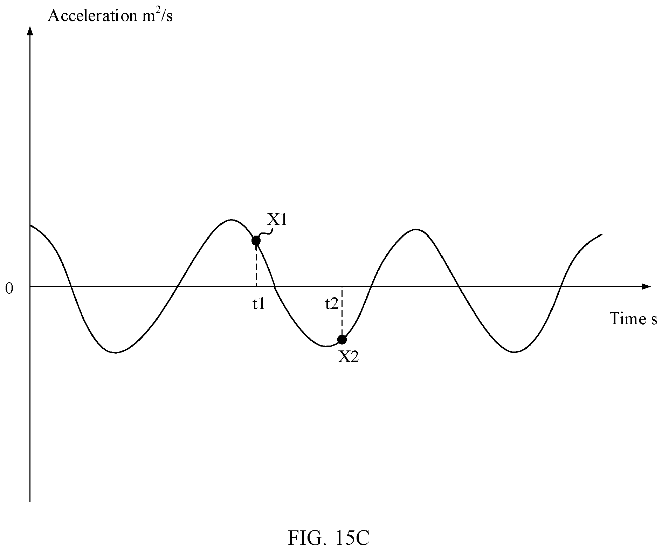

FIG. 15C shows an example of a schematic diagram of the sum acceleration data collected by the electronic device 100 in the rowing process of the user.

In FIG. 15C, a horizontal axis represents time, and a vertical axis represents the magnitude of the sum acceleration data. In FIG. 15C, when the sum acceleration data is greater than 0, it indicates that the user is performing an action of pulling the grip rod backward. When the sum acceleration data is less than 0, it indicates that the user is performing an action of pushing the grip rod forward. In the rowing process, the arm of the user periodically pulls the grip rod backward and pushes the grip rod forward. Therefore, the magnitude of the sum acceleration data collected by the electronic device 100 also periodically changes, and the magnitude of the sum acceleration data collected by the electronic device 100 periodically changes between a positive value and a negative value. For example, the acceleration data X1 shown in FIG. 15A may be acceleration data X1 shown at a moment t1 in FIG. 15C. The sum acceleration data X2 shown in FIG. 15B may be acceleration data X2 shown at a moment t2 in FIG. 15C.

In another possible implementation, the electronic device 100 may obtain a motion track map of the arm of the user based on the acceleration data, compare the motion track map of the arm of the user with a template, and determine whether the arm action of the user is the first action or the second action. Alternatively, the electronic device 100 may determine, in another manner, whether the arm action of the user is the first action or the second action. This is not limited in this embodiment of this application.

### III. In a process of performing exercise by using a rowing machine, the electronic device 100 determines specific implementations of the first action and the second action of the user based on the motion data collected by the motion sensor.

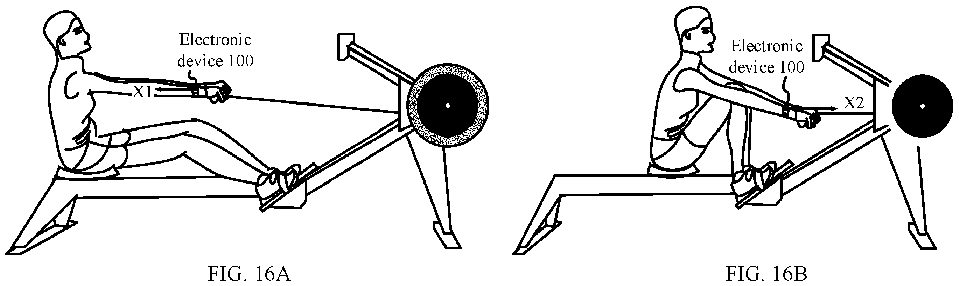

FIG. 16A to FIG. 16C show examples of schematic diagrams in which the electronic device 100 determines, based on acceleration data, whether a wrist motion posture of the user is the first action or the second action.

In a possible implementation, the electronic device 100 may determine, based on acceleration data collected by an acceleration sensor, whether the user is performing the first action or the second action.

The acceleration sensor in the electronic device 100 may collect acceleration data of a wrist part of the user, and determine an arm action of the user based on the acceleration data of the wrist part of the user. The electronic device 100 collects component acceleration data in three directions of an X-axis, a Y-axis, and a Z-axis, and the electronic device 100 obtains sum acceleration data according to the component acceleration data in the three directions of the X-axis, the Y-axis, and the Z-axis, and determines, based on a magnitude of a sum acceleration, whether the user is performing the first action or the second action.

As can be learned from the foregoing, the first action is an arm action away from the rowing machine, and the second action is an arm action close to the rowing machine.

In rowing machine exercise, because the wrist part of the user moves back and forth, in an ideal case, it may be understood that component acceleration data collected by the electronic device 100 in three directions of a Y-axis and a Z-axis is 0. In this case, the electronic device 100 collects only component acceleration data in a direction of an X-axis.

FIG. 16A shows an example of a schematic diagram of a magnitude and a direction of a sum acceleration collected by the electronic device 100 in a process in which the user pulls a handle of the rowing machine to move backward and away from the rowing machine.

In FIG. 16A, component acceleration data that is in the direction of the X-axis and that is collected by the electronic device 100 is acceleration data X1. A direction of the acceleration data X1 is leftward and parallel to a horizontal plane. It may be understood that the acceleration data X1 is sum acceleration data in the three directions of the X-axis, the Y-axis, and the Z-axis.

FIG. 16B shows an example of a schematic diagram of a magnitude and a direction of a sum acceleration collected by the electronic device 100 in a process in which the user pulls a handle of the rowing machine to move forward and close to the rowing machine.

In FIG. 16B, component acceleration data that is in the direction of the X-axis and that is collected by the electronic device 100 is acceleration data X2. A direction of the acceleration data X2 is rightward and parallel to the horizontal plane. It may be understood that the acceleration data X2 is sum acceleration data in the three directions of the X-axis, the Y-axis, and the Z-axis.

In the following embodiments of this application, in a process in which the user moves the handle of the rowing machine, so that a distance between the handle of the rowing machine and the rowing machine reaches a maximum distance, a value of the sum acceleration data gradually increases from zero to a maximum value, and then the value of the sum acceleration data gradually decreases from the maximum value to around 0. In the process in which the user moves the handle of the rowing machine so that the handle of the rowing machine is close to the rowing machine, the value of the sum acceleration data gradually decreases from around 0 to a minimum value, and then the value of the sum acceleration data gradually increases from the minimum value to around 0. The sum acceleration data here is in a vector unit. That is, the sum acceleration data represents not only the magnitude of the acceleration, but also the direction of the acceleration. It can be learned from the foregoing analysis that when the sum acceleration data is greater than 0, it indicates that the user is performing an arm action of moving backward and away from the rowing machine. When the sum acceleration data is less than 0, it indicates that the user is performing an arm action of moving forward and close to the rowing machine. In this way, the electronic device 100 may determine whether the arm action of the user is the first action or the second action based on that the sum acceleration data is positive or negative.

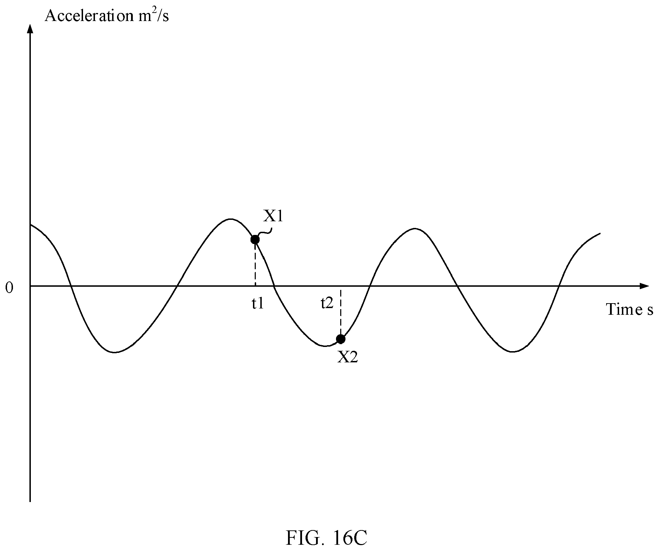

FIG. 16C shows an example of a schematic diagram of sum acceleration data collected by the electronic device 100 in a process in which the user performs exercise by using a rowing machine.

In FIG. 16C, a horizontal axis represents time, and a vertical axis represents the magnitude of the sum acceleration data. In FIG. 16C, when the sum acceleration data is greater than 0, it indicates that the user is performing an arm action away from the rowing machine, and when the sum acceleration data is less than 0, it indicates that the user is performing an arm action close to the rowing machine. In the process of using the rowing machine, the arm of the user periodically moves backward and away from the rowing machine and moves forward and close to the rowing machine. Therefore, the magnitude of the sum acceleration data collected by the electronic device 100 also periodically changes, and the magnitude of the sum acceleration data collected by the electronic device 100 periodically changes between a positive value and a negative value. For example, the acceleration data X1 shown in FIG. 16A may be acceleration data X1 shown at a moment t1 in FIG. 16C. The sum acceleration data X2 shown in FIG. 16B may be acceleration data X2 shown at a moment t2 in FIG. 16C.

In another possible implementation, the electronic device 100 may obtain a motion track map of the arm of the user based on the acceleration data, compare the motion track map of the arm of the user with a template, and determine whether the arm action of the user is the first action or the second action. Alternatively, the electronic device 100 may determine, in another manner, whether the arm action of the user is the first action or the second action. This is not limited in this embodiment of this application.

### IV. In a process of performing exercise by using an elliptical machine, the electronic device 100 determines specific implementations of the first action and the second action of the user based on the motion data collected by the motion sensor.

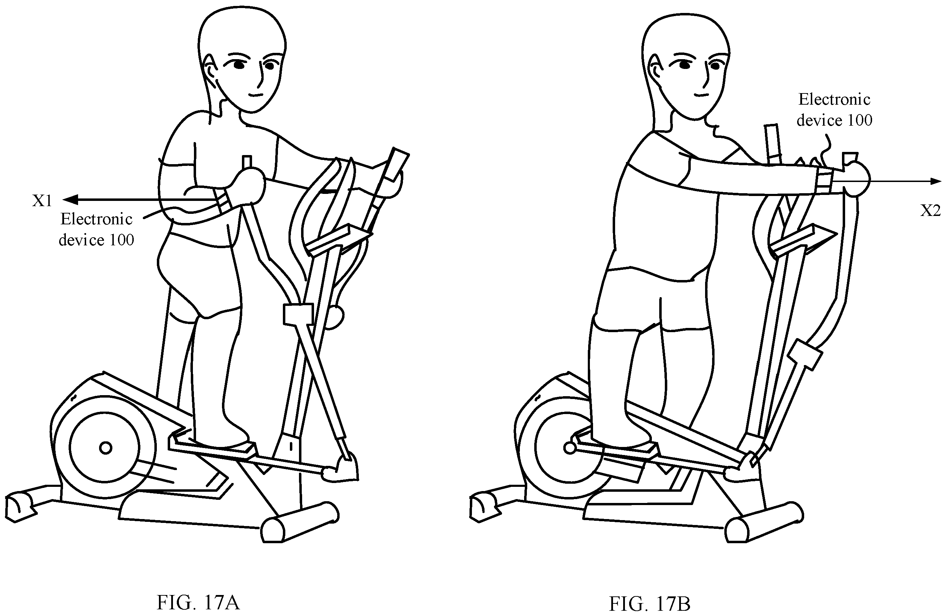

FIG. 17A to FIG. 17C show examples of schematic diagrams in which the electronic device 100 determines, based on acceleration data, whether a wrist motion posture of the user is the first action or the second action.

In a possible implementation, the electronic device 100 may determine, based on acceleration data collected by an acceleration sensor, whether the user is performing the first action or the second action.

The acceleration sensor in the electronic device 100 may collect acceleration data of a wrist part of the user, and determine an arm action of the user based on the acceleration data of the wrist part of the user. The electronic device 100 collects component acceleration data in three directions of an X-axis, a Y-axis, and a Z-axis, and the electronic device 100 obtains sum acceleration data according to the component acceleration data in the three directions of the X-axis, the Y-axis, and the Z-axis, and determines, based on a magnitude of a sum acceleration, whether the user is performing the first action or the second action.

As can be learned from the foregoing, the first action is an arm action of pulling backward an armrest of the elliptical machine, and the second action is an arm action of pushing forward the armrest of the elliptical machine.

In elliptical machine exercise, because the wrist part of the user moves back and forth, in an ideal case, it may be understood that component acceleration data collected by the electronic device 100 in three directions of a Y-axis and a Z-axis is 0. In this case, the electronic device 100 collects only component acceleration data in a direction of an X-axis.

FIG. 17A shows an example of a schematic diagram of a magnitude and a direction of a sum acceleration collected by the electronic device 100 in a process in which the user pulls the armrest of the elliptical machine to move backward and away from the elliptical machine.

In FIG. 17A, component acceleration data that is in the direction of the X-axis and that is collected by the electronic device 100 is acceleration data X1. A direction of the acceleration data X1 is leftward and parallel to a horizontal plane. It may be understood that the acceleration data X1 is sum acceleration data in the three directions of the X-axis, the Y-axis, and the Z-axis.

FIG. 17B shows an example of a schematic diagram of a magnitude and a direction of a sum acceleration collected by the electronic device 100 in a process in which the user pushes the armrest of the elliptical machine to move forward and close to the elliptical machine.

In FIG. 17B, component acceleration data that is in the direction of the X-axis and that is collected by the electronic device 100 is acceleration data X2. A direction of the acceleration data X2 is rightward and parallel to the horizontal plane. It may be understood that the acceleration data X2 is sum acceleration data in the three directions of the X-axis, the Y-axis, and the Z-axis.

In the following embodiments of this application, in a process in which the user moves the armrest of the elliptical machine, so that a distance between the armrest of the elliptical machine and the elliptical machine reaches a maximum distance, a value of the sum acceleration data gradually increases from zero to a maximum value, and then the value of the sum acceleration data gradually decreases from the maximum value to around 0. In the process in which the user moves the armrest of the elliptical machine so that the armrest of the elliptical machine is close to the elliptical machine, the value of the sum acceleration data gradually decreases from around 0 to a minimum value, and then the value of the sum acceleration data gradually increases from the minimum value to around 0. The sum acceleration data here is in a vector unit. That is, the sum acceleration data represents not only the magnitude of the acceleration, but also the direction of the acceleration. It can be learned from the foregoing analysis that when the sum acceleration data is greater than 0, it indicates that the user is performing an arm action of pulling the armrest of the elliptical machine backward. When the sum acceleration data is less than 0, it indicates that the user is performing an arm action of pushing the armrest of the elliptical machine forward. In this way, the electronic device 100 may determine whether the arm action of the user is the first action or the second action based on that the sum acceleration data is positive or negative.

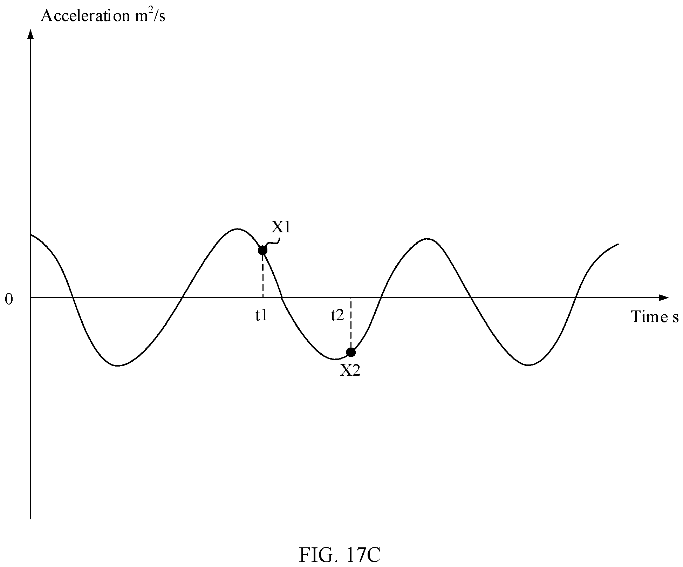

FIG. 17C shows an example of a schematic diagram of sum acceleration data collected by the electronic device 100 in a process in which the user performs exercise by using an elliptical machine.

In FIG. 17C, a horizontal axis represents time, and a vertical axis represents the magnitude of the sum acceleration data. In FIG. 17C, when the sum acceleration data is greater than 0, it indicates that the user is performing an arm action of pulling the armrest of the elliptical machine backward, and when the sum acceleration data is less than 0, it indicates that the user is performing an arm action of pushing the armrest of the elliptical machine forward. In the process of using the elliptical machine, the arm of the user periodically moves backward and away from the elliptical machine and moves forward and close to the elliptical machine. Therefore, the magnitude of the sum acceleration data collected by the electronic device 100 also periodically changes, and the magnitude of the sum acceleration data collected by the electronic device 100 periodically changes between a positive value and a negative value. For example, the acceleration data X1 shown in FIG. 17A may be acceleration data X1 shown at a moment t1 in FIG. 17C. The sum acceleration data X2 shown in FIG. 17B may be acceleration data X2 shown at a moment t2 in FIG. 17C.

In another possible implementation, the electronic device 100 may obtain a motion track map of the arm of the user based on the acceleration data, compare the motion track map of the arm of the user with a template, and determine whether the arm action of the user is the first action or the second action. Alternatively, the electronic device 100 may determine, in another manner, whether the arm action of the user is the first action or the second action. This is not limited in this embodiment of this application.

After the electronic device 100 enables the breathing guidance function, the electronic device 100 needs to monitor arm actions (the first action and the second action) of the user in the foregoing several types of exercise combined with fitness equipment, and provide a specific breathing guidance solution with reference to the arm actions of the user.

When the electronic device 100 guides, based on the arm action of the user in exercise combined with fitness equipment, the user to perform a breathing action, after consecutively detecting the first action for n times, the electronic device 100 may prompt, in the first prompt manner, the user to perform an inhalation action once. After consecutively detecting the second action for m times, the electronic device 100 prompts, in the second prompt manner, the user to perform an exhalation action.

In this way, the electronic device 100 may use different prompt manners, so that the arm action of the user in exercise combined with fitness equipment matches a breathing rhythm, thereby saving physical strength of the user and improving a fitness effect of the user.

Next, breathing guidance solutions in weight lifting exercise, rowing, rowing machine exercise, and elliptical machine exercise are introduced.

### I. Breathing guidance solution in weight lifting exercise

FIG. 14A to FIG. 14C describe a case in which the electronic device 100 determines, based on the motion sensor data collected by the electronic device 100, that arm motion postures of the user are the first action, the second action, and the third action. Specifically, the electronic device 100 collects the motion sensor data, and determines, based on the motion sensor data, whether the action type of the user is the first action, the second action, or the third action. After determining the first action, the electronic device 100 prompts, in the first prompt manner, the user to perform an inhalation action. After determining the second action, the electronic device 100 prompts, in the second prompt manner, the user to perform an exhalation action. After determining the third action, the electronic device 100 prompts, in the third prompt manner, the user to hold breath, or prompts, in the third prompt manner, the user to continuously perform at least one short exhalation action and at least one short inhalation action.

Optionally, the barbell may replace a function of the electronic device 100. That is, the barbell collects the motion sensor data, determines, based on the motion sensor data, whether the action type of the user is the first action, the second action, or the third action, and guides the breathing rhythm of the user. Specifically, the barbell collects the motion sensor data, and determines, based on the motion sensor data, whether the action type of the user is the first action, the second action, or the third action. After determining the first action, the barbell prompts, in the first prompt manner, the user to perform an inhalation action. After determining the second action, the barbell prompts, in the second prompt manner, the user to perform an exhalation action. After determining the third action, the barbell prompts, in the third prompt manner, the user to hold breath, or prompts, in the third prompt manner, the user to continuously perform at least one short exhalation action and at least one short inhalation action.

Optionally, before the electronic device 100 starts to guide the breathing rhythm of the user, the electronic device 100 needs to establish a communication connection to the barbell. The barbell collects the motion sensor data, and sends the motion sensor data to the electronic device 100. After receiving the motion sensor data, the electronic device 100 determines, based on the motion sensor data, whether the action type of the user is the first action, the second action, or the third action, and guides the breathing rhythm of the user. After determining the first action, the electronic device 100 prompts, in the first prompt manner, the user to perform an inhalation action. After determining the second action, the electronic device 100 prompts, in the second prompt manner, the user to perform an exhalation action. After determining the third action, the electronic device 100 prompts, in the third prompt manner, the user to hold breath, or prompts, in the third prompt manner, the user to continuously perform at least one short exhalation action and at least one short inhalation action.

Optionally, before the electronic device 100 starts to guide the breathing rhythm of the user, the electronic device 100 needs to establish a communication connection to the barbell. The barbell collects the motion sensor data, and determines whether the action of the user is the first action, the second action, or the third action based on the motion sensor data. After determining that the action of the user is the first action, the barbell sends an instruction 1 to the electronic device 100. After receiving the instruction 1, the electronic device 100 prompts, in the first prompt manner, the user to inhale. After determining that the action of the user is the second action, the barbell sends an instruction 2 to the electronic device 100. After receiving the instruction 2, the electronic device 100 prompts, in the second prompt manner, the user to perform an exhalation action. After determining that the action of the user is the third action, the barbell sends an instruction 3 to the electronic device 100. After receiving the instruction 3, the electronic device 100 prompts, in the third prompt manner, the user to hold breath, or prompts, in the third prompt manner, the user to continuously perform at least one short exhalation action and at least one short inhalation action.

The foregoing communication connection may be a wired connection or a wireless connection. The wireless connection may be a close-range connection such as a wireless local area network (wireless local area network, WLAN) connection, a wireless fidelity (wireless fidelity, Wi-Fi) connection, a Bluetooth connection, an infrared connection, a near field communication (near field communication, NFC) connection, ZigBee, or another wireless communication technology that appears in subsequent development. Alternatively, the barbell may establish a long-distance connection to the electronic device 100. The long-distance connection includes but is not limited to a long-distance connection based on a mobile network of 2G, 3G, 4G, 5G, and a subsequent standard protocol. Alternatively, the barbell and the electronic device 100 may log in to a same user account (for example, a Huawei account), and then establish a remote connection by using a server.

That the barbell prompts, in the first prompt manner, the user to perform an inhalation action is similar to that the electronic device 100 prompts, in the first prompt manner, the user to perform an inhalation action. Details are not described herein again in this embodiment of this application.

That the barbell prompts, in the second prompt manner, the user to perform an inhalation action is similar to that the electronic device 100 prompts, in the second prompt manner, the user to perform an exhalation action. Details are not described herein again in this embodiment of this application.

The type of the third prompt manner may be any one of vibration, voice, text, and picture, or may be a combination of two or more of vibration, voice, text, and picture.

### II. Breathing guidance solution in rowing exercise

FIG. 15A to FIG. 15C describe a case in which the electronic device 100 determines, based on the motion sensor data collected by the electronic device 100, that the arm motion posture of the user is the first action or the second action. Specifically, the electronic device 100 collects the motion sensor data, and determines, based on the motion sensor data, whether the action type of the user is the first action or the second action. After determining the first action, the electronic device 100 prompts, in the first prompt manner, the user to perform an inhalation action. After determining the second action, the electronic device 100 prompts, in the second prompt manner, the user to perform an exhalation action.

Optionally, a hull (or an oar on the hull) may replace a function of the electronic device 100. That is, the hull collects the motion sensor data, determines, based on the motion sensor data, whether the action type of the user is the first action or the second action, and guides the breathing rhythm of the user. Specifically, the hull collects the motion sensor data, and determines, based on the motion sensor data, whether the action type of the user is the first action, the second action, or the third action. After determining the first action, the hull prompts, in the first prompt manner, the user to perform an inhalation action. After determining the second action, the hull prompts, in the second prompt manner, the user to perform an exhalation action.

Optionally, before the electronic device 100 starts to guide the breathing rhythm of the user, the electronic device 100 needs to establish a communication connection to the hull (or the oar on the hull). The hull collects the motion sensor data, and sends the motion sensor data to the electronic device 100. After receiving the motion sensor data, the electronic device 100 determines, based on the motion sensor data, whether the action type of the user is the first action or the second action, and guides the breathing rhythm of the user. After determining the first action, the electronic device 100 prompts, in the first prompt manner, the user to perform an inhalation action. After determining the second action, the electronic device 100 prompts, in the second prompt manner, the user to perform an exhalation action.

Optionally, before the electronic device 100 starts to guide the breathing rhythm of the user, the electronic device 100 needs to establish a communication connection to the hull (or the oar on the hull). The hull collects the motion sensor data, and determines whether the action of the user is the first action or the second action based on the motion sensor data. After determining that the action of the user is the first action, the hull sends an instruction 1 to the electronic device 100. After receiving the instruction 1, the electronic device 100 prompts, in the first prompt manner, the user to inhale. After determining that the action of the user is the second action, the hull sends an instruction 2 to the electronic device 100. After receiving the instruction 2, the electronic device 100 prompts, in the second prompt manner, the user to perform an exhalation action.

The foregoing communication connection may be a wired connection or a wireless connection. The wireless connection may be a close-range connection such as a wireless local area network (wireless local area network, WLAN) connection, a wireless fidelity (wireless fidelity, Wi-Fi) connection, a Bluetooth connection, an infrared connection, a near field communication (near field communication, NFC) connection, ZigBee, or another wireless communication technology that appears in subsequent development. Alternatively, the hull (or the oar on the hull) may establish a long-distance connection to the electronic device 100. The long-distance connection includes but is not limited to a long-distance connection based on a mobile network of 2G, 3G, 4G, 5G, and a subsequent standard protocol. Alternatively, the hull (or the oar on the hull) and the electronic device 100 may log in to a same user account (for example, a Huawei account), and then establish a remote connection by using a server.

That the hull (or the oar on the hull) prompts, in the first prompt manner, the user to perform an inhalation action is similar to that the electronic device 100 prompts, in the first prompt manner, the user to perform an inhalation action. Details are not described herein again in this embodiment of this application.

That the hull (or the oar on the hull) prompts, in the second prompt manner, the user to perform an inhalation action is similar to that the electronic device 100 prompts, in the second prompt manner, the user to perform an exhalation action. Details are not described herein again in this embodiment of this application.

### III. Breathing guidance solution for the user in exercise using a rowing machine

FIG. 16A to FIG. 16C describe a case in which the electronic device 100 determines, based on the motion sensor data collected by the electronic device 100, that the arm motion posture of the user is the first action or the second action. Specifically, the electronic device 100 collects the motion sensor data, and determines, based on the motion sensor data, whether the action type of the user is the first action or the second action. After determining the first action, the electronic device 100 prompts, in the first prompt manner, the user to perform an inhalation action. After determining the second action, the electronic device 100 prompts, in the second prompt manner, the user to perform an exhalation action.

Optionally, the rowing machine may replace a function of the electronic device 100. That is, the rowing machine collects the motion sensor data, determines, based on the motion sensor data, whether the action type of the user is the first action or the second action, and guides the breathing rhythm of the user. Specifically, the rowing machine collects the motion sensor data, and determines, based on the motion sensor data, whether the action type of the user is the first action, the second action, or the third action. After determining the first action, the rowing machine prompts, in the first prompt manner, the user to perform an inhalation action. After determining the second action, the rowing machine prompts, in the second prompt manner, the user to perform an exhalation action.

Optionally, before the electronic device 100 starts to guide the breathing rhythm of the user, the electronic device 100 needs to establish a communication connection to the rowing machine. The rowing machine collects the motion sensor data, and sends the motion sensor data to the electronic device 100. After receiving the motion sensor data, the electronic device 100 determines, based on the motion sensor data, whether the action type of the user is the first action or the second action, and guides the breathing rhythm of the user. After determining the first action, the electronic device 100 prompts, in the first prompt manner, the user to perform an inhalation action. After determining the second action, the electronic device 100 prompts, in the second prompt manner, the user to perform an exhalation action.

Optionally, before the electronic device 100 starts to guide the breathing rhythm of the user, the electronic device 100 needs to establish a communication connection to the rowing machine. The rowing machine collects the motion sensor data, and determines whether the action of the user is the first action or the second action based on the motion sensor data. After determining that the action of the user is the first action, the rowing machine sends an instruction 1 to the electronic device 100. After receiving the instruction 1, the electronic device 100 prompts, in the first prompt manner, the user to inhale. After determining that the action of the user is the second action, the rowing machine sends an instruction 2 to the electronic device 100. After receiving the instruction 2, the electronic device 100 prompts, in the second prompt manner, the user to perform an exhalation action.

In some embodiments, the rowing machine may have a display screen, and content such as a text, a picture, or a video may be displayed on the display screen to prompt the user to perform an inhalation action or an exhalation action. Alternatively, content such as a text, a picture, or a video may be displayed on the display screen to prompt the user to perform the first action or the second action.

That the rowing machine prompts, in the first prompt manner, the user to perform an inhalation action is similar to that the electronic device 100 prompts, in the first prompt manner, the user to perform an inhalation action. Details are not described herein again in this embodiment of this application.

That the rowing machine prompts, in the second prompt manner, the user to perform an inhalation action is similar to that the electronic device 100 prompts, in the second prompt manner, the user to perform an exhalation action. Details are not described herein again in this embodiment of this application.

The foregoing communication connection may be a wired connection or a wireless connection. The wireless connection may be a close-range connection such as a wireless local area network (wireless local area network, WLAN) connection, a wireless fidelity (wireless fidelity, Wi-Fi) connection, a Bluetooth connection, an infrared connection, a near field communication (near field communication, NFC) connection, ZigBee, or another wireless communication technology that appears in subsequent development. Alternatively, the rowing machine may establish a long-distance connection to the electronic device 100. The long-distance connection includes but is not limited to a long-distance connection based on a mobile network of 2G, 3G, 4G, 5G, and a subsequent standard protocol. Alternatively, the rowing machine and the electronic device 100 may log in to a same user account (for example, a Huawei account), and then establish a remote connection by using a server.

### IV Breathing guidance solution for the user in exercise using an elliptical machine

FIG. 17A to FIG. 17C describe a case in which the electronic device 100 determines, based on the motion sensor data collected by the electronic device 100, that the arm motion posture of the user is the first action or the second action. Specifically, the electronic device 100 collects the motion sensor data, and determines, based on the motion sensor data, whether the action type of the user is the first action or the second action. After determining the first action, the electronic device 100 prompts, in the first prompt manner, the user to perform an inhalation action. After determining the second action, the electronic device 100 prompts, in the second prompt manner, the user to perform an exhalation action.

Optionally, the elliptical machine may replace a function of the electronic device 100. That is, the elliptical machine collects the motion sensor data, determines, based on the motion sensor data, whether the action type of the user is the first action or the second action, and guides the breathing rhythm of the user. Specifically, the elliptical machine collects the motion sensor data, and determines, based on the motion sensor data, whether the action type of the user is the first action, the second action, or the third action. After determining the first action, the elliptical machine prompts, in the first prompt manner, the user to perform an inhalation action. After determining the second action, the elliptical machine prompts, in the second prompt manner, the user to perform an exhalation action.

Optionally, before the electronic device 100 starts to guide the breathing rhythm of the user, the electronic device 100 needs to establish a communication connection to the elliptical machine. The elliptical machine collects the motion sensor data, and sends the motion sensor data to the electronic device 100. After receiving the motion sensor data, the electronic device 100 determines, based on the motion sensor data, whether the action type of the user is the first action or the second action, and guides the breathing rhythm of the user. After determining the first action, the electronic device 100 prompts, in the first prompt manner, the user to perform an inhalation action. After determining the second action, the electronic device 100 prompts, in the second prompt manner, the user to perform an exhalation action.

Optionally, before the electronic device 100 starts to guide the breathing rhythm of the user, the electronic device 100 needs to establish a communication connection to the elliptical machine. The elliptical machine collects the motion sensor data, and determines whether the action of the user is the first action or the second action based on the motion sensor data. After determining that the action of the user is the first action, the elliptical machine sends an instruction 1 to the electronic device 100. After receiving the instruction 1, the electronic device 100 prompts, in the first prompt manner, the user to inhale. After determining that the action of the user is the second action, the elliptical machine sends an instruction 2 to the electronic device 100. After receiving the instruction 2, the electronic device 100 prompts, in the second prompt manner, the user to perform an exhalation action.

In some embodiments, the elliptical machine may have a display screen, and content such as a text, a picture, or a video may be displayed on the display screen to prompt the user to perform an inhalation action or an exhalation action. Alternatively, content such as a text, a picture, or a video may be displayed on the display screen to prompt the user to perform the first action or the second action.

That the elliptical machine prompts, in the first prompt manner, the user to perform an inhalation action is similar to that the electronic device 100 prompts, in the first prompt manner, the user to perform an inhalation action. Details are not described herein again in this embodiment of this application.

That the elliptical machine prompts, in the second prompt manner, the user to perform an inhalation action is similar to that the electronic device 100 prompts, in the second prompt manner, the user to perform an exhalation action. Details are not described herein again in this embodiment of this application.

The foregoing communication connection may be a wired connection or a wireless connection. The wireless connection may be a close-range connection such as a wireless local area network (wireless local area network, WLAN) connection, a wireless fidelity (wireless fidelity, Wi-Fi) connection, a Bluetooth connection, an infrared connection, a near field communication (near field communication, NFC) connection, ZigBee, or another wireless communication technology that appears in subsequent development. Alternatively, the elliptical machine may establish a long-distance connection to the electronic device 100. The long-distance connection includes but is not limited to a long-distance connection based on a mobile network of 2G, 3G, 4G, 5G, and a subsequent standard protocol. Alternatively, the elliptical machine and the electronic device 100 may log in to a same user account (for example, a Huawei account), and then establish a remote connection by using a server.

### Embodiment 2

Embodiment 2 is a breathing guidance solution for a field of health.

In a medical treatment and health examination process, when a user needs to cooperate with a doctor in examination and treatment with reference to a breathing rhythm, and an electronic device 100 detects that another electronic device (for example, medical equipment) performs a first action, the electronic device 100 prompts, in a first prompt manner, the user to inhale. When the electronic device 100 detects that the medical equipment performs a second action, the electronic device 100 prompts, in a second prompt manner, the user to exhale. In this way, the breathing rhythm of the user may cooperate with an examination action of the medical equipment, so that user experience in the examination process can be improved, discomfort can be reduced, and health examination efficiency can also be improved.

Specifically, in a possible implementation, the electronic device 100 needs to establish a communication connection to the medical equipment. The medical equipment collects motion sensor data, and the medical equipment sends the motion sensor data to the electronic device 100 in real time. After obtaining the motion sensor data, the electronic device 100 determines, based on the motion sensor data, whether the action of the medical equipment is the first action or the second action. After determining the action type of the medical equipment, the electronic device 100 prompts, in the first prompt manner, the user to inhale and prompts, in the second prompt manner, the user to exhale.

In another possible implementation, the electronic device 100 needs to establish a communication connection to the medical equipment. The medical equipment collects the motion sensor data, and determines whether the action type of the medical equipment is the first action or the second action based on the motion sensor data. After determining that the action of the user is the first action, the medical equipment sends an instruction 1 to the electronic device 100. After receiving the instruction 1, the electronic device 100 prompts, in the first prompt manner, the user to inhale. After determining that the action of the user is the second action, the medical equipment sends an instruction 2 to the electronic device 100. After receiving the instruction 2, the electronic device 100 prompts, in the second prompt manner, the user to inhale.

Optionally, the medical equipment in the following embodiments of this application may replace a function of the electronic device 100. That is, the medical equipment collects the motion sensor data, and determines, based on the motion sensor data, whether a working type of the medical equipment is the first action or the second action. After determining the action type, the medical equipment prompts, in the first prompt manner, the user to inhale and prompts, in the second prompt manner, the user to exhale.

The communication connection between the medical equipment and the electronic device 100 may be a wired connection or a wireless connection. The wireless connection may be a close-range connection such as a wireless local area network (wireless local area network, WLAN) connection, a wireless fidelity (wireless fidelity, Wi-Fi) connection, a Bluetooth connection, an infrared connection, a near field communication (near field communication, NFC) connection, ZigBee, or another wireless communication technology that appears in subsequent development. Alternatively, the medical equipment may establish a long-distance connection to the electronic device 100. The long-distance connection includes but is not limited to a long-distance connection based on a mobile network of 2G, 3G, 4G, 5G, and a subsequent standard protocol. Alternatively, the medical equipment and the electronic device 100 may log in to a same user account (for example, a Huawei account), and then establish a remote connection by using a server. The medical treatment and health examination described above may include, but are not limited to, gastroscopy, respiratory medicine treatment, monitoring of respiratory diseases (for example, asthma), and the like.

### I. Breathing guidance in gastroscopy

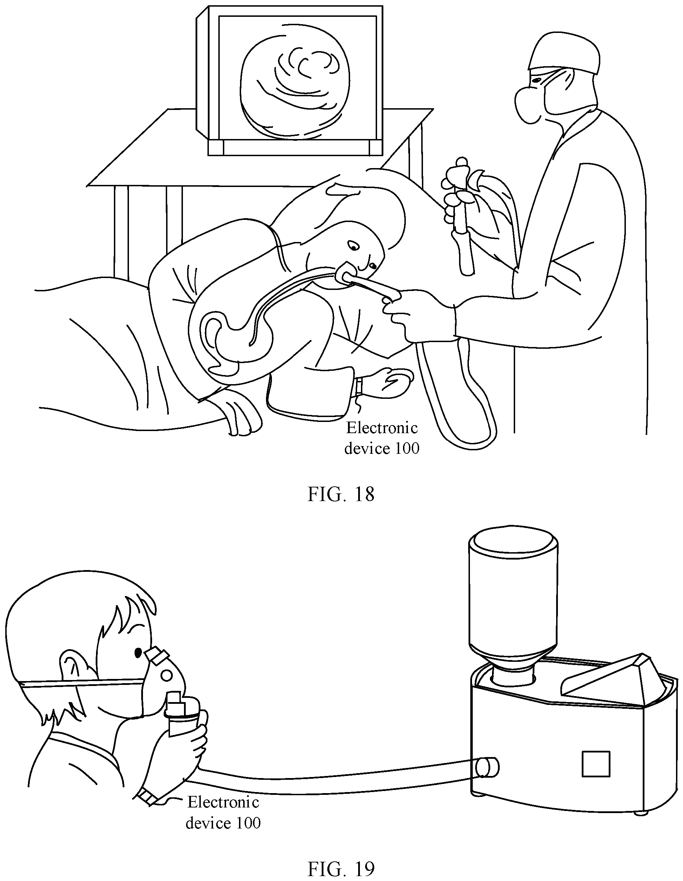

FIG. 18 shows an example of a schematic diagram of a scenario in gastroscopy.

During gastroscopy, a gastric tube enters the stomach from the inside of the mouth of a patient through the esophagus. As the gastric tube enters the stomach through the esophagus, user experience is poor, often with symptoms such as vomiting and breathing discomfort. During gastroscopy, usually oral breathing guidance of a doctor is used to prompt the breathing rhythm of the user, thereby reducing user discomfort. To make breathing guidance during gastroscopy more intelligent, in the following embodiments of this application, the electronic device 100 or a main body of the gastric tube may provide breathing guidance with reference to an action type of a gastroscope.

During gastroscopy, the first action is an action in which the gastric tube goes deep into the stomach along the esophagus of the gastric tube, and the second action is an action in which the gastric tube suspends deepening into the stomach along the esophagus of the gastric tube.

When the main body of the gastric tube provides a breathing guidance suggestion, the main body of the gastric tube may replace a function of the electronic device 100. Specifically, the main body of the gastric tube may collect motion sensor data of a motion sensor on the gastric tube, and determine the action type of the gastric tube based on the motion sensor data. When the main body of the gastric tube determines that the action type of the gastric tube is the first action, that is, when the gastric tube starts to go deep into the stomach along the esophagus of the gastric tube, the main body of the gastric tube prompts, in the first prompt manner, the user to perform an inhalation action. When the main body of the gastric tube determines that the action type of the gastric tube is the second action, that is, when the gastric tube suspends deepening downward along the esophagus of the gastric tube, the main body of the gastric tube prompts, in the second prompt manner, the user to perform an exhalation action. That the main body of the gastric tube prompts, in the first prompt manner, the user to perform an inhalation action is similar to that the electronic device 100 prompts, in the first prompt manner, the user to perform an inhalation action. Details are not described herein again in this embodiment of this application.

That the main body of the gastric tube prompts, in the second prompt manner, the user to perform an inhalation action is similar to that the electronic device 100 prompts, in the second prompt manner, the user to perform an exhalation action. Details are not described herein again in this embodiment of this application.

In some embodiments, the main body of the gastric tube may have a display screen, and content such as a text, a picture, or a video may be displayed on the display screen to prompt the user to perform an inhalation action or an exhalation action. Alternatively, content such as a text, a picture, or a video may be displayed on the display screen to prompt the user to perform the first action or the second action. Optionally, the main body of the gastroscope may alternatively prompt, in a voice manner or a vibration manner, the user to exhale and inhale.

When the electronic device 100 provides a breathing guidance suggestion, before the electronic device 100 starts to guide the breathing rhythm of the user, the electronic device 100 needs to establish a communication connection to the main body of the gastric tube. The main body of the gastric tube collects the motion sensor data, and sends the motion sensor data to the electronic device 100. After receiving the motion sensor data, the electronic device 100 determines, based on the motion sensor data, whether the action type of the user is the first action or the second action, and guides the breathing rhythm of the user. When the electronic device 100 determines that the action type of the gastric tube is the first action, that is, when the gastric tube starts to go deep into the stomach along the esophagus of the gastric tube, the electronic device 100 prompts, in the first prompt manner, the user to perform an inhalation action. When the electronic device 100 determines that the action type of the gastric tube is the second action, that is, when the gastric tube suspends deepening into the stomach along the esophagus of the gastric tube, the electronic device 100 prompts, in the second prompt manner, the user to perform an exhalation action.

When the electronic device 100 provides a breathing guidance suggestion, before the electronic device 100 starts to guide the breathing rhythm of the user, the electronic device 100 needs to establish a communication connection to the main body of the gastric tube. The main body of the gastric tube collects the motion sensor data, and determines whether the action type of the gastric tube is the first action or the second action based on the motion sensor data. When the main body of the gastric tube determines that the action of the user is the first action, that is, when the gastric tube starts to go deep into the stomach along the esophagus of the gastric tube, the main body of the gastric tube sends an instruction 1 to the electronic device 100. After receiving the instruction 1, the electronic device 100 prompts, in the first prompt manner, the user to inhale. When the main body of the gastric tube determines that the action of the user is the second action, that is, when the gastric tube suspends deepening into the stomach along the esophagus of the gastric tube, the main body of the gastric tube sends an instruction 2 to the electronic device 100. After receiving the instruction 2, the electronic device 100 prompts, in the second prompt manner, the user to exhale.

In this way, when the gastric tube goes deep into the stomach of the patient, the user inhales deeply to relax the entire body and expand the chest as much as possible. When the gastric tube suspends deepening into the stomach of the patient, the user can cooperatively exhale slowly to reduce discomfort during examination. In the prompt manner of the electronic device 100, the breathing rhythm of the patient can match a deep-into action and a suspending action of the gastric tube, thereby reducing discomfort of the user during gastroscopy.

Optionally, in different stages of gastroscopy, that is, when a head end of the gastric tube reaches different positions of the esophagus of the patient, breathing prompts provided by the electronic device 100 may also be different.

For example, when the gastroscope just passes through the mouth of the patient and reaches the throat (that is, the esophagus), discomfort of the user is the strongest during entire gastroscopy in this case. In this case, the electronic device 100 may prompt, in a strong vibration manner, the user to inhale. A frequency of strong vibration of the electronic device 100 may be a frequency 3. The frequency 3 is greater than a frequency 1. In this way, a stronger vibration frequency of the electronic device 100 indicates a longer time for the user to inhale, and discomfort can be reduced to some extent.

Optionally, the electronic device 100 may prompt, by using different voice content, the user to inhale in different stages of gastroscopy. When the gastroscope just passes through the mouth of the patient and reaches the throat (that is, the esophagus), the voice content may be "deeply inhale through the nasal cavity and last for a period of time (for example, 5s)".

Optionally, before the patient starts to undergo examination, the electronic device 100 may display breathing actions corresponding to different prompt manners. In this way, the user may know in advance the prompt manner of the electronic device 100, and when to exhale and when to exhale. For a specific implementation, refer to the embodiments in FIG. 5D to FIG. 5F. Principles are similar. Details are not described herein again in this embodiment of this application.

### II. Breathing guidance in respiratory medicine treatment

Respiratory medicine treatment may include, but is not limited to, nebulized inhalation therapy, ventilator therapy, and the like. In the following embodiments of this application, nebulized inhalation therapy is used as an example for description.

In respiratory medicine treatment, the first action is an action of delivering medicine by a nebulizer, and the second action is an action of suspending medicine delivery by the nebulizer.

FIG. 19 shows an example of a schematic diagram of a scenario in nebulized inhalation therapy.

In nebulized inhalation therapy, medicine enters the throat through normal breathing by changing from liquid to vapor by using a nebulizer or a nebulizer pump, to achieve a purpose of treating respiratory diseases.

Before starting nebulized inhalation therapy, the nebulizer may have a display screen. The display screen may also display a text, a picture, or a video to remind the user when the nebulizer starts to deliver medicine and when to suspend medicine delivery. Alternatively, the nebulizer may prompt the user by voice when the nebulizer starts to deliver medicine and when to suspend medicine delivery.

Alternatively, the electronic device 100 may establish a communication connection to the nebulizer before starting nebulized inhalation therapy. The nebulizer sends an electronic version of a tutorial to the electronic device 100 by using a communication module. The patient can learn precautions for using the nebulizer by playing the electronic version of the tutorial through the electronic device 100, for example, how to achieve better treatment results by controlling the breathing rhythm to match a rhythm of medicine delivery of the nebulizer. Alternatively, the electronic device 100 may display breathing actions and the like corresponding to different prompt manners.

When the nebulizer provides a breathing guidance suggestion, the nebulizer may replace a function of the electronic device 100. When the nebulizer starts to deliver medicine, the nebulizer prompts the user, in the first prompt manner, to perform an inhalation action. In this way, when the nebulizer delivers medicine, the patient starts to inhale, so that the medicine can reach the respiratory tract of the patient, and a treatment effect is better. When the nebulizer suspends medicine delivery, the nebulizer prompts, in the second prompt manner, the user to perform an exhalation action. In this way, when the nebulizer suspends medicine delivery, the patient starts to exhale, to achieve a purpose of relaxing the entire body.

That the nebulizer prompts, in the first prompt manner, the user to perform an inhalation action is similar to that the electronic device 100 prompts, in the first prompt manner, the user to perform an inhalation action. Details are not described herein again in this embodiment of this application.

That the nebulizer prompts, in the second prompt manner, the user to perform an inhalation action is similar to that the electronic device 100 prompts, in the second prompt manner, the user to perform an exhalation action. Details are not described herein again in this embodiment of this application.

In some embodiments, the nebulizer may have a display screen, and content such as a text, a picture, or a video may also be displayed on the display screen to prompt the user to perform an inhalation action or an exhalation action. Optionally, the nebulizer may alternatively prompt, in a voice manner or a vibration manner, the user to perform an inhalation action or an exhalation action.

When the electronic device 100 provides a breathing guidance suggestion, before the electronic device 100 starts to guide the breathing rhythm of the user, the electronic device 100 needs to establish a communication connection to the nebulizer. When the nebulizer starts to deliver medicine, the nebulizer sends an instruction 1 to the electronic device 100 by using the communication module. After receiving the instruction 1, the electronic device 100 prompts, in the first prompt manner, the patient to start to inhale. When the nebulizer suspends medicine delivery, the nebulizer sends an instruction 2 to the electronic device 100 by using the communication module. After receiving the instruction 2, the electronic device 100 prompts, in the second prompt manner, the patient to start to exhale. In this way, when the nebulizer delivers medicine, the patient starts to inhale, so that the medicine can reach the respiratory tract of the patient, and a treatment effect is better.

The communication connection between the electronic device 100 and the nebulizer may be a wired connection or a wireless connection. The wireless connection may be a close-range connection such as a wireless local area network (wireless local area network, WLAN) connection, a wireless fidelity (wireless fidelity, Wi-Fi) connection, a Bluetooth connection, an infrared connection, a near field communication (near field communication, NFC) connection, ZigBee, or another wireless communication technology that appears in subsequent development. Alternatively, the nebulizer may establish a long-distance connection to the electronic device 100. The long-distance connection includes but is not limited to a long-distance connection based on a mobile network of 2G, 3G, 4G, 5G, and a subsequent standard protocol. Alternatively, the nebulizer and the electronic device 100 may log in to a same user account (for example, a Huawei account), and then establish a remote connection by using a server.

### III. Monitoring of respiratory diseases (for example, asthma)

The electronic device 100 may collect, by using the electronic device 100, physiological data such as a heart rate, a respiratory rate, a blood oxygen level, and an asthma sound sent from the lung of the user, and determine whether an asthma attack occurs on the user.

When the electronic device 100 determines that an asthma attack occurs on the user, the electronic device 100 may provide a breathing guidance suggestion, which may relieve a symptom of the user when the asthma attack occurs.

Optionally, before the electronic device 100 provides the breathing guidance suggestion, the electronic device 100 may display content such as a text, a picture, or a video teaching on the display screen to provide some suggestions, or provide some suggestions in a voice manner, so that the user knows how to relieve the symptom in an asthma attack process.

For example, the electronic device 100 prompts, in a voice form, the user to take a deep breath, take an upright lying posture, use related medicine treatment, and the like. Voice content may be "deeply inhale, slowly exhale, and the like". The voice content may alternatively be "choose a semi-recumbent position, sit at an upright position, untie the button near the neck, and ensure smooth breathing". The voice content may alternatively be "please use the bronchodilator inhaler".

For example, the electronic device 100 may display an animation on the display screen, and the electronic device 100 prompts the user to perform an action along with the animation on the display screen, for example, choosing a correct lying posture and how to control the breathing rhythm.

When the electronic device 100 provides the breathing guidance suggestion, the electronic device 100 may prompt, in the first prompt manner, the user to deeply inhale. Then, after a specific period of time (for example, 2 seconds), the electronic device 100 may prompt, in the second prompt manner, the user to slowly exhale. In this way, the symptom of the user when the asthma attack occurs can be relieved by rhythmic breathing.

Optionally, the electronic device 100 may prompt, through voice, a text, or vibration, the user to take a medicine, and display a corresponding medicine name and corresponding medicine taking instructions on the display screen of the electronic device 100.

Optionally, if the electronic device 100 detects that the physiological data of the user is higher than a normal value, the electronic device 100 may ask, by using a voice module, whether the user needs help. When a speech recognition result is "yes" or the electronic device 100 does not receive any reply when a specific period of time expires, the electronic device 100 may consider that the user needs help. On one hand, the electronic device 100 stores contact information of an emergency contact, and the electronic device 100 may directly send the physiological data of the user and possible symptom information (for example, asthma) to the emergency contact. On the other hand, the electronic device 100 may send a current location of the user, a name of the user, a home address, a disease history, a telephone number, the physiological data of the user, and the possible symptom information to a server. The server invokes a map service to find a community health service center closest to the current location of the user, and obtains a name, an on-duty worker, and a telephone number of the community health service center. The server notifies, by using an application or in another manner, the community health service center closest to the user of the current location of the user, the name, the home address, the disease history, the telephone number, the physiological data of the user, and the possible symptom information. Then, if the on-duty worker of the community health service center closest to the user sees the information of the user, the on-duty worker will contact the user according to the telephone number sent by the server to ask if help is needed and take further first aid measures and assistance.

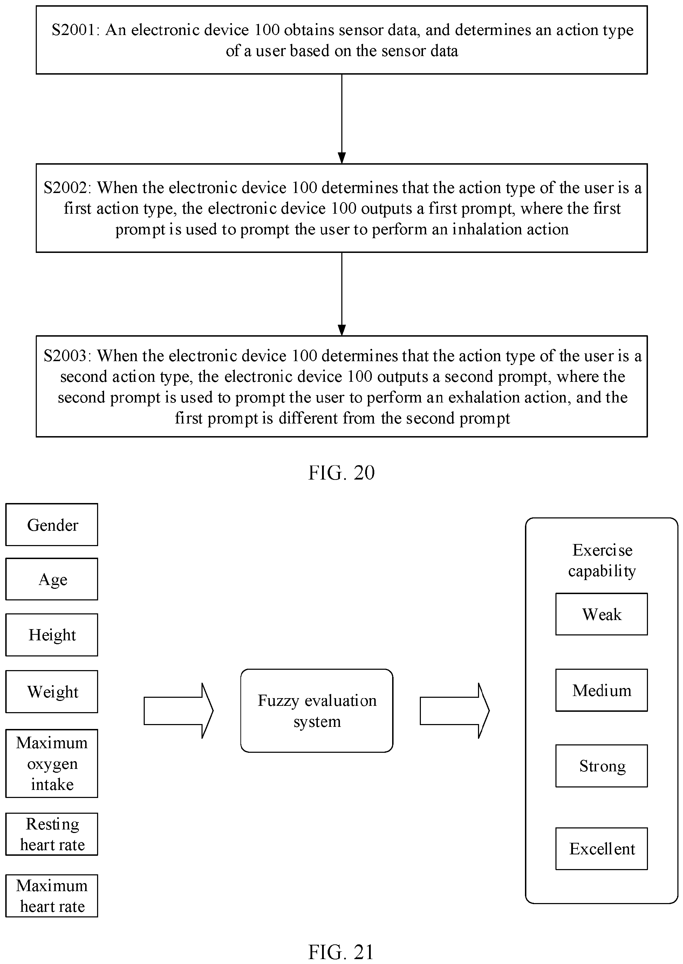

FIG. 20 shows an example of a schematic method flowchart of a breathing guidance method according to an embodiment of this application.

As shown in FIG. 20, the method includes the following steps:
S2001: An electronic device 100 (a first electronic device) obtains sensor data, and determines an action type of a user based on the sensor data.

The electronic device 100 may be a mobile phone, a wearable device, a headset, smart glasses, an augmented reality (augmented reality, AR) device, a virtual reality (virtual reality, VR) device, or the like. The wearable device may be a wrist-supported device, for example, a smartwatch, a smart band, or a smart wrist strap. Alternatively, the wearable device may be an ankle-supported device, for example, a smart anklet, smart shoes, smart socks, or another device that can be worn on a leg. Alternatively, the wearable device may be a head-supported device, for example, a smart helmet or a smart head strap (which may also be referred to as a smart headband).

Alternatively, the electronic device 100 may be a medical equipment device, a fitness equipment device, or the like. The medical equipment device may include but is not limited to a ventilator, a nebulizer, a gastroscope device, a chest X-ray detection device, and the like. The fitness equipment device may include but is not limited to a hull, a rowing machine, an elliptical machine, a barbell, and the like.

The sensor data includes one or more of acceleration data, gyroscope data, image data, gravity data, and pressure data.

Before the electronic device 100 obtains the sensor data, the electronic device 100 receives a first input operation, and responds to the first input operation to determine a first exercise mode, where the first exercise mode is any one of the following: a running mode, a swimming mode, a weight lifting mode, an elliptical machine exercise mode, a rowing machine exercise mode, and a rowing mode. The electronic device 100 determines the action type of the user in the first exercise mode based on the sensor data. In this way, the electronic device 100 may enable different exercise modes before the user exercises, and provide different breathing guidance solutions in different exercise modes. Specifically, refer to the embodiments shown in FIG. 3A and FIG. 3B to FIG. 3D. Details are not described herein again in this embodiment of this application.

In another possible implementation, the electronic device 100 may adaptively enable the first exercise mode (for example, the running mode) based on collected motion sensor data. After the electronic device 100 adaptively enables the first exercise mode, the electronic device 100 displays a first interface, and a first control is displayed in the first interface. The electronic device 100 receives and responds to an input operation of the user for the first control, and the electronic device 100 cancels enabling of the first exercise mode. In this way, the electronic device 100 is prevented from mistakenly enabling the first exercise mode, which increases consumption of the electronic device 100. The first interface may be the user interface shown in FIG. 3E, and the first control may be the control 314 shown in FIG. 3E. Specifically, refer to the embodiments shown in FIG. 3A to FIG. 3E. Details are not described herein again in this embodiment of this application.

Before the electronic device 100 obtains the sensor data, the electronic device 100 receives the sensor data sent by a third electronic device. The third electronic device may be a fitness equipment device, or may be a medical equipment device. That is, the first electronic device establishes a communication connection to the first electronic device. The third electronic device collects motion sensor data on the third electronic device in real time, and sends the motion sensor data to the first electronic device in real time.

Optionally, before the electronic device 100 starts to guide the user to breathe, the electronic device 100 may display a guiding action on a display screen. The guiding action is used to indicate breathing actions corresponding to different action types to the user. Specifically, refer to the embodiments shown in FIG. 5D to FIG. 5F

Optionally, before the electronic device 100 starts to guide the user to breathe, the electronic device 100 may display an animation on the display screen. The animation is used to indicate breathing actions corresponding to different action types to the user.

S2002: When the electronic device 100 determines that the action type of the user is a first action type, the electronic device 100 outputs a first prompt, where the first prompt is used to prompt the user to perform an inhalation action.

In a possible implementation, a first action may be that the electronic device 100 determines, based on the motion data of the user collected by the electronic device 100, that a motion posture of the user is the first action.

When the first action is a motion posture of the user, the first action may be an arm action of the user, or the first action may be a leg action of the user. In an exercise process, the arm action and the leg action are coordinately performed. An example in which the first action is the arm action of the user is used for description in this embodiment of this application.

For related description and introduction of the first action in the exercise process, refer to FIG. 5A and FIG. 5B, FIG. 6A and FIG. 6B, FIG. 7A and FIG. 7B, FIG. 8A and FIG. 8B, FIG. 9A and FIG. 9B, FIG. 10A and FIG. 10B, FIG. 11A and FIG. 11B, FIG. 12A and FIG. 12B, and FIG. 13A and FIG. 13B. Details are not described again in this embodiment of this application.

In another possible implementation, the first action may alternatively be that the third electronic device determines a motion posture of the third electronic device based on the motion data collected by the third electronic device, and infers the motion posture of the user based on the motion posture of the third electronic device. After the third electronic device infers the motion posture of the user, the third electronic device sends an instruction to the electronic device 100, where the instruction is used to indicate the electronic device 100 that the user is performing the first action.

Before the user starts exercise, the third electronic device needs to establish a communication connection to the electronic device 100. The communication connection may be a wired connection or a wireless connection. The wireless connection may be a short-distance connection such as a wireless fidelity (wireless fidelity, Wi-Fi) connection, a Bluetooth connection, an infrared connection, an NFC connection, or a ZigBee connection, or may be a long-distance connection. The long-distance connection includes but is not limited to a long-distance connection based on 2G, 3G, 4G, 5G, and a mobile network of a subsequent standard protocol. For example, the electronic device 100 and the third electronic device may log in to a same user account (for example, a Huawei account), and then establish a remote connection by using a server.

After the third electronic device infers that the motion posture of the user is the first action, the third electronic device sends an instruction 1 to the electronic device 100. After the electronic device 100 receives the instruction 1, the electronic device 100 prompts, in a first prompt manner, the user to inhale.

In another possible implementation, the first action is a motion posture of another electronic device (for example, medical equipment). The another electronic device determines the motion posture of the another electronic device based on motion data collected by the another electronic device. When the another electronic device determines that the motion posture of the another electronic device is the first action, the another electronic device sends an instruction 1 to the electronic device 100. After the electronic device 100 receives the instruction 1, the electronic device 100 prompts, in the first prompt manner, the user to inhale. In this way, a breathing rhythm of the user can be matched with the motion posture of the another electronic device (such as the medical equipment), especially in some health fields when the user undergoes health examination and treatment, for example, gastroscopy, nebulization treatment, chest X-ray examination, and the like. When the motion posture of the another electronic device is the first action, the another electronic device sends the instruction 1 to the electronic device 100. After the electronic device 100 receives the instruction 1, that is, the electronic device 100 detects that the motion posture of the another electronic device is the first action, the electronic device 100 prompts, in the first prompt manner, the user to inhale. In this way, the breathing rhythm of the user can be matched with the action of the medical equipment, discomfort of the user in health examination can be relieved, and a treatment effect can be improved. Specifically, refer to Embodiment 2 described in FIG. 18 and FIG. 19. Details are not described herein again in this embodiment of this application.

S2003: When the electronic device 100 determines that the action type of the user is a second action type, the electronic device 100 outputs a second prompt, where the second prompt is used to prompt the user to perform an exhalation action, and the first prompt is different from the second prompt.

The second action is similar to the first action. Specifically, refer to the related explanation of the first action in S2002. Details are not described again in this embodiment of this application.

Optionally, when the electronic device 100 outputs the first prompt, the electronic device 100 sends a first instruction to a second electronic device, where the first instruction is used to instruct the second electronic device to output a third prompt, the third prompt is used to prompt the user to perform an inhalation action, and a type of the third prompt is any one or more of the following: vibration, voice, text, and picture. In this way, when outputting the first prompt, the electronic device 100 may output the third prompt by using another electronic device (the second electronic device is a headset or a mobile phone) or the like to which the first electronic device establishes a connection. In another possible implementation, the electronic device 100 may not output any content, but output the third prompt by using another electronic device (the second electronic device is a headset or a mobile phone) or the like to which the first electronic device establishes a connection.

Optionally, a third electronic device collects the sensor data, and determines the action type of the user based on the sensor data. When the third electronic device determines that the action type of the user is the first action type, the third electronic device sends an instruction 1 to the first electronic device. After receiving the instruction 1, the first electronic device outputs the first prompt, where the first prompt is used to prompt the user to perform an inhalation action. When the third electronic device determines that the action type of the user is the second action type, the third electronic device sends an instruction 2 to the first electronic device. After receiving the instruction 2, the first electronic device outputs the second prompt, where the second prompt is used to prompt the user to perform an exhalation action, and the first prompt is different from the second prompt.

A type of the first prompt manner is any one or more of the following: vibration, voice, text, and picture.

A type of the second prompt is any one or more of the following: vibration, voice, text, and picture.

For example, a vibration frequency of the first prompt is different from a vibration frequency of the second prompt. Alternatively, voice content of the first prompt is different from voice content of the second prompt. Alternatively, a type of the first prompt is any one or more of the following: vibration, voice, text, and picture; and the second prompt does not output any content; or the first prompt does not output any content; and a type of the second prompt is any one or more of the following: vibration, voice, text, and picture.

In this way, the electronic device 100 may prompt, in different prompt manners, the user when to inhale and when to inhale, so that a rhythm of an action matches a breathing rhythm of the user, thereby saving physical energy and improving an exercise capability of the user.

Optionally, after the electronic device 100 outputs the first prompt and before the electronic device 100 outputs the second prompt, when the electronic device 100 determines that the action type of the user is a third action type, the electronic device 100 outputs a fourth prompt, where the fourth prompt is used to prompt the user to perform a breath holding action, and a type of the fourth prompt is any one or more of the following: vibration, voice, text, and picture. For example, in weight lifting exercise, after the user completes a first action, the user holds a barbell at a highest point for a period of time. In this period of time, the action of the user may be referred to as a third action. For example, in swimming exercise, after the user completes a first action, the user keeps an arm action stationary for a period of time. The user action herein may also be referred to as a third action. When the user performs the third action, in a possible implementation, the electronic device 100 prompts, in a fourth prompt manner, the user to hold breath. In another possible implementation, the electronic device 100 prompts, in a fourth prompt manner, the user to perform one or more sets of brief exhalation and inhalation actions.

Optionally, the electronic device 100 outputs the first prompt when the electronic device 100 determines that the action type of the user is the first action type and the electronic device 100 detects that the user has completed the first action, where the first prompt is used to prompt the user to perform an inhalation action. For example, in a swimming application scenario, only when the user completes the first action, the head of the user can extend out of the water to perform an inhalation action. In another possible implementation, the electronic device 100 outputs the second prompt when the electronic device 100 determines that the action type of the user is the second action type and the electronic device 100 detects that the user has completed a second action, where the second prompt is used to prompt the user to perform an exhalation action. For example, in a swimming application scenario, when the user completes the second action, the electronic device 100 prompts the user to perform an exhalation action.

Optionally, the electronic device 100 may continuously provide breathing guidance to the user after the user starts exercise and before the user ends exercise. In this way, the electronic device 100 can always provide breathing guidance for the user in the exercise process of the user, and the user can better coordinate a motion action with the breathing rhythm. For example, in a process in which the user runs, when detecting an arm action that an arm of the user wearing the electronic device 100 swings forward, the electronic device 100 prompts, in the first prompt manner, the user to inhale. When detecting an arm action that the arm of the user wearing the electronic device 100 swings backward, the electronic device 100 prompts, in the second prompt manner, the user to exhale. Until the user ends exercise, the electronic device 100 stops guiding the user to perform a breathing action. In this way, the electronic device 100 can always guide breathing of the user in the running process of the user, and the user can better coordinate an arm swing action and the breathing rhythm in the running process.

Optionally, in the exercise process of the user, the user may actively trigger to suspend and/or continue breathing guidance provided by the electronic device 100 to the user. For example, the electronic device 100 may receive and respond to voice of the user in the running process of the user, to suspend or continue guiding of the breathing action of the user in combination with the arm swing action of the user. For example, when the device 100 is guiding the breathing of the user, the user may wake up the electronic device 100 through voice "Xiao A Xiao A, suspend the breathing guidance". After identifying the voice of the user, the electronic device 100 suspends monitoring of the arm action of the user during running, that is, the electronic device 100 suspends guiding of the breathing action of the user. When the user wants to use the electronic device 100 to guide the breathing action of the user again, the user may wake up the electronic device 100 through voice "Xiao A Xiao A, continue the breathing guidance". After identifying the voice of the user, the electronic device 100 continues to monitor the arm action of the user during running, and guides the breathing action of the user again in combination with the arm action of the user during running. In this way, the user can flexibly control the electronic device 100 based on a requirement of the user, thereby improving user experience.

In the exercise process of the user, the electronic device 100 can adaptively suspend breathing guidance for the user. For example,
in a process in which the user runs, when detecting an arm action that an arm of the user wearing the electronic device 100 swings forward, the electronic device 100 prompts, in the first prompt manner, the user to inhale. When detecting an arm action that the arm of the user wearing the electronic device 100 swings backward, the electronic device 100 prompts, in the second prompt manner, the user to exhale. A period of time (first time) later after the electronic device 100 guides the user to perform a breathing action, the electronic device 100 suspends monitoring of the arm action of the user during running, and the electronic device 100 also suspends guiding of the breathing action of the user. In this way, power consumption of the electronic device 100 can be reduced compared with a case that the breathing of the user is always guided in the running process of the user.

Optionally, in the exercise process of the user, the electronic device 100 suspends breathing guidance for the user, and then the electronic device 100 may adaptively enable breathing guidance for the user. For example, after the electronic device 100 suspends monitoring of the arm action of the user and guiding the breathing action, the electronic device 100 may collect the respiratory rate of the user by using a microphone built in the electronic device 100, or the headset is connected to the electronic device 100, and the user listens to music while exercising with the headset; or the electronic device 100 may collect the respiratory rate of the user by using a microphone on the headset connected to the electronic device 100. When the electronic device 100 determines that the respiratory rate of the user does not match the respiratory rate in the breathing guidance solution of the running mode, the electronic device 100 may prompt, via voice, vibration, text, or the like, the user whether breathing guidance is needed; or when the electronic device 100 determines that the respiratory rate of the user is greater than the respiratory rate in the breathing guidance solution of the running mode, the electronic device 100 may prompt the user to slow down the respiratory rate. When the electronic device 100 determines that the respiratory rate of the user is less than the respiratory rate (preset frequency) in the breathing guidance solution of the running mode, the electronic device 100 may prompt the user to accelerate the respiratory rate. After the electronic device 100 continues to monitor the arm action of the user and guide the breathing action for a period of time, when the electronic device 100 determines that the respiratory rate of the user matches the respiratory rate in the breathing guidance solution of the running mode, the electronic device 100 may suspend the monitoring of the arm action of the user and guide the breathing action of the user. In this way, flexibility of the electronic device 100 in providing breathing guidance for the user is reflected.

Optionally, in the exercise process of the user, the electronic device 100 may obtain the heart rate of the user in real time, and determine rest time of the user based on the heart rate of the user. For example, in the running process, the electronic device 100 may obtain the heart rate of the user in real time, and determine the rest time of the user based on the heart rate of the user. Specifically, the electronic device 100 determines a metabolic manner of the user based on a heart rate change status of the user, including an aerobic manner and an anaerobic manner, or an aerobic manner, an anaerobic lactic manner, an anaerobic non-lactic manner, or the like. Different metabolic manners may correspond to different rest time. After the user ends exercise in a specific stage, the electronic device 100 may remind the user of available rest time. Alternatively, further, in the running process of the user, when the electronic device 100 determines that the heart rate of the user is not within a range specified by a preset heart rate (preset heart rate value), the electronic device 100 may prompt, via voice, vibration, text, or the like, the user to stop exercise and start to rest. Alternatively, further, in a process in which the user rests, the electronic device 100 may prompt, based on the heart rate change status of the user, the user to stop resting and start exercise.

Optionally, in the exercise process of the user, the electronic device 100 suspends breathing guidance for the user, and then the electronic device 100 may adaptively enable breathing guidance for the user. For example, after the electronic device 100 suspends monitoring of the arm action of the user and guidance of the breathing action of the user, the electronic device 100 may collect the heart rate of the user. When the electronic device 100 determines that the heart rate of the user is not within the range specified by the preset heart rate, the electronic device 100 may prompt, via voice, vibration, text, or the like, the user whether breathing guidance is needed. After the electronic device 100 continues to monitor the arm action of the user and guide the breathing action for a period of time, when the electronic device 100 determines that the heart rate of the user is within the range specified by the preset heart rate, the electronic device 100 may suspend the monitoring of the arm action of the user and guide the breathing action of the user. In this way, flexibility of the electronic device 100 in providing breathing guidance for the user is reflected.

Optionally, the electronic device 100 may determine the preset heart rate of the user based on an exercise capability of the user. When the electronic device 100 suspends breathing guidance for the user, or before the user exercises, the electronic device 100 does not enable breathing guidance, if the heart rate of the user is higher than the preset heart rate of the user, the electronic device 100 may adaptively enable breathing guidance. For example, in the running process, the electronic device 100 suspends breathing guidance for the user, or the user does not enable breathing guidance for the electronic device 100 before starting running. The electronic device 100 may obtain the heart rate of the user in real time in the running process of the user. If the electronic device 100 detects that the heart rate of the user is greater than the preset heart rate, the electronic device 100 may ask, via voice, vibration, or text, the user whether the breathing guidance function needs to be enabled, or the electronic device 100 may automatically enable the breathing guidance function, and guide the breathing action of the user in combination with the arm swing action of the user during running, so that the arm swing action of the user in the running process matches the breathing rhythm.

Optionally, in the exercise process of the user, the electronic device 100 suspends breathing guidance for the user, or the electronic device 100 does not enable the breathing guidance function before the user exercises. In this case, the electronic device 100 may prompt, based on the exercise capability of the user and in combination with one or more factors of an exercise type, an exercise goal, a temperature, a humidity, and atmospheric pressure, the user whether breathing guidance is needed after the user exercises for a period of time.

Optionally, the electronic device 100 may prompt, based on the exercise capability of the user, whether the user needs to rest after the user exercises for a period of time.

In a possible implementation, the electronic device 100 may determine preset exercise time of the user based on the exercise capability of the user. If running time of the user exceeds the preset exercise time, the electronic device 100 may prompt the user whether to take a rest.

In another possible implementation, the electronic device 100 may determine the preset heart rate of the user based on the exercise capability of the user. The electronic device 100 may obtain the heart rate of the user in real time. If the electronic device 100 detects that the heart rate of the user is greater than the preset heart rate, the electronic device 100 may prompt the user whether to take a rest.

The following describes how the electronic device 100 determines the exercise capability of the user.

FIG. 21 shows an example of a schematic diagram in which the electronic device 100 obtains the exercise capability of the user according to personal information of the user.

First, the electronic device 100 obtains the personal information of the user. The personal information of the user includes but is not limited to: gender, age, height and weight, maximum oxygen uptake, resting heart rate, maximum heart rate, and the like. The maximum oxygen intake may be a maximum value in oxygen intake data of the user obtained by the electronic device 100 in a previous exercise process of the user. The maximum heart rate may be a maximum value of heart rates of the user obtained by the electronic device 100 in the previous exercise process of the user. Personal data of the user may be stored in the electronic device 100, or may be input by the user, or may be obtained from another device that establishes a connection to the electronic device 100.

Then, the electronic device 100 evaluates the exercise capability of the user based on the personal information of the user and a fuzzy evaluation system.

The fuzzy evaluation system refers to using a method of fuzzy mathematics to provide, based on an evaluation criterion, a possibility of obtaining a specific comment for a thing/an object affected by a plurality of parameters. In other words, if a proportion of each parameter is different, an output result of the fuzzy evaluation system is also different. The electronic device 100 may set a same proportion for each parameter input to the fuzzy evaluation system, or may set a different proportion for each parameter input to the fuzzy evaluation system. This is not limited in this embodiment of this application.

Each parameter in the personal information is related to the exercise capability of the user. For example, the exercise capability of men is stronger than that of women. The exercise capability of the young is stronger than that of the juvenile and old. The exercise capability of people with high oxygen intake is stronger than that of people with low oxygen intake and so on.

The electronic device 100 inputs the personal information of the user into the fuzzy evaluation system, and the fuzzy evaluation system outputs the exercise capability of the user. The exercise capability may be classified into weak, medium, strong, and excellent levels. Certainly, the exercise capability may alternatively be classified into more or fewer levels. This is not limited in this embodiment of this application.

Because the personal information of the user changes, the electronic device 100 needs to periodically update the personal information of the user. In this case, the electronic device 100 may also periodically update the exercise capability of the user.

After the user starts exercise, the electronic device 100 may display an exercise result. The exercise result displayed by the electronic device 100 may include but is not limited to the following:

### I. Motion data of the user

The motion data of the user includes a heart rate, calories, a respiratory rate, exercise time, an exercise date, exercise days, and the like. In this way, the user may learn about an exercise status of the user in detail based on the exercise result. In addition, the exercise status of the user may include a current exercise status and a historical exercise status. In this way, the user may compare the current exercise status of the user with a previous exercise status, to check whether the user has made progress, whether the body has been exercised, and the like.

### II. Dietary precautions

The electronic device 100 may determine, based on data such as an energy consumption status and exercise time of the user, a nutrient that needs to be ingested by the user after exercise. For example, the electronic device 100 may display, in the exercise result, a nutrient intake proportion or a recommended food for the user. The nutrient intake proportion may be used to indicate a proportion of protein, sugar, fat, dietary fiber, water, or the like that the user needs to ingest after the exercise. The recommended food may include milk, eggs, beef, and the like, and the electronic device 100 may recommend different foods according to exercise intensity of the user. This can help the user recover physical strength more quickly to compensate for energy consumed in the exercise process.

It may be understood that the exercise result is not limited to the foregoing content, and may further include data such as basic user information. This is not limited in this embodiment of this application.

Further, the electronic device 100 may further provide a sharing function, and the user may share the exercise result of the user with another device or another social platform by using the sharing function.

Various implementations of this application may be combined randomly to implement different technical effects.

Persons of ordinary skill in the art may understand that all or some of the procedures of the methods in the foregoing embodiments may be implemented by a computer program instructing related hardware. The program may be stored in a computer-readable storage medium. When the program is executed, the procedures in the method embodiments may be included. The foregoing storage medium includes any medium that can store program code, such as a ROM, a random access memory RAM, a magnetic disk, or an optical disc.

In conclusion, the foregoing descriptions are merely embodiments of the technical solutions of the present invention, but are not intended to limit the protection scope of the present invention. Any modification, equivalent replacement, or improvement made according to the disclosure of the present invention shall fall within the protection scope of the present invention.

## Claims

1. A breathing guidance method, wherein the method comprises:
obtaining, by a first electronic device, sensor data;
determining, by the first electronic device, an action type of a user based on the sensor data; and
outputting, by the first electronic device, a first prompt when the first electronic device determines that the action type of the user is a first action type, wherein the first prompt is used to prompt the user to perform an inhalation action; or
outputting, by the first electronic device, a second prompt when the first electronic device determines that the action type of the user is a second action type, wherein the second prompt is used to prompt the user to perform an exhalation action, and the first prompt is different from the second prompt.

2. The method according to claim 1, wherein a type of the first prompt is any one or more of the following:
vibration, voice, text, and picture; and
a type of the second prompt is any one or more of the following: vibration, voice, text, and picture.

3. The method according to claim 2, wherein that the first prompt is different from the second prompt specifically comprises:
a vibration frequency of the first prompt is different from a vibration frequency of the second prompt.

4. The method according to claim 2, wherein that the first prompt is different from the second prompt specifically comprises:
voice content of the first prompt is different from voice content of the second prompt.

5. The method according to claim 1, wherein that the first prompt is different from the second prompt specifically comprises:
a type of the first prompt is any one or more of the following: vibration, voice, text, and picture; and
the second prompt does not output any content; or
the first prompt does not output any content; and
a type of the second prompt is any one or more of the following: vibration, voice, text, and picture.

6. The method according to any one of claims 1 to 5, wherein the method further comprises:
when the first electronic device outputs the first prompt, sending, by the first electronic device, a first instruction to a second electronic device, wherein the first instruction is used to instruct the second electronic device to output a third prompt, the third prompt is used to prompt the user to perform an inhalation action, and a type of the third prompt is any one or more of the following: vibration, voice, text, and picture.

7. The method according to any one of claims 1 to 6, wherein the outputting, by the electronic device, a first prompt when the first electronic device determines that the action type of the user is a first action type specifically comprises:
outputting, by the electronic device, the first prompt when the first electronic device determines, for n consecutive times, that the action type of the user is the first action type, wherein n is a positive integer greater than or equal to 1.

8. The method according to any one of claims 1 to 7, wherein after the outputting, by the first electronic device, a first prompt, and before the outputting, by the first electronic device, a second prompt, the method further comprises:
outputting, by the first electronic device, a fourth prompt when the first electronic device determines that the action type of the user is a third action type, wherein the fourth prompt is used to prompt the user to perform a breath holding action, and a type of the fourth prompt is any one or more of the following: vibration, voice, text, and picture.

9. The method according to any one of claims 1 to 7, wherein the outputting, by the first electronic device, a first prompt when the first electronic device determines that the action type of the user is a first action type, wherein the first prompt is used to prompt the user to perform an inhalation action specifically comprises:
outputting, by the first electronic device, the first prompt when the first electronic device determines that the action type of the user is the first action type and the first electronic device detects that the user has completed the first action, wherein the first prompt is used to prompt the user to perform an inhalation action.

10. The method according to any one of claims 1 to 9, wherein before the obtaining, by a first electronic device, sensor data, the method further comprises:
receiving, by the first electronic device, a first input operation, and responding to the first input operation to determine a first exercise mode, wherein the first exercise mode is any one of the following: a running mode, a swimming mode, a weight lifting mode, an elliptical machine exercise mode, a rowing machine exercise mode, and a rowing mode; and
the determining, by the first electronic device, an action type of a user based on the sensor data specifically comprises:
determining, by the first electronic device, the action type of the user in the first exercise mode based on the sensor data.

11. The method according to any one of claims 1 to 10, wherein before the obtaining, by a first electronic device, sensor data, the method further comprises:
receiving, by the first electronic device, the sensor data sent by a third electronic device

12. The method according to claim 11, wherein the sensor data comprises one or more of acceleration data, gyroscope data, image data, gravity data, and pressure data.

13. The method according to any one of claims 10 to 12, wherein within first time after the first electronic device determines the first exercise mode, the first electronic device suspends obtaining of the sensor data.

14. The method according to claim 13, wherein after the first electronic device suspends obtaining of the sensor data, if the first electronic device detects that a heart rate of the user is greater than a preset heart rate value and/or a respiratory rate of the user is greater than a preset frequency, the first electronic device continues obtaining the sensor data.

15. An electronic device, wherein the electronic device comprises one or more processors and one or more memories, the one or more memories are coupled to the one or more processors, the one or more memories are configured to store computer program code, the computer program code comprises computer instructions, and the one or more processors invoke the computer instructions to enable the electronic device to perform the method according to any one of claims 1 to 14.

16. A computer-readable storage medium, comprising instructions, wherein when the instructions are run on an electronic device, the electronic device is enabled to perform the method according to any one of claims 1 to 14.

17. A computer program product, wherein when the computer program product runs on an electronic device, the electronic device is enabled to perform the method according to any one of claims 1 to 14.
